# EUROPEAN PATENT SPECIFICATION

(11) **EP 4 243 905 B1**
(45) Date of publication and mention of the grant of the patent: **23.04.2025**
(21) Application number: 21890409.2
(22) Date of filing: 12.11.2021
(51) Int. Cl.: A61M 16/06, A61M 16/08, A61M 16/20

(54) **PATIENT INTERFACE SYSTEM**
PATIENTENSCHNITTSTELLENSYSTEM
SYSTÈME D'INTERFACE PATIENT

(30) Priority: 13.11.2020 AU 2020904177; 11.06.2021 AU 2021901765
(43) Date of publication of application: 20.09.2023
(73) Proprietor: ResMed Pty Ltd, Bella Vista, New South Wales 2153 (AU)
(72) Inventor: EVES, Matthew, Bella Vista, New South Wales 2153 (AU)
(74) Representative: Vossius & Partner Patentanwälte Rechtsanwälte mbB
(86) International application number: PCT/AU2021/051348
(87) International publication number: WO 2022/099377

(56) References cited:
- WO-A1-2012/040792
- WO-A1-2015/020535
- WO-A1-2020/157559
- WO-A1-2020/191463
- WO-A2-2015/187995
- US-A1- 2014 276 177
- US-A1- 2015 246 199
- US-A1- 2019 022 343
- US-A1- 2020 345 963

## Description

### 1 CROSS-REFERENCE TO RELATED APPLICATIONS

This application claims priority to Australian Provisional Application Nos. 2020904177, filed November 13, 2020, and 2021901765, filed June 11, 2021.

### 2 BACKGROUND OF THE TECHNOLOGY

### 2.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 2.2 DESCRIPTION OF THE RELATED ART

### 2.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

### 2.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 2.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

### 2.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

### 2.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 2.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063328 and WO 2006/130903 (describing amongst other things aspects of the ResMed Limited MIRAGE LIBERTYTM full-face mask); International Patent Application WO 2009/052560 (describing amongst other things aspects of the ResMed Limited SWIFTTM FX nasal pillows).

### 2.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 2.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

### 2.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

### 2.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 2.2.3.5 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

The vent may comprise an orifice and gas may flow through the orifice in use of the mask. Many such vents are noisy. Others may become blocked in use and thus provide insufficient washout. Some vents may be disruptive of the sleep of a bed partner 1100 of the patient 1000, e.g. through noise or focussed airflow.

ResMed Limited has developed a number of improved mask vent technologies. See International Patent Application Publication No. WO 1998/034,665; International Patent Application Publication No. WO 2000/078,381; US Patent No. 6,581,594; US Patent Application Publication No. US 2009/0050156; US Patent Application Publication No. 2009/0044808.

US 2020/345963 A1 discloses: A patient interface to provide breathable gas to a patient, comprising: a plenum chamber assembly, comprising: a nasal plenum chamber at least partly defining an upper gas chamber; an oral plenum chamber at least partly defining a lower gas chamber; and a decoupling structure at least partly connecting the nasal plenum chamber and the oral plenum chamber and at least party defining a flow path; a top plate including at least one connection feature configured to releasably retain a first portion of a positioning and stabilising structure; and a faceplate configured to releasably retain a second portion of a positioning and stabilising structure.

WO 2020/191463 A1 discloses: A patient interface may include a plenum chamber and a positioning and stabilising structure. The plenum chamber may include a seal-forming structure and a fascia portion. At least a medial portion of the fascia portion is flexible. In embodiments, the patient interface may include a rigidiser to control flexing of the fascia portion.

US 2019/022343 A1 discloses: Aspects of the present technology comprise a positioning and stabilising structure to hold a seal-forming structure in a therapeutically effective position on a head of a patient. The positioning and stabilising structure may comprise at least one gas delivery tube to deliver the flow of air to the entrance of a patient's airways via the seal-forming structure. The at least one gas delivery tube may be constructed and arranged to contact, in use, at least a region of the patient's head superior to an otobasion superior of the patient's head. The positioning and stabilising structure may comprise an adjustment mechanism for adjustment of a length of the at least one gas delivery tube to enable the positioning and stabilising structure to fit different size heads. The positioning and stabilising structure may comprise a bias mechanism to impart a biasing force along at least a part of a length of the at least one gas delivery tube to urge the seal-forming structure towards the entrance of the patient's airways in use.

US 2015/246199 A1 discloses: A cushion for use in a patient interface device includes a first portion adapted to sealingly engage about the nares of a patient, a second portion adapted to sealingly engage about the mouth of the patient, and a third portion configured to allow for the relative positioning of the first portion and the second portion to be selectively adjusted.

### SUMMARY OF THE INVENTION

The present invention provides a patient interface as defined in claim 1. Further preferred embodiments of the present invention are defined in the dependent claims 2 to 14.

### 3 BRIEF SUMMARY OF THE TECHNOLOGY

Reference will now be made to various exemplary embodiments or aspects of the present technology. Embodiments or aspects disclosed herein which do not fall under the scope of the appended claims do not form part of the present invention, but are useful for understanding the principles of the invention. The scope of the present invention is defined by the appended claims. The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

An aspect of the present technology is directed to a device for controlling air flow out of a patient's mouth while receiving a flow of pressurized air through the patient's nares to treat a respiratory disorder, the device comprising: a flexible portion configured to leave the patient's nose uncovered during use, the flexible portion having an oral hole; a support portion connected to the flexible portion; a positioning and stabilizing structure configured to retain the flexible portion against the patient's face during use; a valve; and a vent.

Another aspect of the present technology is directed to a device for controlling air flow out of a patient's mouth while receiving a flow of pressurized air through the patient's nares to treat a respiratory disorder, the device comprising: a flexible portion configured to contact the patient's lip superior and lip inferior and leave the patient's nose uncovered during use, the flexible portion having an oral hole; a support portion connected to the flexible portion and configured to support the flexible portion against the patient's face during use; a pair of connectors, each of the connectors being positioned on a corresponding lateral side of the device; a positioning and stabilizing structure including a strap configured to be connected to each of the connectors to retain the flexible portion against the patient's face during use; a valve configured to allow the patient to orally inhale ambient air through the valve in the absence of the flow of pressurized air, and the valve being configured to prevent the flow of pressurized air from escaping to ambient through the valve if the patient's mouth is open while receiving the flow of pressurized air through the patient's nares; and a vent configured to allow a flow of air to pass to ambient continuously throughout the patient's respiratory cycle and independent of whether the patient's mouth is open.

In examples of any of the aspects of the preceding paragraphs: (a) the vent may be positioned on the support portion, (b) the vent may be positioned on the flexible portion, (c) the valve may include the vent, (d) the vent may comprise one or more holes that are configured to remain uncovered through the patient's respiratory cycle, (e) the vent may be configured to permit a vent flow to ambient equal to or less than approximately 5L/min at 12 cmH₂O of pressure within the device, (f) the valve may comprise a valve frame, a valve hole formed through the valve frame, and a valve flap, the valve flap being configured to be pulled away from the valve frame to open the valve hole by negative pressure within the device in response to oral inhalation by the patient, and the valve flap being configured to occlude the valve hole when pressure with the device is equal to or greater than ambient air pressure, (g) the pair of connectors may be positioned on the support portion, (h) the valve may be positioned on the support portion, (i) two valves may each be positioned on a corresponding lateral side of the device, (j) the flexible portion may be constructed from silicone rubber, (k) the support portion may be constructed from a more rigid material than the flexible portion, and/or (l) the support portion may be constructed from polycarbonate.

A patient interface may comprise: a plenum chamber pressurisable to a therapeutic pressure of at least 4 cmH2O above ambient air pressure by a flow of air at the therapeutic pressure for breathing by a patient; a seal-forming structure connected to the plenum chamber, the seal-forming structure being constructed and arranged to seal with a region of the patient's face that at least partly surrounds an entrance to the patient's nasal airways, the seal-forming structure having a hole therein to deliver the flow of air at the therapeutic pressure to the patient's nares during use, the seal-forming structure being constructed and arranged to maintain the therapeutic pressure within the plenum chamber throughout the patient's respiratory cycle in use, and the seal-forming structure being configured to leave the patient's mouth uncovered; a positioning and stabilising structure configured to hold the seal-forming structure in a therapeutically effective position on the patient's head; a vent assembly configured to allow a vent flow of exhaled gases to pass to atmosphere throughout the patient's respiratory cycle; and the device of any of the aspects or examples described in the preceding paragraphs.

In examples of the patient interface of the preceding paragraph: (a) the plenum chamber may comprise two plenum chamber connectors, each of the plenum chamber connectors being positioned on a corresponding lateral side of the plenum chamber and configured to receive the flow of air at the therapeutic pressure, and the positioning and stabilising structure may comprise two conduits and a central section between the conduits, each of the conduits being configured to be positioned on a corresponding lateral side of the patient's head in use and being configured to connect to a corresponding one of the plenum chamber connectors and/or (b) a patient interface connector may join the device to the plenum chamber.

An aspect of the present technology is directed to a device for controlling leak from a patient's mouth while receiving a flow of pressurized air through the patient's nares to treat a respiratory disorder, the device comprising: a flexible portion configured to leave the patient's nose uncovered during use, the flexible portion having a contacting surface configured to contact the patient, and the contacting surface surrounding an oral hole through the flexible portion; a support portion configured to support the flexible portion against the patient's face during use, and the support portion having an air flow hole; a positioning and stabilizing structure configured to retain the flexible portion against the patient's face during use; a porous structure covering the air flow hole.

Another aspect of the present technology is directed to a device for controlling leak from a patient's mouth while receiving a flow of pressurized air through the patient's nares to treat a respiratory disorder, the device comprising: a flexible portion configured to contact the patient's lip superior and lip inferior and leave the patient's nose uncovered during use, the flexible portion having a contacting surface configured to contact the patient, and the contacting surface surrounding an oral hole through the flexible portion; a support portion connected to the flexible portion and configured to support the flexible portion against the patient's face during use, the support portion having a pair of connectors, each of the connectors being positioned on a corresponding lateral side of the support portion, and the support portion having an air flow hole; a positioning and stabilizing structure including a strap configured to be connected to each of the connectors to retain the flexible portion against the patient's face during use; a porous structure covering the air flow hole and configured to restrict air flow into and out of the device during use, wherein the flexible portion has a thickness such that when the device is worn by the patient, the porous structure is offset from the contacting surface in an anterior direction relative to the patient.

In examples of any of the aspects of the preceding paragraphs: (a) the flexible portion may be shaped and dimensioned to allow the patient's upper vermilion, lower vermilion, and cheilions to fit into the oral hole during use, (b) the flexible portion may comprise foam, (c) the flexible portion may comprise textile, (d) the flexible portion may be constructed from a composite of textile and foam, (e) the support portion may be constructed from a more rigid material than the flexible portion, (f) the support portion may be constructed from silicone rubber, and/or (g) the air flow hole and the porous structure may be configured to permit a vent flow to ambient equal to or less than approximately 5L/min at 12 cmH2O of pressure within the device.

A patient interface may comprise: a plenum chamber pressurisable to a therapeutic pressure of at least 4 cmH2O above ambient air pressure by a flow of air at the therapeutic pressure for breathing by a patient; a seal-forming structure connected to the plenum chamber, the seal-forming structure being constructed and arranged to seal with a region of the patient's face that at least partly surrounds an entrance to the patient's nasal airways, the seal-forming structure having a hole therein to deliver the flow of air at the therapeutic pressure to the patient's nares during use, the seal-forming structure being constructed and arranged to maintain the therapeutic pressure within the plenum chamber throughout the patient's respiratory cycle in use, and the seal-forming structure being configured to leave the patient's mouth uncovered; a positioning and stabilising structure configured to hold the seal-forming structure in a therapeutically effective position on the patient's head; a vent assembly configured to allow a vent flow of exhaled gases to pass to atmosphere throughout the patient's respiratory cycle; and the device of any of the aspects or examples described in the preceding paragraphs.

In examples of the patient interface of the preceding paragraph: (a) the plenum chamber may comprise two plenum chamber connectors, each of the plenum chamber connectors being positioned on a corresponding lateral side of the plenum chamber and configured to receive the flow of air at the therapeutic pressure, and the positioning and stabilising structure may comprise two conduits and a central section between the conduits, each of the conduits being configured to be positioned on a corresponding lateral side of the patient's head in use and being configured to connect to a corresponding one of the plenum chamber connectors and/or (b) a patient interface connector may join the device to the plenum chamber.

One form of the present technology comprises a passageway formed between a nasal cavity and an oral cavity and configured to provide fluid communication between the oral and nasal cavities.

One form of the present technology comprises a decoupling structure of a patient interface connecting a nasal portion to an oral portion.

Another aspect of one form of the present technology is a decoupling structure that decouples forces applied to one portion of the plenum chamber form influencing another portion of the plenum chamber.

Another aspect of one form of the present technology is a decoupling portion that allows pivotable movement between an oral portion and a nasal portion of a patient interface so that forces applied by a positioning and stabilizing structure to the oral portion do not substantially affect the nasal portion.

Another aspect of one form of the present technology is an airflow path formed between a nasal cavity and an oral cavity and configured to allow the flow of pressurized breathable gas between the nasal and oral cavities, and wherein a decoupling structure forms at least a portion of a perimeter of the airflow path and is configured to pivot in order to decouple forces applied to the oral portion from substantially affecting the nasal portion.

One aspect of the present technology comprises a patient interface for sealed delivery of a flow of air at a continuously positive pressure with respect to ambient air pressure to an entrance to a patient's airways including at least an entrance of a patient's nares, wherein the patient interface is configured to maintain a therapy pressure in a range of about 4 cmH2O to about 30 cmH2O above ambient air pressure in use, throughout a patient's respiratory cycle, while the patient is sleeping, to ameliorate sleep disordered breathing.

Another aspect of the present technology comprises a patient interface comprising:
a plenum chamber at least partially forming a cavity pressurisable to a therapeutic pressure of at least 4 cmH2O above ambient air pressure; and
a seal-forming structure constructed and arranged to form a seal with a region of the patient's face surrounding an entrance to the patient's nares and/or the patient's mouth.

Another aspect of the present technology comprise a patient interface comprising:
a plenum chamber at least partially forming a cavity pressurisable to a therapeutic pressure of at least 4 cmH2O above ambient air pressure, the plenum chamber including at least one plenum chamber inlet port configured to convey the flow of air into the plenum chamber; and
a seal-forming structure constructed and arranged to form a seal with a region of the patient's face surrounding an entrance to the patient's mouth and/or the patient's nares, said seal-forming structure having a hole therein such that the flow of air at said therapeutic pressure is delivered to at least the entrance to the patient's mouth and/or the patient's nares, the seal-forming structure constructed and arranged to maintain said therapeutic pressure in the plenum chamber throughout the patient's respiratory cycle in use.

Another aspect of the present technology comprises a patient interface comprising:
a cushion comprising,
a nasal portion comprising:
   a nasal plenum chamber at least partially forming a nasal cavity pressurisable to a therapeutic pressure of at least 4 cmH2O above ambient air pressure, the nasal plenum chamber including at least one plenum chamber inlet port configured to convey the flow of air into the nasal plenum chamber; and
   a nasal seal-forming structure constructed and arranged to form a seal with a region of the patient's face surrounding an entrance to the patient's nares, said nasal seal-forming structure having a hole therein such that the flow of air at said therapeutic pressure is delivered to at least an entrance to the patient's nares, the nasal seal-forming structure constructed and arranged to maintain said therapeutic pressure in the nasal plenum chamber throughout the patient's respiratory cycle in use;
a oral portion comprising:
   an oral plenum chamber at least partially forming an oral cavity pressurisable above ambient air pressure; and
   an oral seal-forming structure constructed and arranged to form a seal with a region of the patient's face surrounding an entrance to the patient's mouth, said oral seal-forming structure having a hole therein such that air in the oral cavity is delivered to at least an entrance to the patient's mouth, the oral seal-forming structure constructed and arranged to maintain said therapeutic pressure in the oral plenum chamber throughout the patient's respiratory cycle in use;
a positioning and stabilising structure to provide a force to hold the nasal seal-forming structure and the oral seal-forming structure in a therapeutically effective position on the patient's head; and
a vent structure connected to the nasal plenum chamber and configured to allow a continuous flow of gases exhaled by the patient from an interior of the nasal plenum chamber to ambient, said vent structure being sized and shaped to maintain the therapeutic pressure in the nasal plenum chamber in use;
wherein the patient interface is configured to allow the patient to breath from ambient through their mouth in the absence of a flow of pressurised air through the plenum chamber inlet port;
wherein the nasal portion is permanently connected to the oral portion with a decoupling portion formed between the nasal plenum chamber and the oral plenum chamber, the decoupling portion including an angled portion extending toward the nasal seal-forming structure and the oral seal-forming structure, the decoupling portion configured to allow the nasal portion to pivot relative to the oral portion; and
wherein a curved surface opposite to the decoupling portion connects the nasal seal-forming structure and the oral seal-forming structure.

In some forms: a) the nasal seal-forming structure is configured to contact the patient's columella and is not configured to contact the patient's nasal ridge superior to the patient's pronasale; b) the curved surface forms a continuous surface between the nasal seal-forming structure and the oral seal forming structure; c) the curved surface is configured stretch toward a planar orientation while worn by the patient; and/or d) the curved surface is configured to not seal against the patient's face.

In some forms: a) the at least one plenum chamber inlet port includes a pair of plenum chamber inlet ports; b) each of the at least one plenum chamber inlet ports is positioned at a lateral side of the nasal plenum chamber; c) conduits connect to the pair of plenum chamber inlet ports; d) the conduits are configured to convey the flow of air through each plenum chamber inlet port of the pair of plenum chamber inlet ports; e) the conduits form a portion of the positioning and stabilizing structure and are configured to provide a tensile force to the nasal portion in use; and/or f) the conduits include concertina sections configured to expand upon application of a tensile force.

In some forms: a) the at least one plenum chamber inlet port is a single plenum chamber inlet port disposed substantially centrally in the nasal portion; b) the nasal portion further includes a pair of loops; c) each loop of the pair of loops is positioned at a lateral side of the nasal portion; and/or d) upper straps of the positioning and stabilizing structure are configured to wrap around the pair of loops.

In some forms: a) the oral portion includes a pair of connectors spaced apart from one another; b) the pair of connectors are magnets; c) the oral portion is molded around the pair of connectors; d) the pair of connectors are selectively removable from the oral portion; e) an insert includes the pair of connectors; f) the insert is selectively coupled to the oral plenum chamber with a press fit, friction fit, and/or snap fit; g) the insert includes an anti-asphyxia valve (AAV); h) the AAV is biased toward a closed position and is configured to open when the patient inhales through his mouth; and/or i) the positioning and stabilizing structure includes lower straps selectively connected to the oral portion via the connectors.

In some forms: a) the decoupling portion is configured to at least partially decouple a force applied by the positioning and stabilizing structure to the oral portion from the nasal portion; b) an angle of the decoupling portion between the oral portion and the nasal portion in a relaxed position is about 1° to about 90°; c) the angle is about 10° to about 50° in the relaxed position; and/or d) the angle is about 20° to about 40° in the relaxed position.

In some aspects: a) a passage is formed between the nasal cavity and the oral cavity; b) the passage is configured to provide fluid communication between the nasal cavity and the oral cavity; c) the passage is a substantially elliptical shape; and/or d) the passage is substantially aligned with the decoupling portion and the curved surface.

In some aspects: a) a membrane formed between the nasal cavity and the oral cavity; b) the membrane extends between the decoupling portion and the curved surface; c) the membrane is slack before the patient interface contacts the patient; d) the membrane is substantially solid and is configured to prevent airflow from traveling within the cushion between the nasal cavity and the oral cavity; e) the membrane is configured to allow airflow from traveling within the cushion between the nasal cavity and the oral cavity through at least one hole; f) the at least one hole is a plurality of spaced apart holes; g) the plurality of spaced apart holes are sized to allow a predetermined flow of air between the nasal cavity and the oral cavity; and/or h) the membrane includes a porosity on a microscopic and/or molecular level that is configured to allow a predetermined flow of air traveling within the cushion to flow between the nasal cavity and the oral cavity.

Another aspect of the present technology comprises a patient interface comprising:
a cushion comprising,
a nasal portion comprising:
   a nasal plenum chamber at least partially forming a nasal cavity pressurisable to a therapeutic pressure of at least 4 cmH2O above ambient air pressure, the nasal plenum chamber including a pair of plenum chamber inlet ports configured to convey the flow of air into the nasal plenum chamber; and
   a nasal seal-forming structure constructed and arranged to form a seal with a region of the patient's face surrounding an entrance to the patient's nares, said nasal seal-forming structure having a hole therein such that the flow of air at said therapeutic pressure is delivered to at least an entrance to the patient's nares, the nasal seal-forming structure constructed and arranged to maintain said therapeutic pressure in the nasal plenum chamber throughout the patient's respiratory cycle in use;
a oral portion comprising:
   an oral plenum chamber at least partially forming an oral cavity pressurisable to a therapeutic pressure of at least 4 cmH2O above ambient air pressure, the oral plenum chamber having at least one connection point; and
   an oral seal-forming structure constructed and arranged to form a seal with a region of the patient's face surrounding an entrance to the patient's mouth, said oral seal-forming structure having a hole therein such that the flow of air at said therapeutic pressure is delivered to at least an entrance to the patient's mouth, the oral seal-forming structure constructed and arranged to maintain said therapeutic pressure in the oral plenum chamber throughout the patient's respiratory cycle in use;
a positioning and stabilising structure to provide a force to hold the nasal seal-forming structure and the oral seal-forming structure in a therapeutically effective position on the patient's head, the positioning and stabilizing structure comprising:
   a pair of conduits removably connected to the pair of plenum chamber inlet ports, the pair of conduits configured to convey the flow of air to the nasal plenum chamber; and
   headgear straps removably connected to the connection point;
a vent structure connected to the nasal plenum chamber and configured to allow a continuous flow of gases exhaled by the patient from an interior of the nasal plenum chamber to ambient, said vent structure being sized and shaped to maintain the therapeutic pressure in the nasal plenum chamber in use;
wherein the patient interface is configured to allow the patient to breath from ambient through their mouth in the absence of a flow of pressurised air through the pair of plenum chamber inlet ports;
wherein the nasal portion is permanently connected to the oral portion with a decoupling portion formed between the nasal plenum chamber and the oral plenum chamber, the decoupling portion including an angled portion extending toward the nasal seal-forming structure and the oral seal-forming structure, the decoupling portion configured to allow the nasal portion to pivot relative to the oral portion, the decoupling portion configured to limit application of the force from the pair of conduits on the oral portion, and decoupling portion configured to limit application of the force from the headgear straps on the nasal portion;
wherein a curved surface opposite to the decoupling portion connects the nasal seal-forming structure and the oral seal-forming structure; and
wherein a flow path is formed between the decoupling portion and the curved surface to fluidly connect the nasal plenum chamber to the oral plenum chamber.

In some forms: a) the nasal seal-forming structure is configured to contact the patient's columella and is not configured to contact the patient's nasal ridge superior to the patient's pronasale; b) the curved surface forms a continuous surface between the nasal seal-forming structure and the oral seal forming structure; c) the curved surface is configured stretch toward a planar orientation while worn by the patient; and/or d) the curved surface is configured to not seal against the patient's face.

In some forms: a) the conduits include concertina sections configured to expand upon application of a tensile force; b) the at least one connection point is a pair of spaced apart connection points; c) the pair of connection points are magnets; d) the oral portion is molded around the pair of connection points; e) the pair of connection points are selectively removable from the oral portion; f) an insert including the pair of connection points; g) the insert being selectively coupled to the oral plenum chamber with a press fit, friction fit, and/or snap fit; and/or h) the insert includes an anti-asphyxia valve (AAV); and/or i) the AAV is biased toward a closed position and is configured to open when the patient inhales through his mouth.

In some forms: a) the decoupling portion is configured to at least partially decouple a force applied by the positioning and stabilizing structure to the oral portion from the nasal portion; b) an angle of the decoupling portion between the oral portion and the nasal portion in a relaxed position is about 1° to about 90°; c) the angle is about 10° to about 50° in the relaxed position; and/or d) the angle is about 20° to about 40° in the relaxed position.

In some forms: a) a passage formed between the nasal cavity and the oral cavity; b) the passage configured to provide fluid communication between the nasal cavity and the oral cavity and the flow path flowing through the passage; c) the passage is a substantially elliptical shape; and/or d) the passage is substantially aligned with the decoupling portion and the curved surface.

In some forms: a) comprising a membrane formed between the nasal cavity and the oral cavity; b) the membrane extends between the decoupling portion and the curved surface; c) the membrane is slack before the patient interface contacts the patient; d) tension of the membrane changes as a result of contact with the patient; e) the membrane is configured to allow airflow from traveling within the cushion between the nasal cavity and the oral cavity through at least one hole; f) the at least one hole is a plurality of spaced apart holes; g) the plurality of spaced apart holes are sized to allow a predetermined flow of air between the nasal cavity and the oral cavity; and/or h) the membrane includes a porosity on a microscopic and/or molecular level that is configured to allow a predetermined flow of air traveling within the cushion to flow between the nasal cavity and the oral cavity along the flow path.

In some forms: a) each conduit of the pair of conduits is configured to contact the patient in a space between an ear and an eye and overlay the patient's temporal bone and/or the sphenoid bone; b) each conduit of the pair of conduits is configured to contact a superior portion of the patient's head and overlay the frontal bone and/or the parietal bone; c) the vent structure is removably connected to the nasal portion; d) the nasal plenum chamber includes a vent receiving region recessed into the nasal plenum chamber; and/or e) the vent structure is configured to be substantially flush with a surrounding surface of the nasal plenum chamber when removably positioned within the vent receiving region.

Another aspect of the present technology comprises a patient interface comprising:
a cushion comprising,
a nasal portion comprising:
   a nasal plenum chamber at least partially forming a nasal cavity pressurisable to a therapeutic pressure of at least 4 cmH2O above ambient air pressure, the nasal plenum chamber including a pair of plenum chamber inlet ports configured to convey the flow of air into the nasal plenum chamber; and
   a nasal seal-forming structure constructed and arranged to form a seal with a region of the patient's face surrounding an entrance to the patient's nares, said nasal seal-forming structure having a hole therein such that the flow of air at said therapeutic pressure is delivered to at least an entrance to the patient's nares, the nasal seal-forming structure constructed and arranged to maintain said therapeutic pressure in the nasal plenum chamber throughout the patient's respiratory cycle in use;
an oral portion comprising:
   an oral plenum chamber at least partially forming an oral cavity, the oral plenum chamber having at least one connection point; and
   an oral seal-forming structure constructed and arranged to form a seal with a region of the patient's face surrounding an entrance to the patient's mouth, said oral seal-forming structure having a hole therein such that the flow of air at said therapeutic pressure is delivered to at least an entrance to the patient's mouth, the oral seal-forming structure constructed and arranged to maintain said therapeutic pressure in the oral plenum chamber throughout the patient's respiratory cycle in use;
a positioning and stabilising structure to provide a force to hold the nasal seal-forming structure and the oral seal-forming structure in a therapeutically effective position on the patient's head, the positioning and stabilizing structure comprising:
   a pair of conduits removably connected to the pair of plenum chamber inlet ports, the pair of conduits configured to convey the flow of air to the nasal plenum chamber; and
   headgear straps removably connected to the connection point;
a vent structure connected to the nasal plenum chamber and configured to allow a continuous flow of gases exhaled by the patient from an interior of the nasal plenum chamber to ambient, said vent structure being sized and shaped to maintain the therapeutic pressure in the nasal plenum chamber in use;
wherein the patient interface is configured to allow the patient to breath from ambient through their mouth in the absence of a flow of pressurised air through the pair of plenum chamber inlet ports;
wherein the nasal portion is permanently connected to the oral portion with a decoupling portion formed between the nasal plenum chamber and the oral plenum chamber, the decoupling portion including an angled portion extending toward the nasal seal-forming structure and the oral seal-forming structure, the decoupling portion configured to allow the nasal portion to pivot relative to the oral portion, the decoupling portion configured to limit application of the force from the pair of conduits on the oral portion, and decoupling portion configured to limit application of the force from the headgear straps on the nasal portion;
wherein a curved surface opposite to the decoupling portion connects the nasal seal-forming structure and the oral seal-forming structure; and
wherein a membrane extends between the decoupling portion and the curved surface to block fluid connection from the nasal plenum chamber to the oral plenum chamber.

In some forms: a) the nasal seal-forming structure is configured to contact the patient's columella and is not configured to contact the patient's nasal ridge superior to the patient's pronasale; b) the curved surface forms a continuous surface between the nasal seal-forming structure and the oral seal forming structure; c) the curved surface is configured stretch toward a planar orientation while worn by the patient; and/or d) the curved surface is configured to not seal against the patient's face.

In some forms: a) the conduits include concertina sections configured to expand upon application of a tensile force; b) the at least one connection point is a pair of spaced apart connection points; c) the pair of connection points are magnets; d) the oral portion is molded around the pair of connection points; e) the oral portion includes vent configured to allow the patient to breathe through his mouth in the absence of a flow of pressurised air through the pair of plenum chamber inlet ports; f) the pair of connection points are selectively removable from the oral portion; g) an insert includes the pair of connection points; h) the insert is selectively coupled to the oral plenum chamber with a press fit, friction fit, and/or snap fit; and/or i) the insert includes an anti-asphyxia valve (AAV); and/or j) the AAV is biased toward a closed position and is configured to open when the patient inhales through his mouth.

**In** some forms: a) the decoupling portion is configured to at least partially decouple a force applied by the positioning and stabilizing structure to the oral portion from the nasal portion; b) an angle of the decoupling portion between the oral portion and the nasal portion in a relaxed position is about 1° to about 90°; c) the angle is about 10° to about 50° in the relaxed position; and/or d) the angle is about 20° to about 40° in the relaxed position.

In some forms: a) the membrane is slack before the patient interface contacts the patient; b) tension in the membrane changes as a result of contact with the patient's face; c) a thickness of the membrane is about 0.1 mm to about 1 mm; d) the thickness of the membrane is about 0.35 mm; and/or e) the membrane is constructed from a different material than the oral plenum chamber and the nasal plenum chamber.

Another aspect of the present technology comprises a patient interface comprising:
a cushion comprising,
a nasal portion comprising:
   a nasal plenum chamber at least partially forming a nasal cavity pressurisable to a therapeutic pressure of at least 4 cmH2O above ambient air pressure, the nasal plenum chamber including a plenum chamber inlet port that is configured to convey the flow of air into the nasal plenum chamber; and
   a nasal seal-forming structure constructed and arranged to form a seal with a region of the patient's face surrounding an entrance to the patient's mouth, said nasal seal-forming structure having a hole therein such that the flow of air at said therapeutic pressure is delivered to at least an entrance to the patient's mouth, the nasal seal-forming structure constructed and arranged to maintain said therapeutic pressure in the nasal plenum chamber throughout the patient's respiratory cycle in use;
an oral portion comprising:
   an oral plenum chamber at least partially forming an oral cavity pressurisable to a therapeutic pressure of at least 4 cmH2O above ambient air pressure, the oral plenum chamber having at least one connection point; and
   an oral seal-forming structure constructed and arranged to form a seal with a region of the patient's face surrounding an entrance to the patient's mouth, said oral seal-forming structure having a hole therein such that the flow of air at said therapeutic pressure is delivered to at least an entrance to the patient's mouth, the oral seal-forming structure constructed and arranged to maintain said therapeutic pressure in the oral plenum chamber throughout the patient's respiratory cycle in use;
a positioning and stabilising structure to provide a force to hold the nasal seal-forming structure and the oral seal-forming structure in a therapeutically effective position on the patient's head, the positioning and stabilizing structure comprising:
   a conduit removably connected to the plenum chamber inlet port, the conduit configured to convey the flow of air to the nasal plenum chamber;
   upper headgear straps removably connected to the nasal portion; and
   lower headgear straps removably connected to the connection point;
a vent structure connected to the nasal plenum chamber and configured to allow a continuous flow of gases exhaled by the patient from an interior of the nasal plenum chamber to ambient, said vent structure being sized and shaped to maintain the therapeutic pressure in the nasal plenum chamber in use;
wherein the patient interface is configured to allow the patient to breath from ambient through their mouth in the absence of a flow of pressurised air through the plenum chamber inlet port;
wherein the nasal portion is permanently connected to the oral portion with a decoupling portion formed between the nasal plenum chamber and the oral plenum chamber, the decoupling portion including an angled portion extending toward the nasal seal-forming structure and the oral seal-forming structure, the decoupling portion configured to allow the nasal portion to pivot relative to the oral portion, the decoupling portion configured to limit application of the force from the upper headgear straps on the oral portion, and decoupling portion configured to limit application of the force from the lower headgear straps on the nasal portion;
wherein a curved surface opposite to the decoupling portion connects the nasal seal-forming structure and the oral seal-forming structure; and
wherein a flow path is formed between the decoupling portion and the curved surface to fluidly connect the nasal plenum chamber to the oral plenum chamber.

In some forms: a) the nasal seal-forming structure is configured to contact the patient's columella and is not configured to contact the patient's nasal ridge superior to the patient's pronasale; b) the curved surface forms a continuous surface between the nasal seal-forming structure and the oral seal forming structure; c) the curved surface is configured stretch toward a planar orientation while worn by the patient; and/or d) the curved surface is configured to not seal against the patient's face.

In some forms: a) the nasal portion further includes a pair of loops; b) each loop of the pair of loops is positioned at a lateral side of the nasal portion; c) the upper headgear straps of the positioning and stabilizing structure are configured to wrap around the pair of loops; d) the oral portion includes a pair of connectors spaced apart from one another; e) the pair of connectors are magnets; f) the oral portion is molded around the pair of connectors; g) the pair of connectors are selectively removable from the oral portion; h) an insert includes the pair of connectors; i) the insert is selectively coupled to the oral plenum chamber with a press fit, friction fit, and/or snap fit; j) the insert includes an anti-asphyxia valve (AAV); and/or k) the AAV is biased toward a closed position and is configured to open when the patient inhales through his mouth.

In some forms: a) the decoupling portion is configured to at least partially decouple a force applied by the positioning and stabilizing structure to the oral portion from the nasal portion; b) an angle of the decoupling portion between the oral portion and the nasal portion in a relaxed position is about 1° to about 90°; c) the angle is about 10° to about 50° in the relaxed position; and/or d) the angle is about 20° to about 40° in the relaxed position.

In some forms: a) a passage formed between the nasal cavity and the oral cavity, the passage configured to provide fluid communication between the nasal cavity and the oral cavity; b) the passage is a substantially elliptical shape; and/or c) the passage is substantially aligned with the decoupling portion and the curved surface.

In some forms: a) a membrane formed between the nasal cavity and the oral cavity; b) the membrane extends between the decoupling portion and the curved surface; c) the membrane is slack before the patient interface contacts the patient; d) the membrane is configured to allow airflow from traveling within the cushion between the nasal cavity and the oral cavity through at least one hole; e) the at least one hole is a plurality of spaced apart holes; f) the plurality of spaced apart holes are sized to allow a predetermined flow of air between the nasal cavity and the oral cavity; and/or g) the membrane includes a porosity on a microscopic and/or molecular level that is configured to allow a predetermined flow of air traveling within the cushion to flow between the nasal cavity and the oral cavity.

Another aspect of the present technology comprises a patient interface comprising:
a cushion comprising,
a nares portion comprising:
   an nares plenum chamber at least partially forming a cavity pressurisable to a therapeutic pressure of at least 4 cmH2O above ambient air pressure, the nares plenum chamber including a pair of plenum chamber inlet ports configured to convey the flow of air into the nares plenum chamber; and
   an nares seal-forming structure constructed and arranged to form a seal with a region of the patient's face surrounding an entrance to the patient's mouth, said nares seal-forming structure having a hole therein such that the flow of air at said therapeutic pressure is delivered to at least an entrance to the patient's mouth, the nares seal-forming structure constructed and arranged to maintain said therapeutic pressure in the nares plenum chamber throughout the patient's respiratory cycle in use;
an oral portion comprising:
   an oral plenum chamber at least partially forming a cavity pressurisable to a therapeutic pressure of at least 4 cmH2O above ambient air pressure, the oral plenum chamber having at least one connection point; and
   an oral seal-forming structure constructed and arranged to form a seal with a region of the patient's face surrounding an entrance to the patient's mouth, said oral seal-forming structure having a hole therein such that the flow of air at said therapeutic pressure is delivered to at least an entrance to the patient's mouth, the oral seal-forming structure constructed and arranged to maintain said therapeutic pressure in the oral plenum chamber throughout the patient's respiratory cycle in use;

a positioning and stabilising structure to provide a force to hold the nares seal-forming structure and the oral seal-forming structure in a therapeutically effective position on the patient's head, the positioning and stabilizing structure comprising:
   a pair of conduits removably connected to the pair of plenum chamber inlet ports, the pair of conduits configured to convey the flow of air to the nares plenum chamber; and
   headgear straps removably connected to the connection point;
a vent structure connected to the nares plenum chamber and configured to allow a continuous flow of gases exhaled by the patient from an interior of the nares plenum chamber to ambient, said vent structure being sized and shaped to maintain the therapeutic pressure in the nares plenum chamber in use;
wherein the patient interface is configured to allow the patient to breath from ambient through their mouth in the absence of a flow of pressurised air through the pair of plenum chamber inlet ports, or the patient interface is configured to leave the patient's mouth uncovered;
wherein the nares portion is permanently connected to the oral portion with a decoupling portion formed between the nares plenum chamber and the oral plenum chamber, the decoupling portion including an angled portion extending toward the nares seal-forming structure and the oral seal-forming structure, the decoupling portion configured to allow the nares portion to pivot relative to the oral portion, the decoupling portion configured to limit application of the force from the pair of conduits on the oral portion, and decoupling portion configured to limit application of the force from the headgear straps on the nares portion;
wherein a curved surface opposite to the decoupling portion connects the nares seal-forming portion and the oral seal-forming portion; and
wherein a flow path is formed between the decoupling portion and the curved surface to fluidly connect the nares plenum chamber to the oral plenum chamber.

Another aspect is a method of transitioning a patient receiving respiratory therapy from breathing through their mouth to breathing exclusively through their nose while receiving the therapy, the method comprising:
providing a first patient interface and a second patient interface, each of the first patient interface and the second patient interface configured to form a seal with a region of the patient's face surrounding an entrance to the patient's mouth and the patient's nares, both the first patient interface and the second patient interface include a nasal cavity of pressurisable to a therapeutic pressure above ambient air pressure and an oral cavity;
applying the therapeutic pressure to the first patient interface, the patient configured to inhale the therapeutic pressure through either their nose or their mouth;
monitoring a level of inhalation by the patient through their mouth;
applying the therapeutic pressure to the second patient interface, the patient configured to inhale the therapeutic pressure through only their nose.

In some forms: a) the first patient interface may include a passage between the nasal cavity and the oral cavity; and/or b) the cavity configured to provide fluid communication between the nasal cavity and the oral cavity.

In some forms: a) the first patient interface includes a membrane dividing the oral cavity and the nasal cavity that is configured to allow for fluid flow there between; b) the membrane is a permeable membrane; and/or c) the membrane includes holes configured to allow for fluid flow between the nasal cavity and the oral cavity.

In some forms: a) the second patient interface includes a membrane dividing the oral cavity and the nasal cavity that is configured to prevent the flow of fluid there between; and/or b) the membrane is impermeable.

In some forms: a) the method further includes recommending that the patient switch between the first patient interface and the second patient interface; b) recommending is done by a flow generator based on the monitoring; and/or c) recommending is a signal sent by a clinician as a result of the monitoring.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Other features of the technology will be apparent from consideration of the information contained in the following detailed description and drawings.

### 4 BRIEF DESCRIPTION OF THE DRAWINGS

The present technology is illustrated by way of example, and not by way of limitation, in the figures of the accompanying drawings, in which like reference numerals refer to similar elements including:

### 4.1 RESPIRATORY THERAPY SYSTEMS

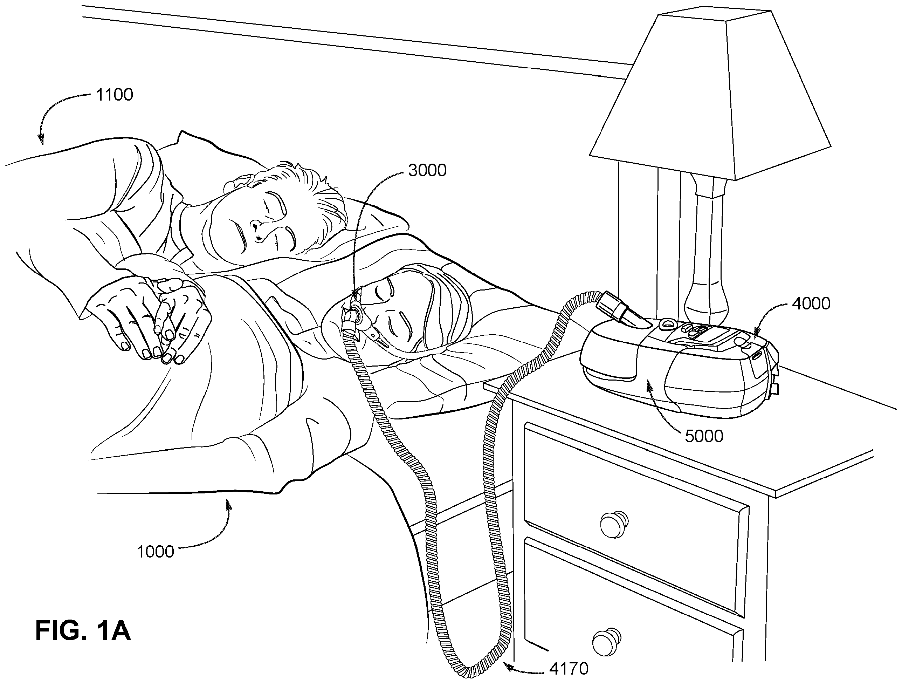

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

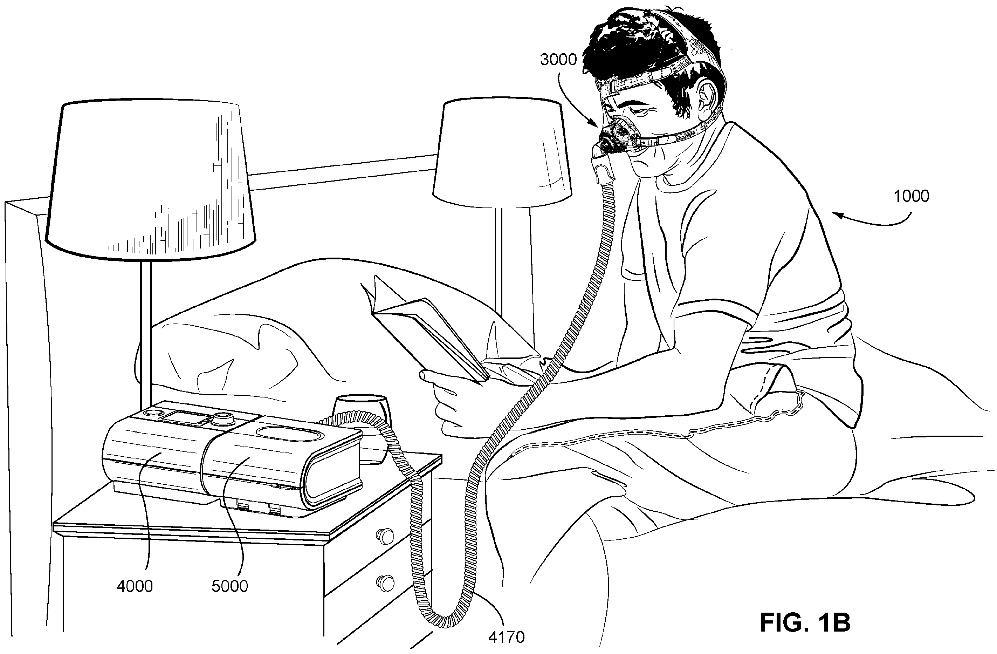

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

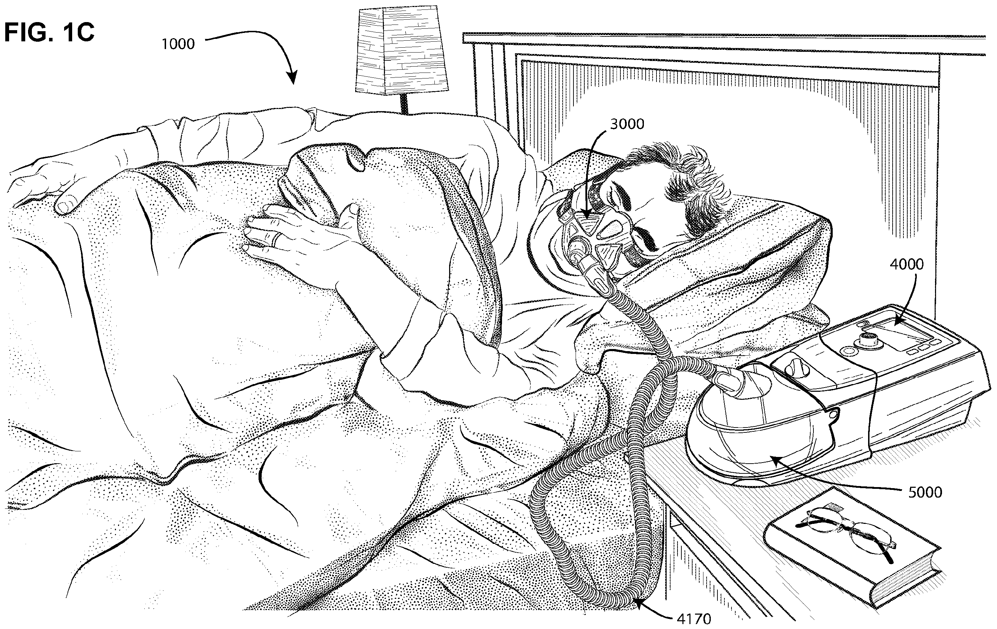

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

Fig. 2A shows an overview of a human respiratory system including the nasal and oral cavities, the larynx, vocal folds, oesophagus, trachea, bronchus, lung, alveolar sacs, heart and diaphragm.
Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.
Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.
Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.
Fig. 2E is a further side view of a head. The approximate locations of the Frankfort horizontal and nasolabial angle are indicated. The coronal plane is also indicated.
Fig. 2F shows a base view of a nose with several features identified including naso-labial sulcus, lip inferior, upper Vermilion, naris, subnasale, columella, pronasale, the major axis of a naris and the midsagittal plane.
Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.
Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.
Fig. 2L shows an anterolateral view of a nose.

### 4.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

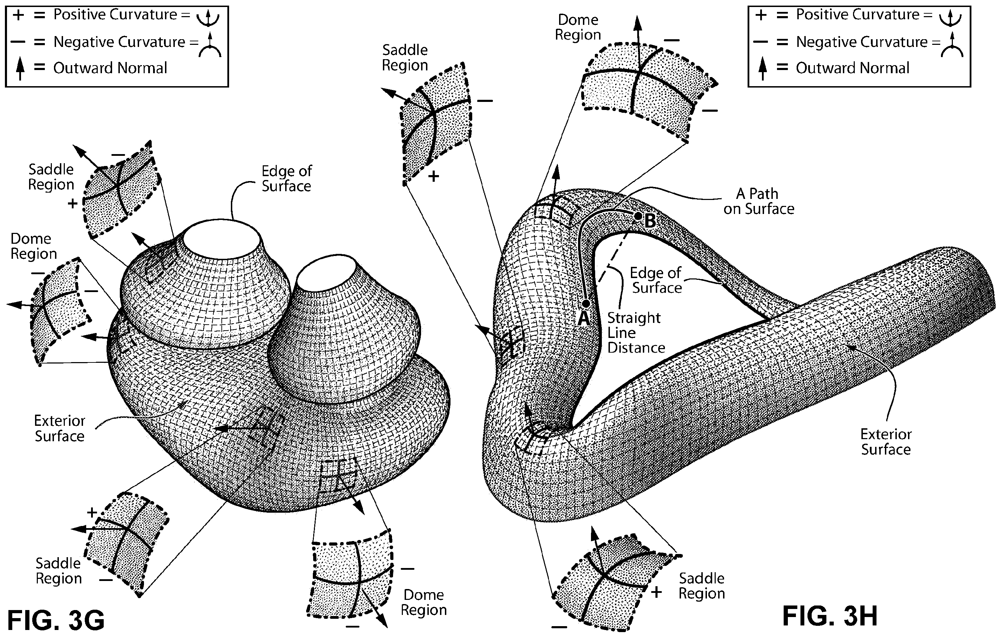

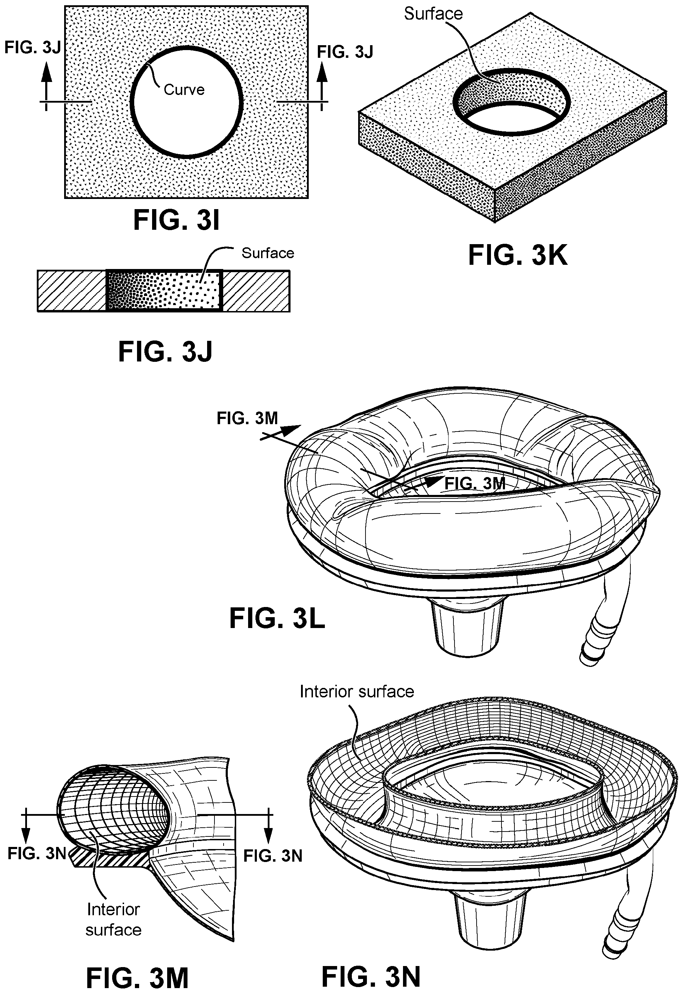

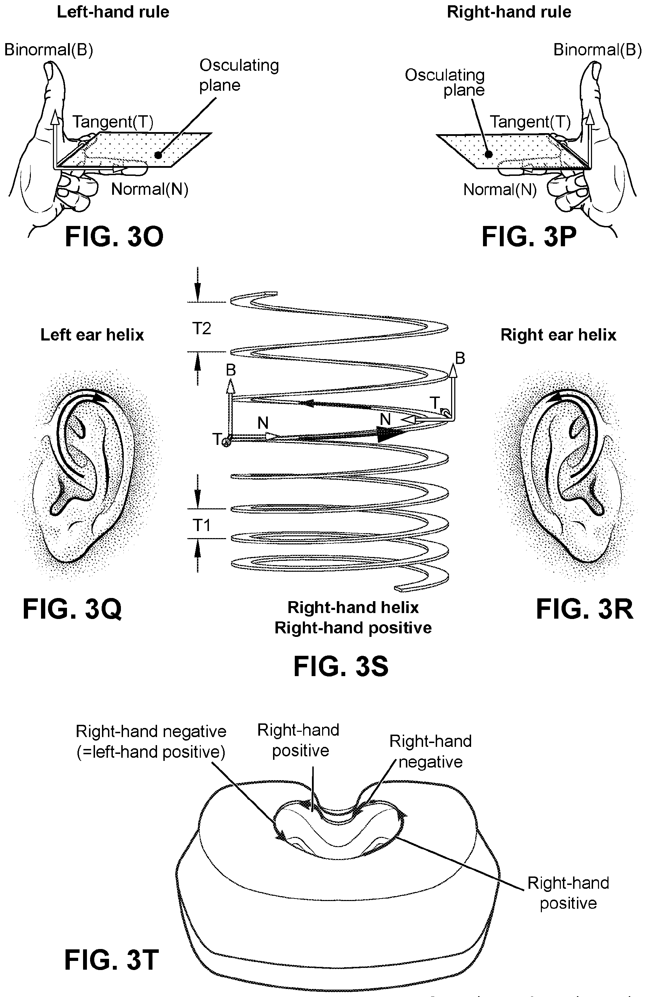

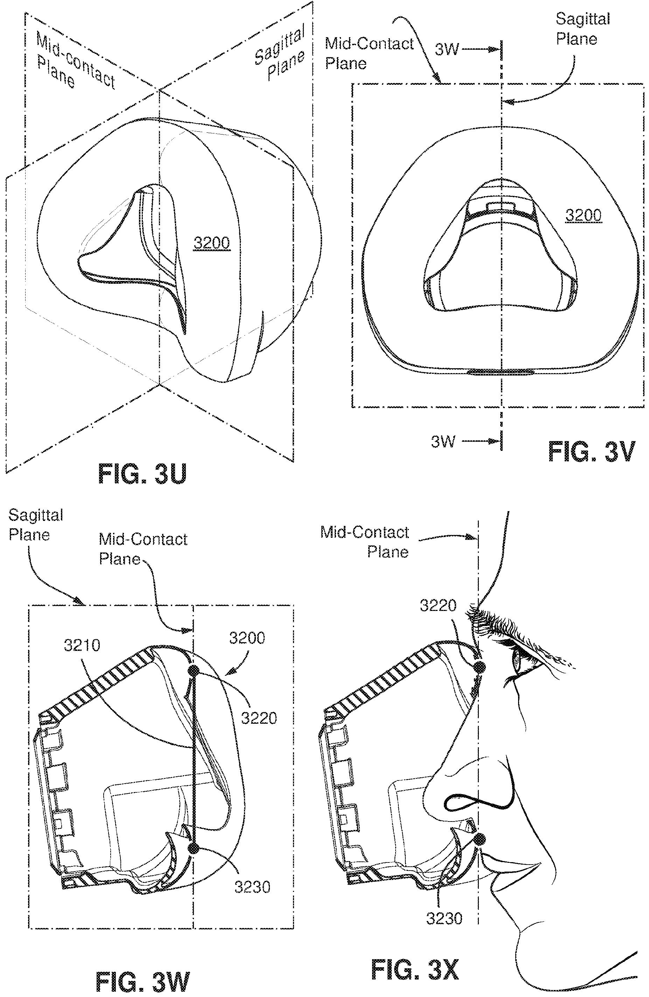

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

### 4.4 RPT DEVICE

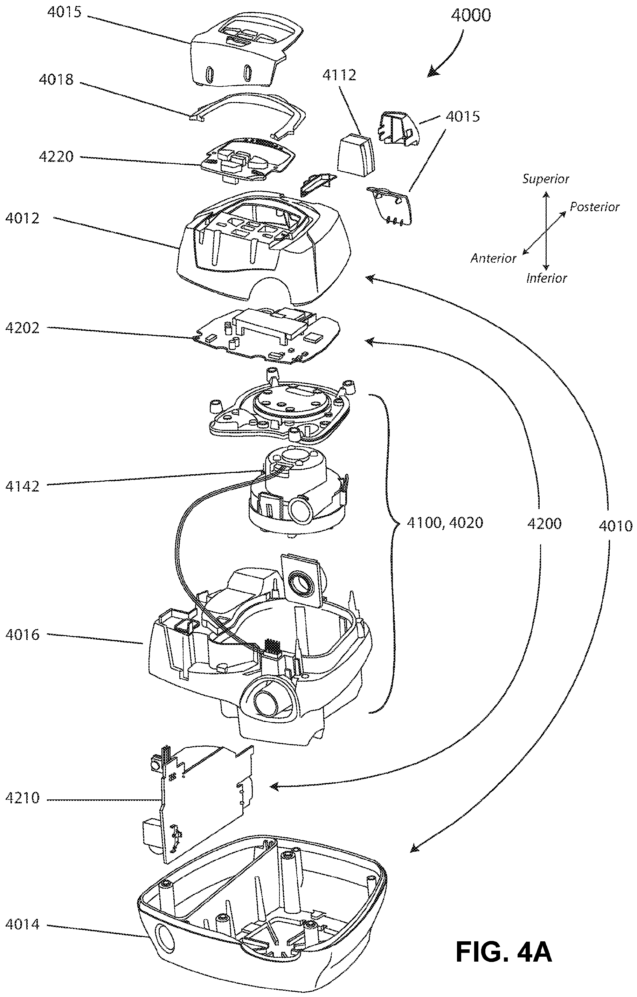

Fig. 4A shows an RPT device in accordance with one form of the present technology.

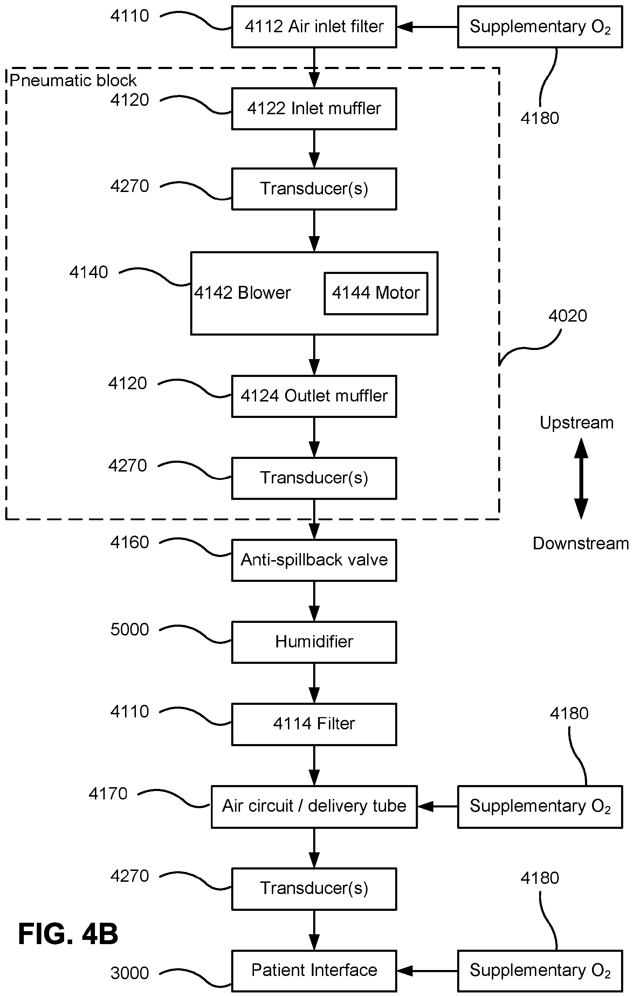

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 4.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 4.6 BREATHING WAVEFORMS

Fig. 6 shows a model typical breath waveform of a person while sleeping.

### 4.7 MOUTH LEAK CONTROLLING DEVICE

Fig. 7 shows a front view of a patient wearing a patient interface and a mouth leak controlling device according to an example of the present technology.
Fig. 8 shows a front view of a patient wearing a mouth leak controlling device according to an example of the present technology.
Fig. 9A shows a front view of a mouth leak controlling device according to an example of the present technology.
Fig. 9B shows a rear view of a mouth leak controlling device according to an example of the present technology.
Fig. 10A shows a front view of a mouth leak controlling device according to another example of the present technology.
Fig. 10B shows a rear view of a mouth leak controlling device according to another example of the present technology.
Fig. 11A shows a front view of a patient wearing a patient interface and a mouth leak controlling device according to another example of the present technology.
Fig. 11B shows a front view of a mouth leak controlling device according to another example of the present technology.
Fig. 11C shows a front view of a mouth leak controlling device according to another example of the present technology.

### 4.8 DECOUPLING

Fig. 12 shows a front view of a patient wearing one form of a patient interface for sealing around the patient's nares and around the patient's mouth, and illustrating the force vectors when the patient is in an upright position.
Fig. 12-1 is a front view of a patient interface according to Fig. 12, and illustrating the force vectors when the patient is laying on his back
Fig. 12-2 is a front view of a patient interface according to Fig. 12, and illustrating the force vectors when the patient is laying on his side
Fig. 13 shows a front view of a cushion of the patient interface of Fig. 12.
Fig. 14 shows a front view of the cushion of Fig. 13 with vents removed from the cushion.
Fig. 14-1 shows a perspective view of an insert configured to removably connect to the cushion of Fig. 14 in order to connect a vent to the cushion.
Fig. 15 shows a rear view of the cushion of Fig. 14.
Fig. 16 shows a lower rear view of the cushion of Fig. 13, illustrating a passage between a nasal portion and an oral portion of the plenum chamber.
Fig. 17 shows a cross-sectional view of the cushion of Fig. 14, illustrating the passage between the nasal portion and the oral portion of the plenum chamber.
Fig. 17-1 shows the cross-sectional view of the cushion of Fig. 14 being worn by the patient.
Fig. 18 shows a cross-sectional view of an alternate form of the cushion of Fig. 17, illustrating a smooth transition between the nasal portion and the oral portion of the seal-forming structure.
Fig. 18-1 shows the cross-sectional view of the cushion of Fig. 18 being worn by the patient with the oral portion and the nasal portion forming separate seals.
Fig. 18-2 shows a cross-sectional view of the cushion of Fig. 18 being worn by the patient with the oral portion and the nasal portion forming a continuous seal against the patient's face.
Fig. 19 shows a cross-sectional view of the cushion of Fig. 14, illustrating the nasal portion fluidly isolated from the oral portion of the plenum chamber.
Fig. 19-1 shows an alternate cross-sectional view of the cushion of Fig. 14, illustrating a membrane with holes to partially isolate the oral portion from the nasal portion.
Fig. 20 shows a front view of a patient wearing another form of a patient interface for sealing around the patient's nares and around the patient's mouth.
Fig. 21 shows a front view of a cushion of the patient interface of Fig. 20.

### 4.9 FURTHER MOUTH LEAK CONTROLLING DEVICES

Fig. 22 shows a front view of a patient wearing a patient interface and a mouth leak controlling device according to an example of the present technology.
Fig. 23A shows a perspective view of a patient wearing a patient interface and a mouth leak controlling device according to an example of the present technology.
Fig. 23B shows a front view of a partially disassembled patient interface and a mouth leak controlling device according to an example of the present technology.
Fig. 24A shows a perspective view of a mouth leak controlling device according to an example of the present technology.
Fig. 24B shows a rear perspective view of a mouth leak controlling device according to an example of the present technology.
Fig. 24C shows a rear view of a mouth leak controlling device according to an example of the present technology.
Fig. 24D shows a rear perspective view of a mouth leak controlling device with its valve being assembled according to an example of the present technology.
Fig. 24E shows a rear perspective view of a mouth leak controlling device with its valve being assembled according to an example of the present technology.
Fig. 25A shows a perspective view of a flexible portion and a support portion of a mouth leak controlling device according to an example of the present technology.
Fig. 25B shows a rear perspective view of a flexible portion and a support portion of a mouth leak controlling device according to an example of the present technology.
Fig. 25C shows a rear view of a flexible portion and a support portion of a mouth leak controlling device according to an example of the present technology.
Fig. 26A shows a perspective view of a strap attached to a mouth leak controlling device according to an example of the present technology.
Fig. 26B shows a perspective view of a strap being attached to a mouth leak controlling device according to an example of the present technology.
Fig. 27A shows perspective view of a valve flap for a mouth leak controlling device according to an example of the present technology.
Fig. 27B shows perspective view of a valve flap for a mouth leak controlling device according to an example of the present technology.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

### 5.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 5.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 5.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 4 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

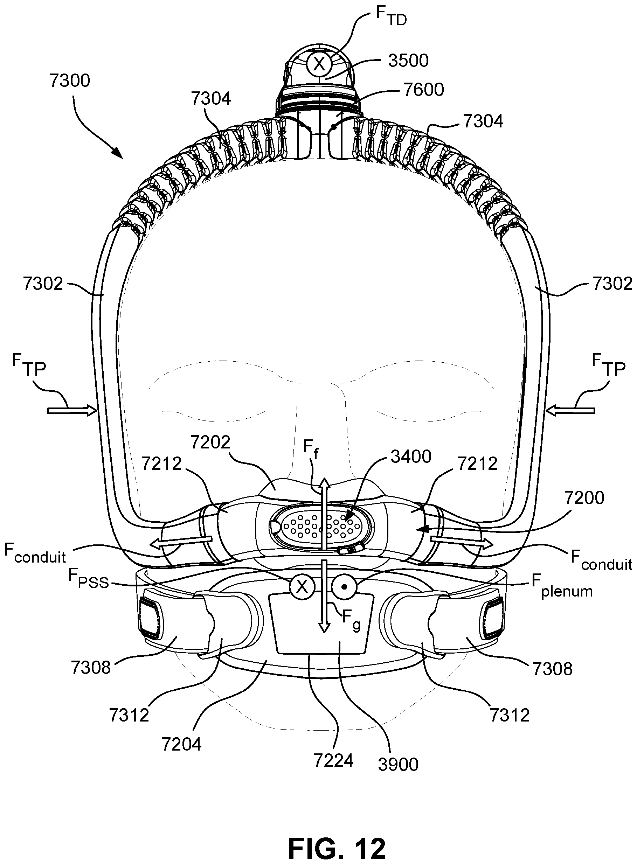

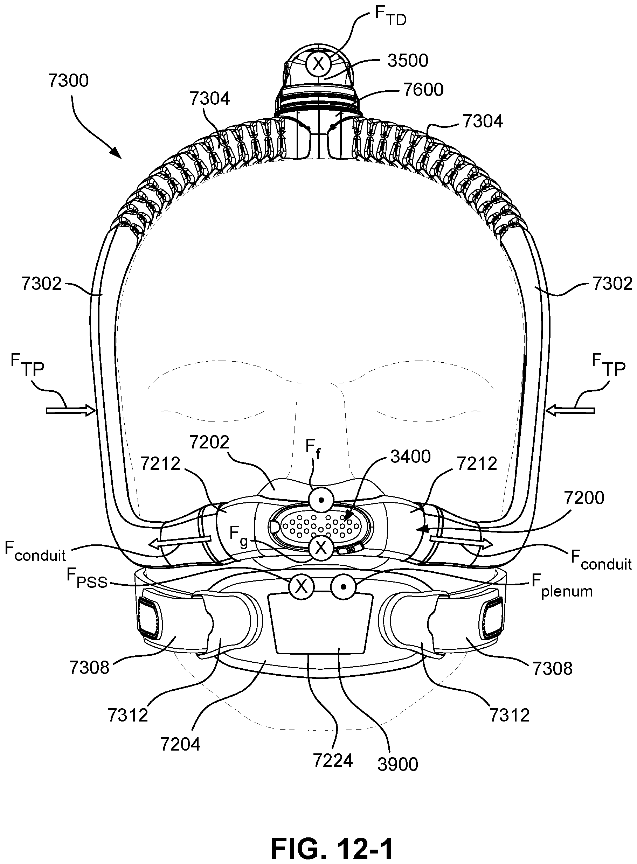

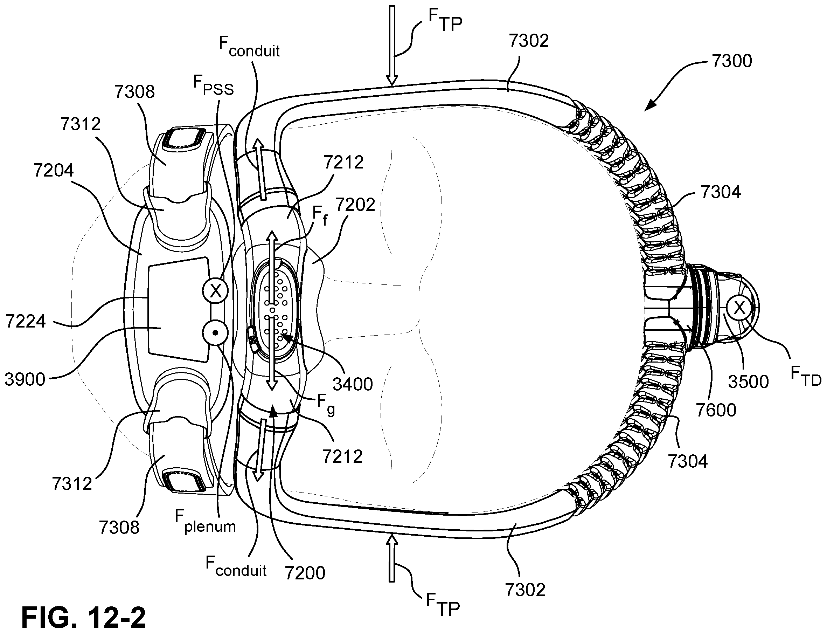

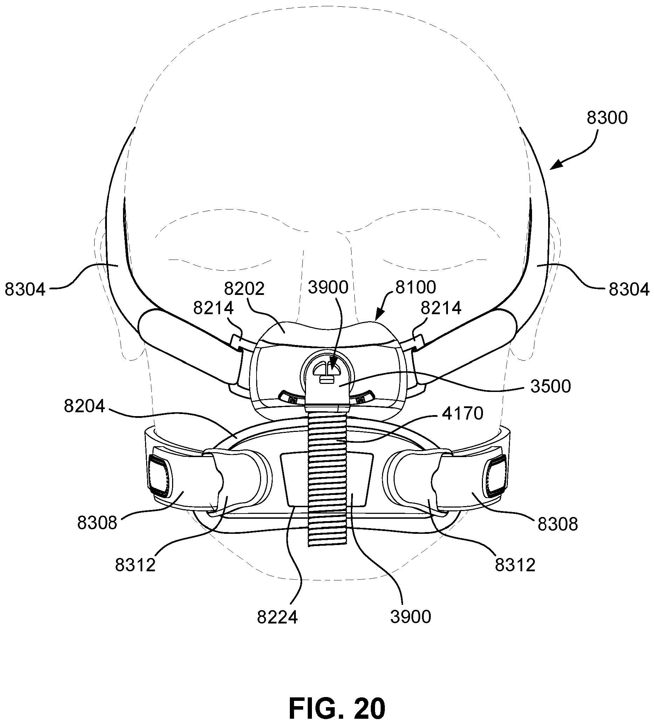

The patient interface 7000 (see e.g., Fig. 12) and the patient interface 8000 (see e.g., Fig. 20) may be similar to the patient interface 3000. Only some similarities and differences may be described below.

### 5.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 5.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 5.3.1.1.1 Separate sealing mechanisms

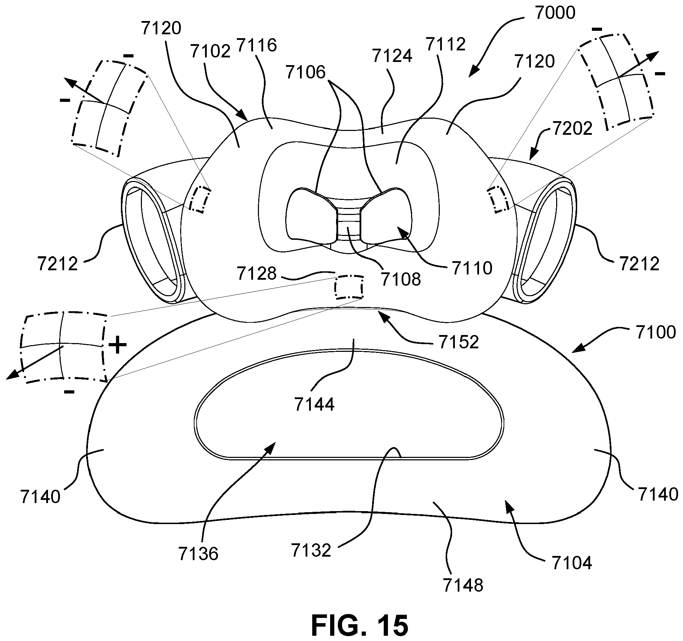

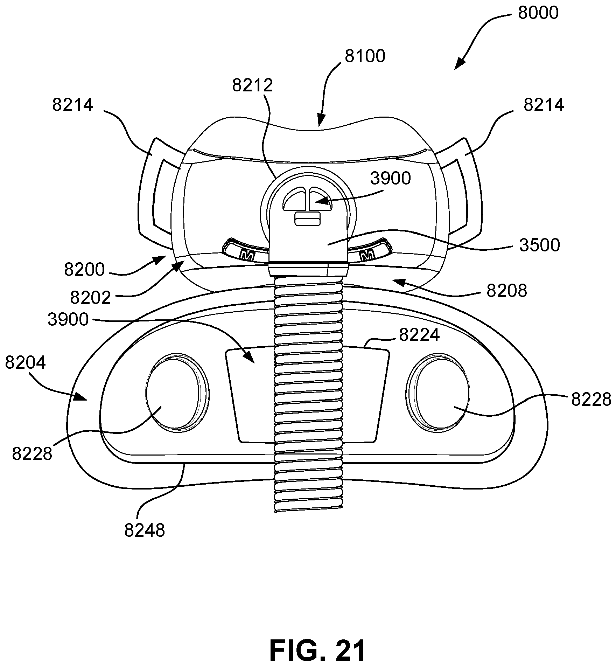

As shown in Fig. 15, the patient interface 7000 may include a seal-forming structure 7100 that includes separate portions for the patient's nose and for the patient's mouth. In other words, the seal-forming structure 7100 may include a nasal seal-forming structure 7102 and an oral seal-forming structure 7104. The patient interface 8000 in Fig. 21 may include a seal-forming structure 8100 with some similarities and differences than the seal-forming structure 7100. Additional description of the seal-forming structure 8100 may not be repeated for the sake of brevity.

In some forms, the nasal seal-forming structure 7102 may include a pair of naris openings 7106 separated by a bridge portion 7108. Each naris opening 7106 may provide fluid communication into or out of a nasal cavity 7110. The patient's columella may contact the bridge portion 7108 in use. When in contact with the bridge portion 7108, the patient's nares may be aligned with the respective naris openings 7106. This may enable pressurized air to flow through the naris openings 7106 between the nasal cavity 7110 and the patient's airways without be substantially occluded by the nasal seal-forming structure 7102 (e.g., the width of the bridge portion 7108 may substantially correspond to a width of a patient's columella).

In certain forms, the bridge portion 7108 may be slack prior to use and may extend partially into the nasal cavity 7110 when not in contact with the patient's face. For example, the bridge portion 7108 may form a curved surface when not in contact with the patient. Contact with the patient's nose may apply tension to the bridge portion 7108 so that the bridge portion is taut during use.

In certain forms, the bridge portion 7108 may be taut prior to use. The bridge portion 7108 may form a less curved surface than the bridge portion 7108 in the slack arrangement. Contact with the patient's nose may apply additional tension to the bridge portion 7108.

In certain forms, the nasal seal-forming structure 7102 may include a single opening for fluid communication with both nares simultaneously. **In** this example, there may be no bridge portion 7108 dividing the single opening.

In some forms, the nasal seal forming structure 7102 includes an inner sealing portion 7112 and an outer sealing portion 7116. The inner sealing portion 7112 may include the naris openings 7106 and the bridge portion 7108. The outer sealing portion 7116 may be disposed radially outside of the inner sealing portion 7112.

In some forms, the inner sealing portion 7112 contacts an underside of the patient's nose in use. As noted above, the bridge portion 7108 may contact the patient's columella in use. The remainder of the inner sealing portion 7112 may contact the surface of the patient's nose forming the alar rims. The inner sealing portion 7112 may also contact the patient's nose between the patient's subnasale and the patient's pronasale.

In some forms, the outer sealing portion 7116 may contact the patient's nose radially outside of the area contacted by the inner sealing portion 7112. For example, lateral sides 7120 may contact the patient's face between an alar crest point and the nasolabial sulcus on either side of the patient's nose. The lateral sides 7120 may be formed as negative domed surfaces. This may help the lateral sides 7120 fit into the tight cervices along the outer portion of the patient's nose to form a more effective seal (e.g., to reduce leaks).

In some forms, the outer sealing portion 7116 may include an upper nasal sealing region 7124, which may be disposed more superior than the inner sealing portion 7112. In the illustrated example, the outer sealing portion 7116 may not extend substantially superior to the inner sealing portion 7112. In other words, the outer sealing portion 7116 may not extend beyond or substantially beyond the patient's pronasale while the patient interface 7000 is in use.

In some forms, the outer sealing portion 7116 may include a lower nasal sealing region 7128, which may be disposed more inferior than the inner sealing portion 7112. In the illustrated example, the lower nasal sealing region 7128 may extend more inferior to the inner sealing portion 7112 than the upper nasal sealing region 7124 extends superior to the lower nasal sealing region 7128.

In certain forms, the inner sealing portion 7112 and the outer sealing portion 7116 may be formed with a smooth transition so that there is substantially no corner between each sealing portion 7112, 7116.

In certain forms, the inner sealing portion 7112 may have a different radius of curvature than the outer sealing portion 7116. This may form a transition between the inner and outer sealing portions 7112, 7116. For example, the inner sealing portion 7112 may have a smaller radius of curvature (i.e., a deeper curve) in order to fit snuggly against the patient's nose (e.g., which may be narrower than the surrounding facial features).

In some forms, the oral seal-forming structure 7104 may surround an oral opening 7132 which may provide fluid communication into or out of an oral cavity 7136. The oral opening 7132 may be sized to receive a patient's mouth while he is wearing the patient interface 7000.

In the illustrated examples, the oral opening 7132 may have a rounded shape. For example, the oral opening 7132 may have a substantially elliptical shape or a circular shape. In other examples, the oral opening 7132 may have a different geometric shape. The shape of the oral opening 7132 may be selected in order to correspond to a shape of the patient's mouth in order to form a comfortable seal. For example, the oral opening 7132 may have a width greater than the mouth width in order to limit occlusion of the patient's mouth by the oral seal-forming structure 7104.

In some forms, lateral side regions 7140 may be formed on either lateral side of the oral seal-forming structure 7104. The lateral side regions 7140 may contact the patient's face laterally outside of the mouth width (i.e., in order to limit occlusion of the patient's mouth). The lateral side regions 7140 may contact the patient between the cheilion and the nasolabial sulcus on either side of the patient's face.

In some forms, an upper oral sealing region 7144 may be formed at a superior region of the oral seal-forming structure 7104. The upper oral sealing region 7144 may extend between the lateral side regions 7140. The upper oral sealing region 7144 may contact the patient's lip superior while the patient interface 7000 is being worn.

In some forms, a lower oral sealing region 7148 may be formed at an inferior region of the oral seal-forming structure 7104. The lower oral sealing region 7148 may extend between the lateral side regions 7140. The lower oral sealing region 7148 may contact the patient's lip inferior while the patient interface 7000 is being worn.

### 5.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

As shown in Figs. 12 and 20, the nasal seal-forming structures (e.g., 7102 in Fig. 12 and unillustrated element in Fig. 20) of each respective patient interface 7000, 8000 may not seal against the patient's nose bridge region or on a nose-ridge region. Each nasal seal-forming structure 7102, may contact the patient's face so that the patient's pronasale remains exposed to the ambient environment in use. For example, the upper nasal sealing region 7124 may not extend substantially beyond the patient's pronasale. This may promote patient comfort because less of the patient's nose is covered and within a pressurized environment. In other examples, the nasal seal-forming structures (e.g., 7102) may be formed so that they seal against the nose bridge region or on a nose-ridge region.

### 5.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

As shown in Figs. 15 and 17 to 19, the lower nasal sealing region 7128 may be positioned at an inferior position of the nasal seal-forming structure 7102, and may be positioned to contact the patient's lip superior while the patient interface 7000 is in use.

As described above, the patient's columella may contact the bridge portion 7108 while wearing the patient interface 7000. Unlike the upper nasal sealing region 7124, the lower nasal sealing region 7128 may extend below further beyond the inner sealing portion 7112 in order to contact additional area on the patient's face. Here, the lower nasal sealing portion 7128 may extend inferior to the patient's subnasale to contact, and seal against, the patient's lip superior. This may provide additional sealing and may be on a location less susceptible to discomfort (e.g., as compared to the nose bridge region or nose-ridge region).

In the illustrated examples, the lower nasal sealing region 7128 may be formed with a saddle region. For example, the nasal seal-forming structure 7102 may have a positive curvature between the lateral sides 7120 and a negative curvature inferior to the inner sealing portion 7112 toward the oral seal-forming structure 7104. As a result of the saddle region, the total contact area between the lower nasal sealing region 7128 and the patient's lip superior may be small.

In some forms, the lower nasal sealing region 7128 may have a thickness of about 0.1 mm thick to about 1 mm thick. In some forms, the lower nasal sealing region 7128 may have a thickness of about 0.2 mm thick to about 0.5 mm thick. In some forms, the lower nasal sealing region 7128 may have a thickness of about 0.3 mm thick to about 0.4 mm thick. In some forms, the lower nasal sealing region 7128 may have a thickness of about 0.35 mm thick. In some forms, the thickness of the lower nasal sealing region 7128 may be the same as the thickness of the remainder of the nasal seal-forming structure 7102 (although the lower nasal sealing region 7128 may be thicker or thinner than other portions of the nasal seal-forming structure 7102).

As shown in Figs. 15 and 17 to 19 the upper oral sealing region 7144 may be positioned at a superior position of the oral seal-forming structure 7104, and may be positioned to contact the patient's lip superior while the patient interface 7000 is in use. The upper oral sealing region 7144 may contact the patient's lip superior at least partially between the upper vermillion and the subnasale. For example, the upper oral sealing region 7144 may contact the patient's lip superior proximate (or adjacent) to the patient's upper vermillion and may be more spaced apart from the patient's subnasale.

In some forms, the curvature of the upper oral sealing region 7144 may be less pronounced than the curvature of the lower nasal sealing region 7128. For example, the lower nasal sealing region 7128 may have a smaller radius of curvature between the lateral sides 7120 than the upper oral sealing portion 7144 has between the lateral side regions 7140. The curvature along the patient's lip superior proximate to the upper vermillion may be flatter than the curvature proximate to the patient's subnasale between the nares.

In some forms, the upper oral sealing region 7144 may have a thickness of about 0.1 mm thick to about 1 mm thick. In some forms, the upper oral sealing region 7144 may have a thickness of about 0.2 mm thick to about 0.5 mm thick. In some forms, the upper oral sealing region 7144 may have a thickness of about 0.3 mm thick to about 0.4 mm thick. In some forms, the upper oral sealing region 7144 may have a thickness of about 0.35 mm thick. In some forms, the thickness of the upper oral sealing region 7144 may be the same as the thickness of the remainder of the oral seal-forming structure 7104 (although the upper oral sealing region 7144 may be thicker or thinner than other portions of the oral seal-forming structure 7104).

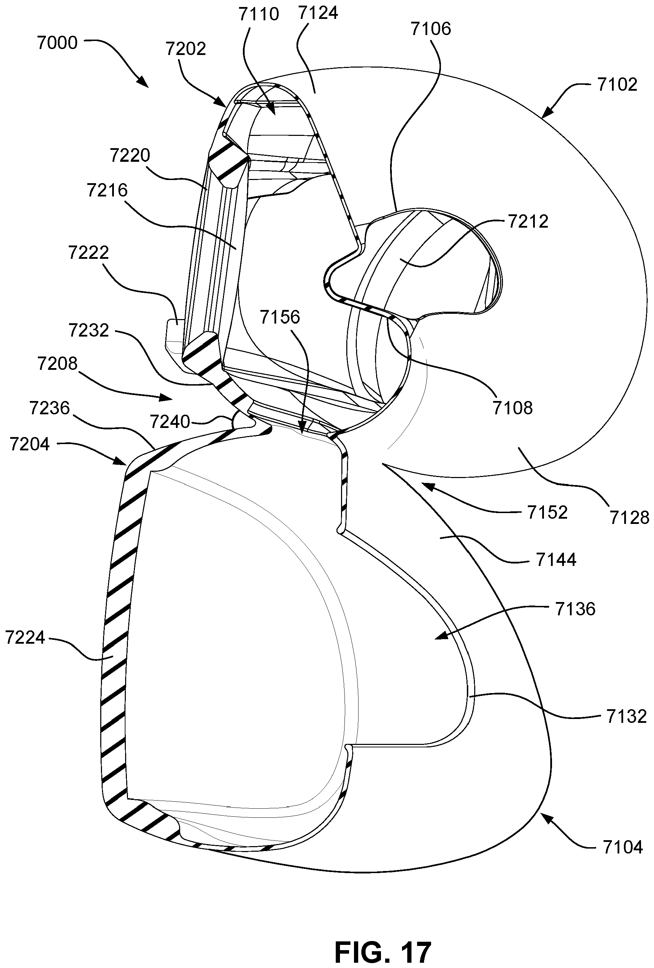

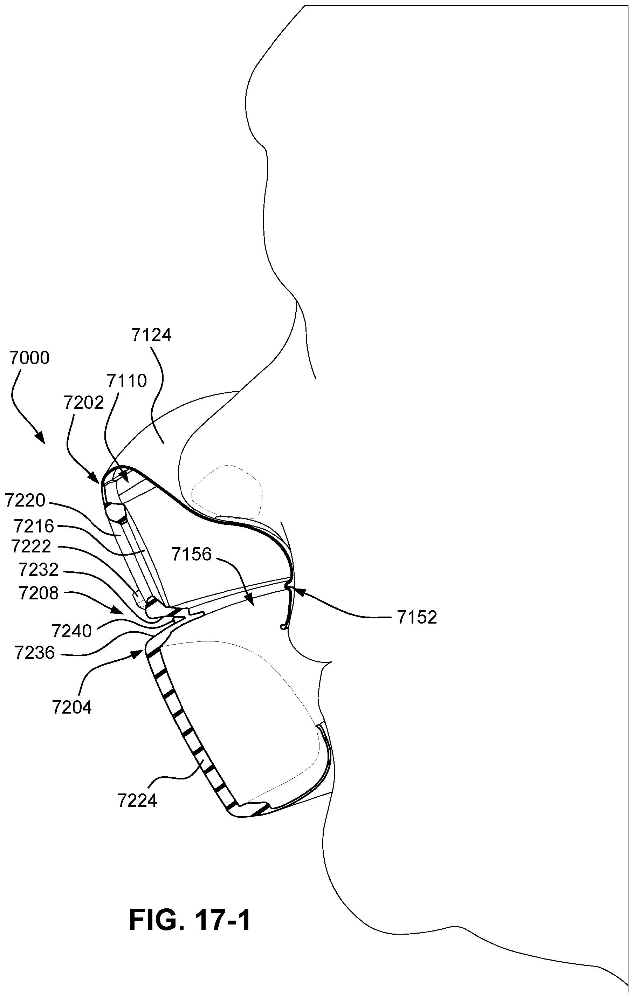

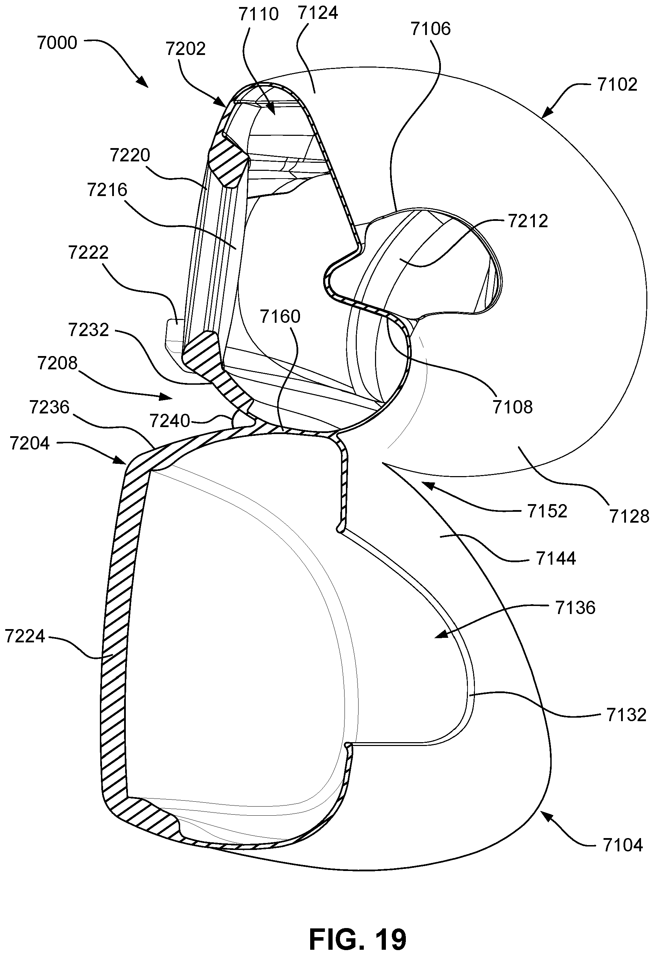

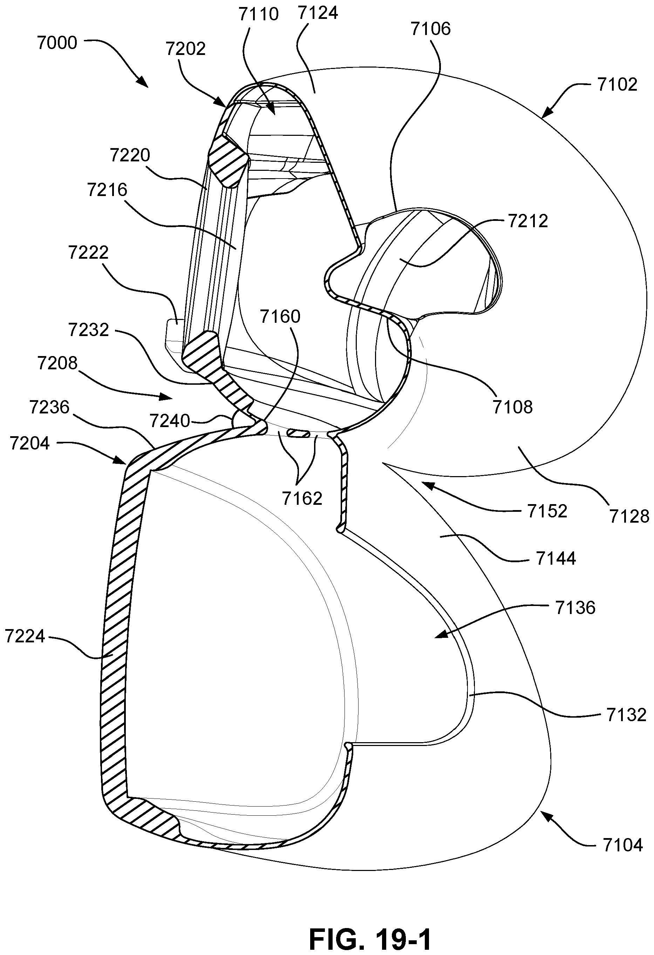

As shown in Figs. 17, 17-1, and 19, the lower nasal sealing region 7128 and upper oral sealing region 7144 may be connected to one another by a connecting region 7152, which may form a transition between the lower nasal sealing region 7128 and the upper oral sealing region 7144.

The connecting region 7152 may be an at least partially curved surface that extends between the lower nasal sealing region 7128 and upper oral sealing region 7144. The connecting region 7152 may at least partially have a positive curvature relative to a patient wearing the patient interface 3000.

As shown in Figs. 17, 17-1, and 19, the lower nasal sealing region 7128 and the upper oral sealing region 7144 may be inclined relative to one another so that the connecting region 7152 is formed at least partially as an angled or corner region. In other words, a portion of the connection region 7152 may form a corner (e.g., at a junction between the lower nasal sealing region 7128 and the upper oral sealing region 7144). However, the connecting region 7152 as a whole may still be formed as a curved surface. For example, the lower nasal sealing region 7128 and the upper oral sealing region 7144 may both be formed as negative curvatures (e.g., in the inferior-superior direction). The negative curved regions may form a curved surface around the connecting region 7152. Additionally, as the negative curved regions connect, they form the positive curvature of the connecting region 7152.

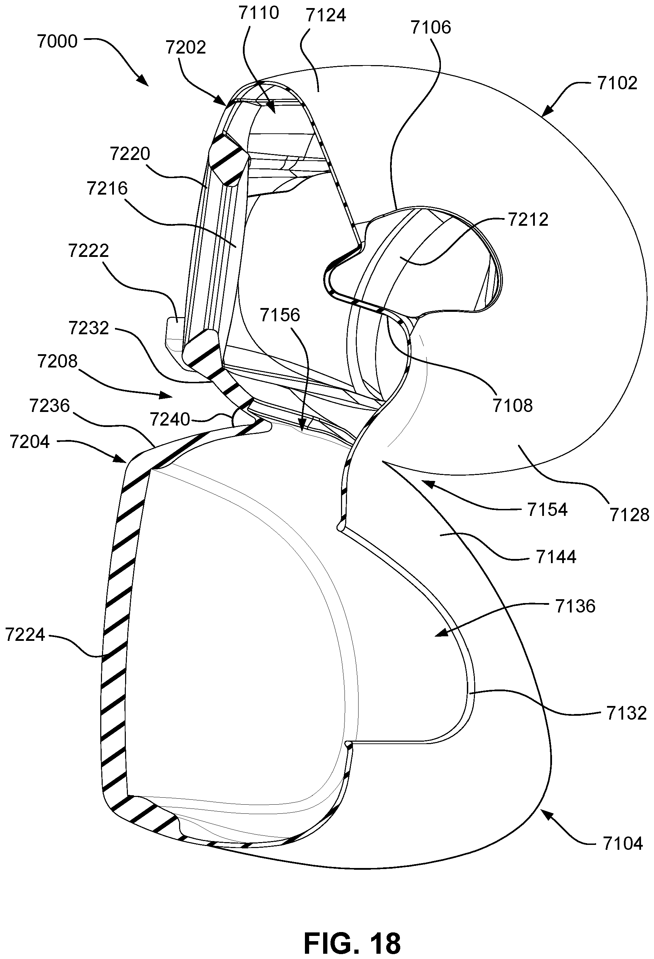

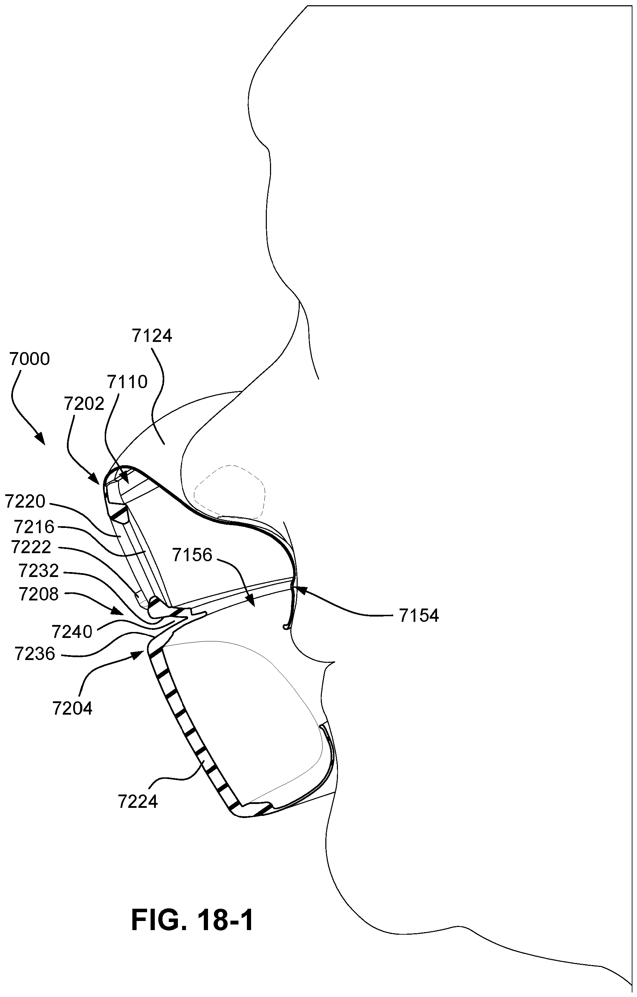

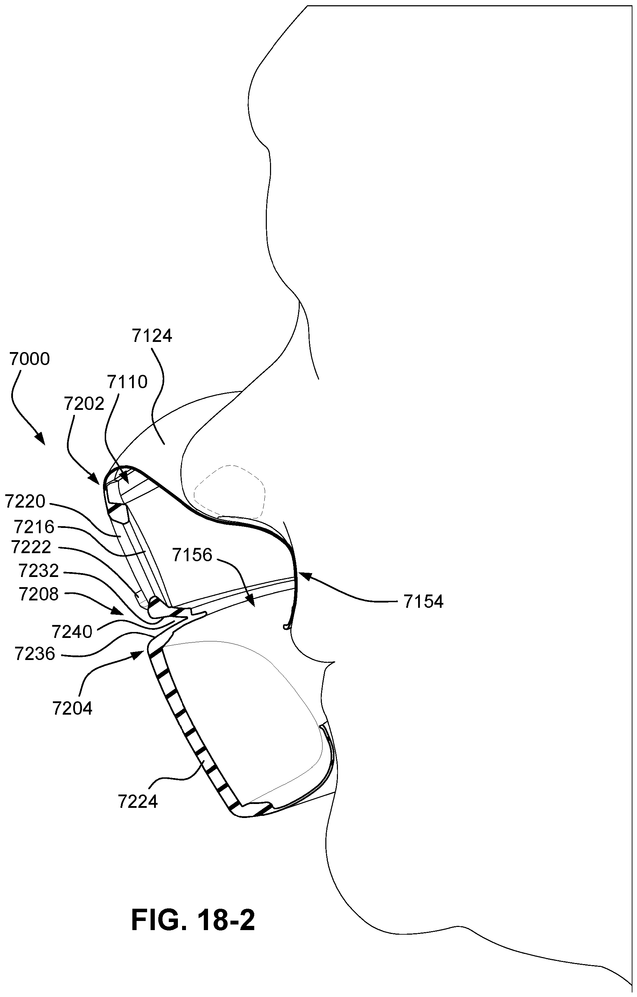

In the illustrated example, there is substantially no transition between the between the negative curved regions of the lower nasal sealing region 7128 and the upper oral sealing region 7144. This forms a corner region of the connecting region 7152 because there is not a larger, smooth transition (e.g., unlike in Fig. 18, described below).

As shown in Fig. 17-1, the connecting region 7152 may be spaced apart from the patient's lip superior during use. In other words, the patient's lip superior may not extend into the corner region while wearing the patient interface 7000 in order to minimize discomfort. The connecting region 7152 therefore may not seal against the patient's face.

While the corner region of the connecting region 7152 may be spaced part from the patient's face, the patient's face may still contact the curved portions of the lower nasal sealing region 7128 and the upper oral sealing region 7144 that transition into the connecting region 7152.

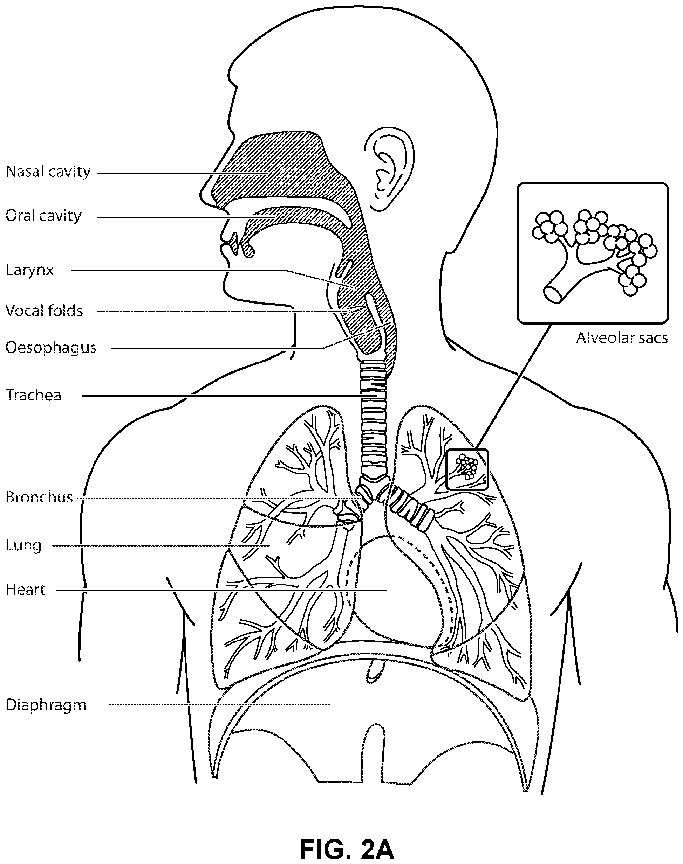

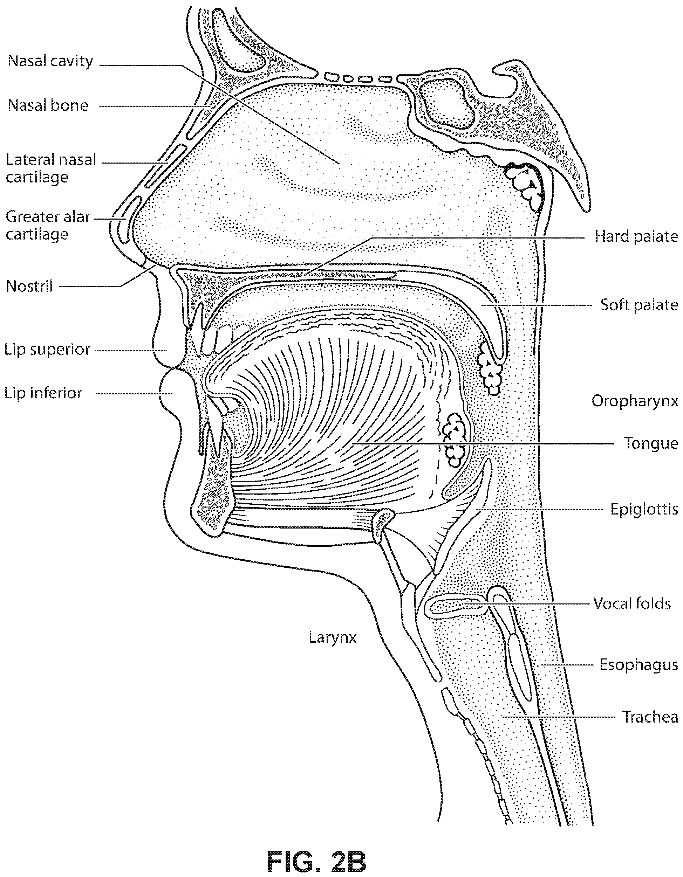

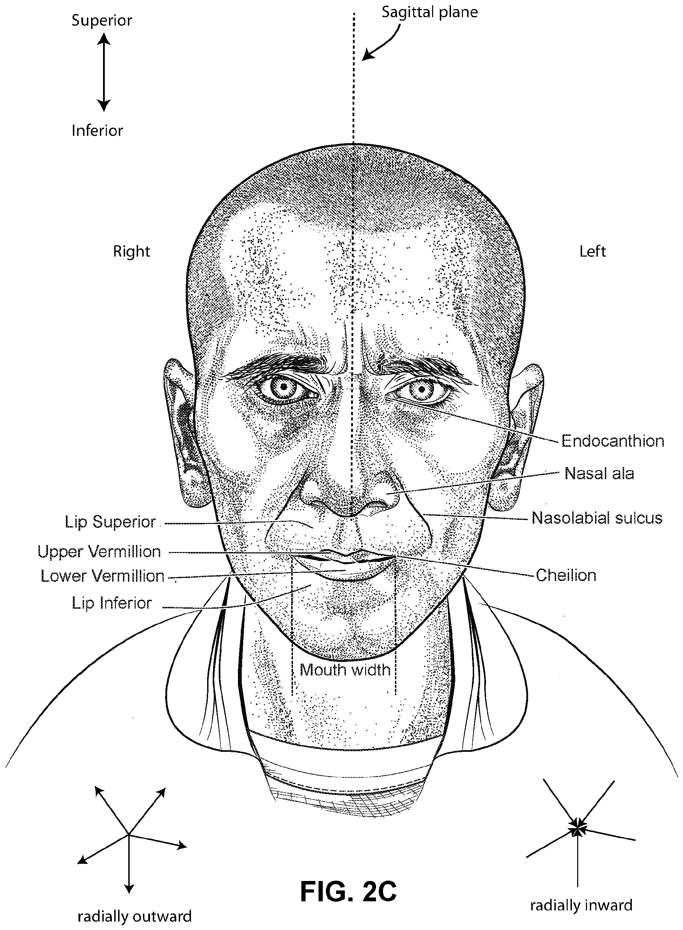

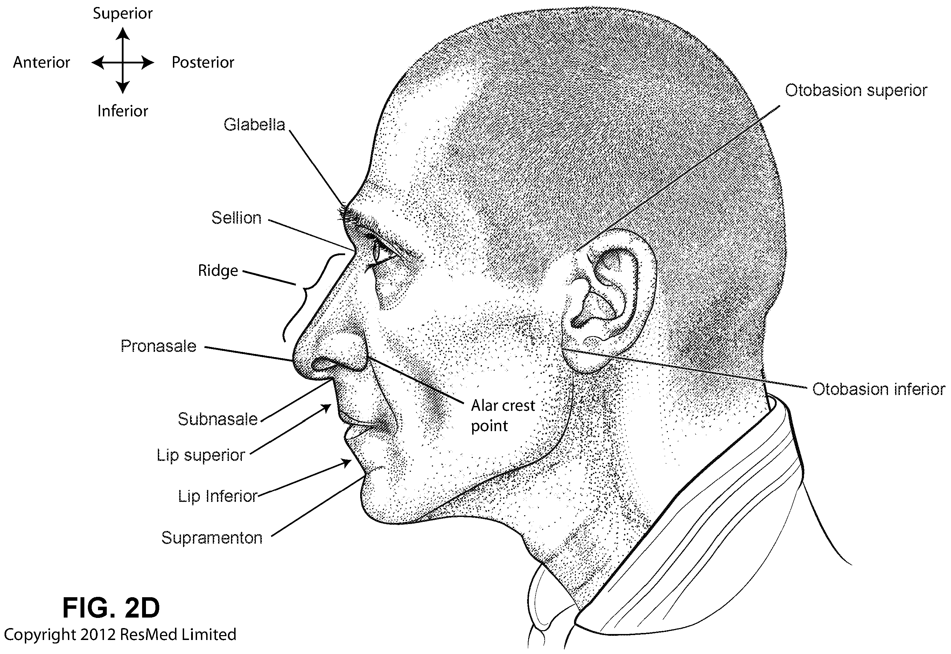

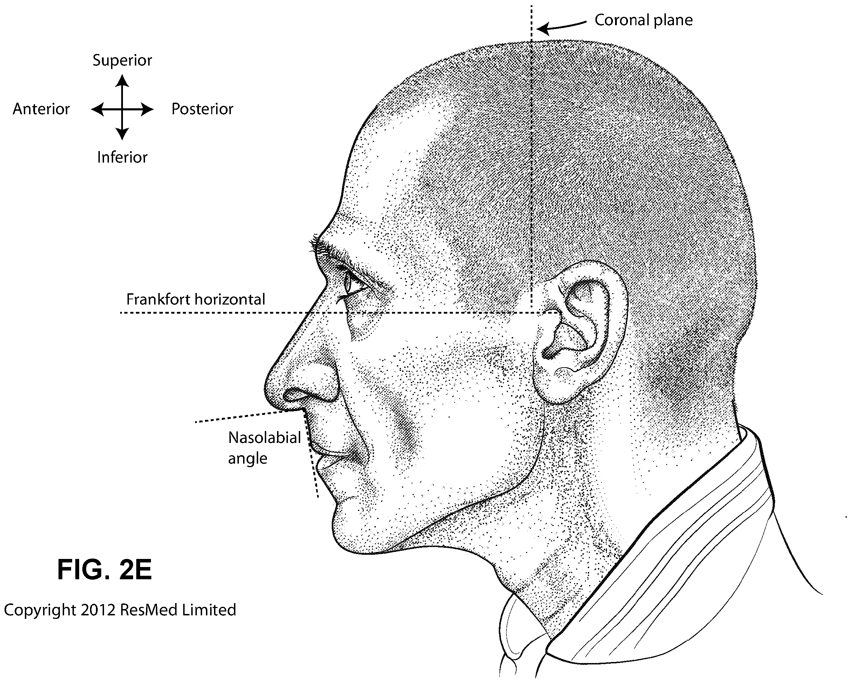

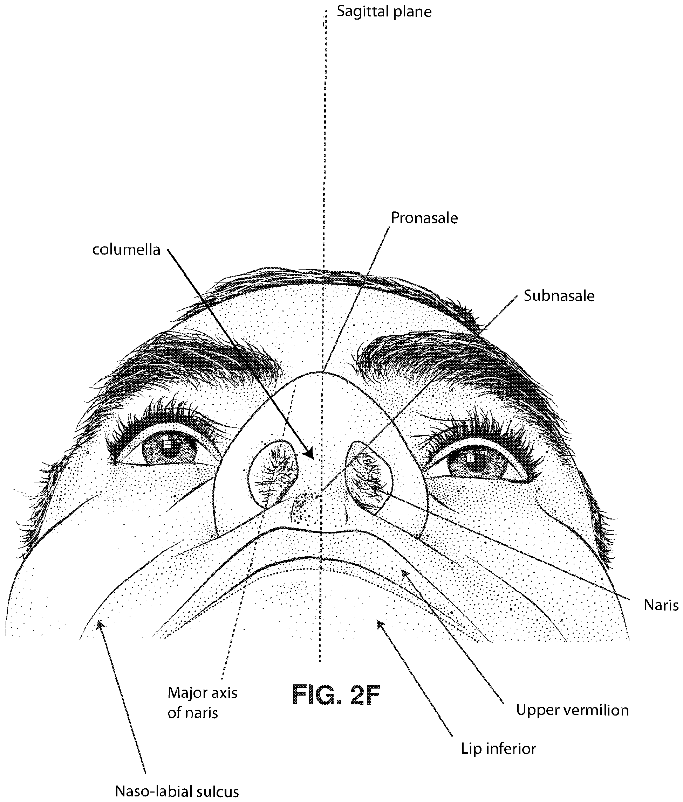

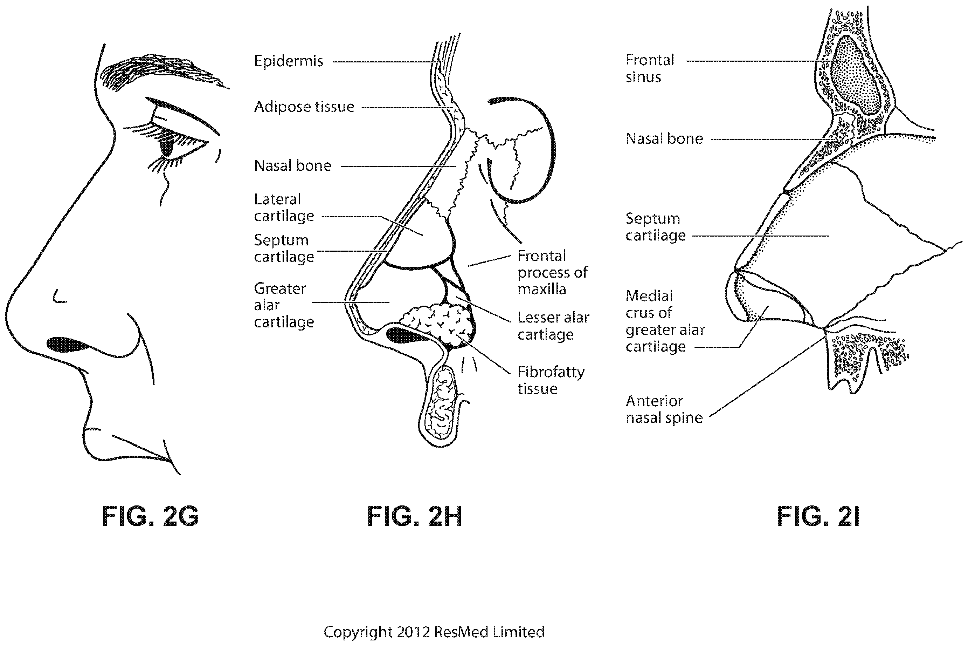

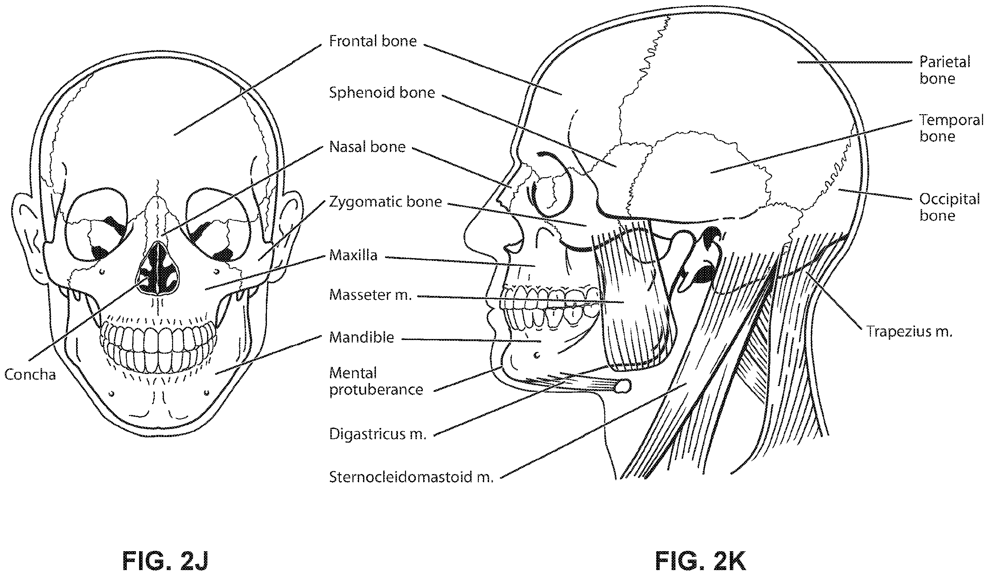

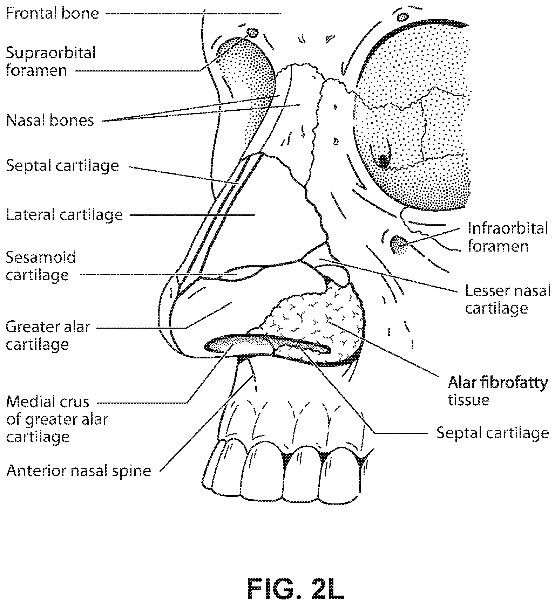

As shown in Figs. 18 to 18-2, a substantially smooth connecting region 7154 may be formed between the lower nasal sealing region 7128 and the upper oral sealing region 7144. Unlike the connecting region 7152, the substantially smooth connecting region 7154 does not form an angle or corner. Instead, the substantially smooth connecting region 7154 may be a more gradually curved surface that extends between the lower nasal sealing region 7128 and the upper oral sealing region 7144. The curved surface of the connecting region 7154 may be more comfortable to contact the patient's face (e.g., as compared to the connecting region 7152). While the smooth connecting region 7154 may contact the patient's lip superior, it may not contribute or substantially contribute to sealing.

In the illustrated example, the connecting region 7154 may still be formed as the convergence between the negative curved regions of the lower nasal sealing region 7128 and the upper oral sealing region 7144. However, the two negative curved regions may be spaced apart so that there is a larger transition in between. This may form a more defined positive curvature than the positive curvature of the connecting region 7152.

The connecting regions 7152, 7154 may act as a hinge between the lower nasal sealing region 7128 and the upper oral sealing region 7144. The nasal seal-forming structure 7102 to move relative to the oral seal-forming structure 7104 without substantially affecting the oral seal-forming structure 7104 (or vice versa). This may allow the respective connecting region 7152, 7154 to at least partially decouple the nasal seal-forming structure 7102 from the oral seal-forming structure 7104. In other words, forces applied to either the nasal seal-forming structure 7102 or the oral seal-forming structure 7104 may not substantially affect the other of the nasal seal-forming structure 7102 or the oral seal-forming structure 7104. This may allow adjustments to be made to improve the quality of one seal (e.g., achieving better contact with the patient's face) without degrading the quality of the other seal (e.g., creating leaks).

The corner region of the connecting region 7152 may be more suited for bending or pivoting movement. For example, the shape of the corner region may form a crease for the hinge. In other words, the connecting region 7152 has a defined location about where the pivoting movement will occur. This may be helpful because the pivoting movement of the connecting region 7152 may consistently take place at the same location.

As shown in Fig. 18-1, the corner region of the connecting region 7152 is spaced apart from the patient's lip superior in use so that the movement may not pinch or otherwise irritate the patient. In other words, the location of pivoting movement may not contact the patient's face and thus may not contribute to sealing against the patient's face. The patient may not substantially feel the movement about the connecting region 7152 as the material is not in contact with the patient's face.

Although the smooth connecting region 7154 may have a pre-defined bending location (e.g., a creased region), it still may be able to bend a sufficient amount in order to decouple forces applied to the patient interface 3000.

Because the connecting regions 7152, 7154 do not substantially contribute to sealing against the patient's lip superior, each connecting region 7152, 7154 is able to act as a decoupling structure because movement of the connecting region 7152, 7154 does not affect the seal. **In** other words, the connecting regions 7152, 7154 may operate as decoupling regions through pivoting movement, which decouples the forces applied to the patient interface 7000, whilst maintaining seal integrity. Each connection region 7152, 7154 may move without disturbing the nasal and oral seal forming structures 7102, 7104 so that the decoupling operation does not itself harm the quality of the seal.

For example, the inner sealing portion 7112 (see Fig. 15) of the nasal seal forming structure 7102 is disposed around the naris openings 7106 and directly seals against the patient's alar rim and subnasale. The lower nasal sealing region 7128 is disposed inferior to and outside of the inner sealing portion 7112. Although the patient's lip superior contacts the lower nasal sealing region 7128, it may not substantially contribute to sealing against the patient's face. **In** other words, the seal to limit leaks of pressurized breathable gas around the patient's nose may be substantially formed by the inner sealing portion 7112. The lower nasal sealing region 7128 may partially contribute to the sealing but also be held against the patient's lip superior without providing any additional sealing.

As a result, small movements of the lower nasal sealing region 7128 (e.g., because of movement of the adjacent connecting region 7152) may not substantially disturb the seal integrity because the small movements may not affect the position of the inner sealing portion 7112 relative to the patient's nose.

Similarly, the inner portion of the oral seal forming structure 7104 is responsible for forming the seal around the patient's mouth. **In** other words, the material proximate to the oral opening 7132 may form the seal around the patient's mouth to limit air leaking from the oral cavity 7136. Material further outside (i.e., radially further from the oral opening 7132) may contact the patient's skin, but may contribute less to limiting leak. Therefore, slight movement in this region of the oral seal forming structure 7104 may not disturb the integrity of the seal.

In use, the patient's face may contact the seal-forming structure 7100 and provide a force at least partially directed in the anterior direction. For example, the force against the oral seal forming structure 7104 may be substantially in the anterior direction. The force against the nasal seal forming structure 7102 may be in both the anterior and inferior directions. As a result of this contact and tightening the positioning and stabilizing structure 7300, the connecting region 7152, 7154 may expand or stretch. For example, the connecting region 7152 may expand about the corner region to increase the radius of curvature of the connection region 7152. The connecting region 7152 in use may look like the connecting region 7154 in Fig. 18, or the connecting region 7152 may retain a portion of the corner region. Similarly, the connecting region 7154 may expand to have an even larger radius of curvature or become substantially planar. In either case, more of the connecting region 7152 may be in contact with the patient's face. However, this material newly in contact with the patient's face may not provide additional sealing. Instead, the expansion may limit or prevent the portions of the seal forming structure 7100 that are responsible for sealing from being disturbed. In other words, expanding the connecting region 7152, 7154 may limit shifting in the inner sealing portion 7112 and/or the oral seal forming structure 7104 directly adjacent to the oral opening 7132.

As shown in Fig. 18-2, the corner region of the connecting region 7154 may be substantially flat and contact the patient's lip superior in use. In other words, the connecting region 7154 may stretch or expand to fully contact the patient's lip superior and contribute to sealing against the patient's face.

The connecting region 7154 may be sized so that it rests smoothly against the patient's face and the single section of the seal may contribute to sealing around the patient's nares and the patient's mouth. For example, the lower nasal sealing region 7128 and the upper oral sealing region 7144, along with the connecting region 7154, may form a continuous seal against the patient's lip superior (e.g., not broken up by spacing between the connecting region 7154 and the patient's lip superior).

In some forms, the thickness of the connecting region 7154 may be thin (e.g., between about 0.25 mm and about 0.55 mm), which may allow the connecting region to stretch and interface with the patient's face. For example, the connecting region 7154 may easily stretch as a result of the reduced thickness so that it can sit fully in contact with the patient's face (e.g., substantially without spacing between a corner region and the lip superior). This may be useful to help create a sufficient seal against the patient's face. For example, the patient may need to be less precise with the seal-forming structure's contact location in order to establish an effective seal. The expanding connecting region 7154 will seal against substantially the entire lip superior of the patient.

In some forms, there may not be a substantial difference between the lower nasal sealing region 7128 and the upper oral sealing region 7144. For example, as shown in Fig. 18, there may be a smooth curvature across the connecting region 7154 between lower nasal sealing region 7128 and the upper oral sealing region 7144. When the connection region 7154 is stretched in use as shown in Fig. 18-2, the smooth curvature may allow the connecting region 7154 to be substantially flat (or have a small curvature to mirror the shape of the patient's lip superior). This may allow the connecting region 7154 to comfortably interface with the patient's face.

In certain forms, this may allow the seal-forming structure 7100 to slightly shift against the patient's face without substantially disturbing the seal. For example, the patient interface 7000 may move as a result of the patient's movements while sleeping. Small shifts in the seal-forming structures 7100 position on the face may not create leaks as a result of the connection region 7154 establishing a continuous seal between the lower nasal sealing region 7128 and the upper oral sealing region 7144.

In use, the patient's face may contact the seal-forming structure 7100 and provide a force at least partially directed in the anterior direction. For example, the force against the oral seal forming structure 7104 may be substantially in the anterior direction. The force against the nasal seal forming structure 7102 may be in both the anterior and inferior directions. As a result of this contact and tightening the positioning and stabilizing structure 7300, the connecting region 7154 may expand or stretch. For example, the connecting region 7154 may expand to have an even larger radius of curvature or become substantially planar. More of the connecting region 7154 may be in contact with the patient's face (e.g., than before the connecting region 7154 was stretched), and this material newly in contact with the patient's face may provide an additional sealing area (which may increase the strength of the seal). Although the connecting region 7154 may be entirely in contact with the patient's lip superior, the patient may not experience substantial discomfort, for example, because the connecting region 7154 is thin. Additionally, the connecting region 7208 of the plenum chamber 7200 may still provide the decoupling forces since the force from the positioning and stabilizing structure 7300 is applied directly to the plenum chamber 7200 (e.g., because the connection points are located on the plenum chamber 7200). Therefore, even when fully stretched and contacting the face, the patient may still experience the decoupling between the nasal and oral plenum chambers 7202, 7204.

In some forms, the shape illustrated in Fig. 18-2 may be achieved by the connecting region 7152 in Fig. 17. In other words, the connecting region 7152 with the corner region may still achieve the substantially flat orientation of Fig. 18-2 when used by the patient so that there is continuous contact along the patient's lip superior.

### 5.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

As shown in Figs. 15 and 17 to 19, the lower oral sealing region 7148 may be positioned at an inferior position of the oral seal-forming structure 7104, and may be positioned to contact the patient's lip inferior while the patient interface 7000 is in use.

As shown in Fig. 12, the lower oral sealing region 7148 may contact the patient's face lip inferior substantially superior to the patient's supramenton. The lower oral sealing region 7148 may contact only part of the patient's chin-region and may not extend completely inferior to the patient's mental protuberance.

### 5.3.1.5 Forehead region

In one form, the seal-forming structure that forms a seal in use on a forehead region of the patient's face. In such a form, the plenum chamber may cover the eyes in use.

### 5.3.1.6 Nasal pillows

In one form the seal-forming structure of the non-invasive patient interface 3000 (or the patient interfaces 7000, 8000) comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 5.3.1.7 Full face seal

In some forms, the seal-forming structure may include a single seal that seals around the patient's nares and the patient's mouth while in use. For example, the seal-forming structure may contact the patient's nasal bridge, extend outwardly toward the patient's nasolabial sulcus, and against the patient's lip inferior.

With a single seal-forming structure, the patient interface may include a single cavity in which the patient's nares and the patient's mouth are both positioned in and may both inhale from. In use, the patient may be able to inhale pressurized breathable gas through either his nares or his mouth.

In some forms, a single cavity may receive the patient's nose and the patient's mouth while in use. This may allow the patient to breathe through either his nose or his mouth while in use.

In some forms, a cavity may be divided so that patient's mouth is configured to be received in one portion and the patient's nose is configured to be received in the other. The portions of the cavity may be fluidly connected, or they may be fluidly isolated. Additionally, pressurized breathable gas may be delivered to both portions of the cavity or only to one portion.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate or a transparent silicone. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 5.3.2.1 Multiple inlet openings

As shown in Figs. 12 to 14, the patient interface 7000 may include a plenum chamber 7200 that includes separate portions for the patient's nose and for the patient's mouth. In other words, the plenum chamber 7200 may include a nasal plenum chamber 7202 and an oral plenum chamber 7204. The plenum chamber 7200 may include a connecting region 7208 between the nasal and oral plenum chambers 7202, 7204.

In some forms, the connecting region 7208 of the plenum chamber 7200 may be substantially opposite to the connecting region 7152, 7154 of the seal-forming structure 7100. In other words, the connecting region 7208 may face the respective connecting region 7152, 7154 across the anterior-posterior direction while the patient is using the patient interface 7000. Said another way, an axis substantially parallel to the patient's Frankfort horizontal may intersect the connecting region 7208 of the plenum chamber 7200 and the connecting region 7152, 7154 of the seal-forming structure 7100.

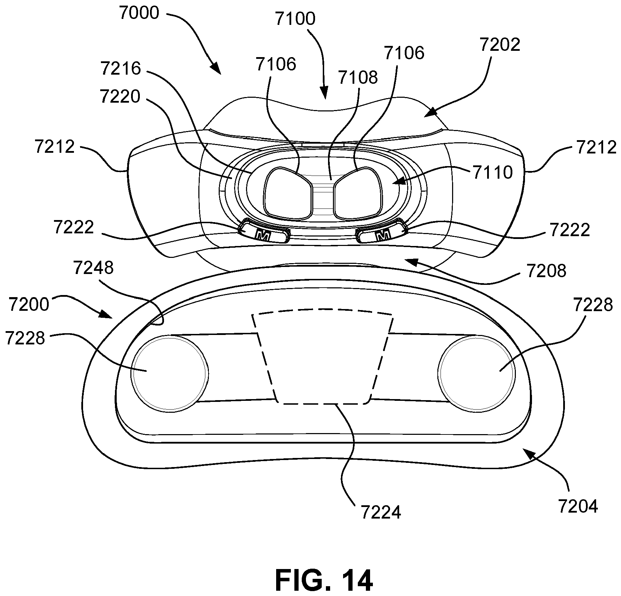

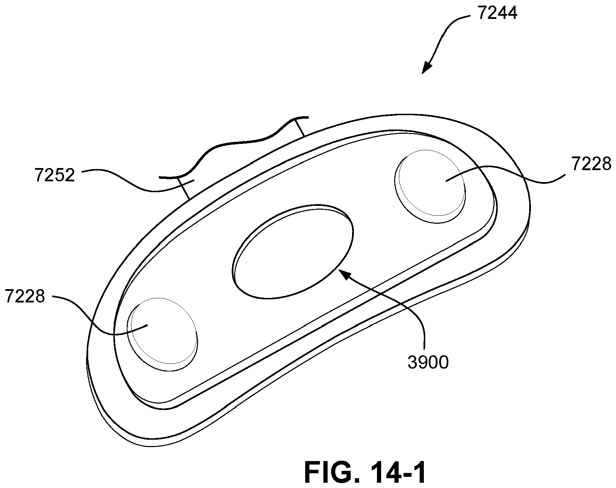

As shown in Fig. 14, the nasal plenum chamber 7202 may include at least one opening. For example, the nasal plenum chamber 7202 may include at least one plenum chamber inlet port 7212 and a vent opening 7216. These ports and openings 7212, 7216 may allow fluid flow into and/or out of the nasal plenum chamber 7202.

For example, the nasal plenum chamber 7202 may include a pair of plenum chamber inlet ports 7212. Each plenum chamber inlet port 7212 may be disposed on opposite lateral sides (e.g., the left and the right sides) of the nasal plenum chamber 7212. Conduits (e.g., conduit headgear - described below) may be connected (e.g., removably or permanently) to the plenum chamber inlet ports 7212. The conduits may convey the flow of pressurized breathable gas into the nasal plenum chamber 7202 through each of the plenum chamber inlet ports 7212.

The vent opening 7216 may receive a vent 3400 (removably or permanently). The vent opening 7216 may be positioned anterior to the patient's nose, and airflow exhaled from the patient's nares may be directed toward the vent opening 7216. The exhaled air may be able to exit the nasal plenum chamber 7202 through the vent opening 7216 (e.g., via the vent 3400), so that exhaled carbon dioxide may be exhausted to the ambient environment. As illustrated in Fig. 14, the naris openings 7106 may be aligned with the vent opening 7216 (e.g., along the posterior-anterior direction in use).

With continued reference to Fig. 14, some forms of the nasal plenum chamber 7202 may include a vent receiving region 7220, which may be partially recessed relative to the surrounding surface of the nasal plenum chamber 7202. The vent receiving region 7220 may have a rounded (e.g., elliptical) shape, although it may have any other shape. The vent receiving region 7220 may include an incline (e.g., toward the vent opening 7216). This may limit the vent 3400 from protruding substantially beyond the anterior surface of the nasal plenum chamber 7202 in order to avoid obscuring the patient's vision.

In some forms, at least one clip 7222 may be disposed proximate to the vent receiving region 7220. For example, the nasal plenum chamber 7202 may include a pair of clips 7222 that extend at least partially over the vent receiving region 7220. The clips 7222 may be constructed from a stiffened material (e.g., plastic, a thicker portion of silicone, etc.), and may engage the vent 3400 in order to limit translation of the vent 3400 relative to the nasal plenum chamber 7202.

As shown in Fig. 14, the oral plenum chamber 7204 may include an anti-asphyxia valve (AAV) receiving region 7224, which may form an opening that extends into the oral cavity 7136. The AAV receiving region 7224 may be sized to receive an AAV 3900.

In some forms, the oral plenum chamber 7204 may include at least one connector 7228 on an anterior surface. For example, the oral plenum chamber 7204 may include a pair of connectors 7228 spaced apart from one another. For example, the connectors 7228 may be disposed proximate to the lateral ends of the oral plenum chamber 7204. The AAV receiving region 7224 may be disposed between the pair of connectors 7228.

In certain forms, the connectors 7228 may be magnets or constructed from a magnetic material. In other examples, the connectors 7228 may be constructed from a different material (e.g., and may be mechanical fasteners).

In certain forms, the connectors 7228 may be permanently connected to the oral plenum chamber 7204. For example, the material of the oral plenum chamber 7204 may be molded around the connectors 7228.

As shown in Fig. 14-1, certain forms of the connectors 7228 may be removable from the oral plenum chamber 7204. In some forms, an insert 7244 may be used to removably connect the connectors 7228 to the oral plenum chamber 7204. The insert 7244 may have a similar profile to the oral plenum chamber 7204 and may include the AAV 3900 disposed between the connectors 7228. The AAV 3900 on the insert 7244 may be inserted into the AAV receiving region (e.g., with a press fit, friction fit, or snap fit) in order to connect the insert 7244 to the oral plenum chamber 7204. This engagement may form a seal to limit airflow from leaving the oral plenum chamber 7204 around the AAV 3900. The oral plenum chamber 7204 may also include a cut out 7248 approximately the same size as the outer perimeter of the insert 7244. When connected to the oral plenum chamber 7204, the insert 7244 may have a further engagement (e.g., with a press fit, friction fit, or snap fit) with the cut out 7248. The cut out 7248 may be recessed a depth that is approximately the same as a thickness of the insert 7244 so that the insert 7244 may be substantially flush with the oral plenum chamber 7204 when connected.

The insert 7244 may also include a projection 7252 on an anterior surface that may extend into an opening (e.g., in substantially the same location as the AAV 3900). The engagement between the projection 7252 and the opening (e.g., via a press fit) may further provide a connection between the insert 7244 to the oral plenum chamber 7204.

As shown in Figs. 17 to 19, the plenum chamber 7200 may include a first face 7232 and a second face 7236, which may together form the connecting region 7208. For example, the first face 7232 may be formed on the nasal plenum chamber 7202 and the second face 7236 may be formed on the oral plenum chamber 7204.

In some forms, the first face 7232 and/or the second face 7236 (e.g., both the first and second faces 7232, 7236) may have a thickness of about 0.1 mm thick to about 1 mm thick. In some forms, the first face 7232 and/or the second face 7236 may have a thickness of about 0.2 mm thick to about 0.75 mm thick. In some forms, the first face 7232 and/or the second face 7236 may have a thickness of about 0.3 mm thick to about 0.5 mm thick. In some forms, the first face 7232 and/or the second face 7236 may have a thickness of about 0.35 mm thick. In some forms, the thickness of the first face 7232 and/or the second face 7236 may be the same as the thickness of the remainder of the plenum chamber 7200 (although the first face 7232 and/or the second face 7236 may be thicker or thinner than other portions of the plenum chamber 7200).

In some forms, an angle 7240 may be formed between the first face 7232 and the second face 7236. In some forms, the angle 7240 may be about 1° to about 90° in a relaxed state (e.g., non-use position). In some forms, the angle 7240 may be about 5° to about 75° in a relaxed state. In some forms, the angle 7240 may be about 10° to about 50° in a relaxed state (e.g., non-use position). In some forms, the angle 7240 may be about 20° to about 40° in a relaxed state (e.g., non-use position).

In use, the first face 7232 and the second face 7236 may move relative to one another depending on the size of the patient's face (see e.g., Figs. 17-1, 18-1, and 18-2). For example, a patient with a larger face may cause the angle 7240 between the first and second faces 7232, 7236 to decrease. The connecting region 7208 may allow the movement between the first and second faces 7232, 7236 in order to decouple the nasal plenum chamber 7202 from the oral plenum chamber 7204.

As shown in Figs. 17-1, 18-1, and 18-2, the movement of the connecting region 7208 of the plenum chamber 7200 may move in the opposite direction as the connecting region 7152, 7154 of the seal-forming structure 7100. As either connection region 7152, 7154 of the seal-forming structure 7100 expands (e.g., stretches), the connecting region 7208 of the plenum chamber 7200 contracts (e.g., compresses). The connecting region 7208 of the plenum chamber 7200 may be similar to the connecting region 7152 with the comer region. In other words, the connecting region 7208 may include a pre-determined fold line to consistently determine when the pivoting occurs. This may be opposite (e.g., aligned along an axis parallel to the Frankfort horizontal in use) the corner region in the connecting region 7152 so that the pivoting is balanced in both the plenum chamber 7200 and the seal-forming structure 7100.

### 5.3.2.2 One inlet opening

As shown in Figs. 20 and 21, the patient interface 8000 may include a plenum chamber 8200 that includes separate portions for the patient's nose and for the patient's mouth. In other words, the plenum chamber 8200 may include a nasal plenum chamber 8202 and an oral plenum chamber 8204. The plenum chamber 8200 may include a connecting region 8208 between the nasal and oral plenum chambers 8202, 8204.

The plenum chamber 8200 of the patient interface 8000 may be similar to the plenum chamber 7200 of the patient interface 7000. Only some similarities and differences may be described below.

As shown in Fig. 21, the nasal plenum chamber 8202 may include one opening. For example, the nasal plenum chamber 8202 may include only one plenum chamber inlet port 8212, which may allow fluid flow into and/or out of the nasal plenum chamber 8202.

For example, the nasal plenum chamber 8202 may include a single plenum chamber inlet port 8212 disposed centrally of the nasal plenum chamber 8212. For example, the sagittal plane may intersect the plenum chamber inlet port 8212 when the patient wears the patient interface 8000. Conduits (e.g., part of air circuit 4170) may be connected (e.g., removably or permanently) to the plenum chamber inlet ports 8212. The conduits may convey the flow of pressurized breathable gas into the nasal plenum chamber 8202 through the plenum chamber inlet ports 8212.

In some forms, the plenum chamber inlet port 8212 may have a round shape (e.g., a circular shape). For example, the plenum chamber inlet port 8212 may have a similar shape to a decoupling structure, which may seal against the plenum chamber inlet port 8212 and deliver pressurized breathable gas to the nasal plenum chamber 8202.

In some forms, the nasal plenum chamber 8202 may include at least one connector 8214. For example, the nasal plenum chamber 8202 in Fig. 21 includes a pair of connectors 8214. Each connector 8214 may be disposed at a lateral side (e.g., a left or right side) of the nasal plenum chamber 8202. As described in more detail below, the connectors 8214 may connect to straps in order to secure the seal-forming structure 8100 to the patient's face during use.

As shown in Fig. 21, the oral plenum chamber 8204 may include an anti-asphyxia valve (AAV) receiving region 8224, which may form an opening that extends into the oral cavity. The AAV receiving region 8224 may be sized to receive an AAV 3900.

In some forms, the oral plenum chamber 8204 may include at least one connector 8228 on an anterior surface. For example, the oral plenum chamber 8204 may include a pair of connectors 8228 spaced apart from one another. For example, the connectors 8228 may be disposed proximate to the lateral ends of the oral plenum chamber 8204. The AAV receiving region 8224 may be disposed between the pair of connectors 8228.

In certain forms, the connectors 8228 may be magnets or constructed from a magnetic material. In other examples, the connectors 8228 may be constructed from a different material (e.g., and may be mechanical fasteners).

In certain forms, the connectors 8228 may be permanently connected to the oral plenum chamber 8204. For example, the material of the oral plenum chamber 8204 may be molded around the connectors 8228.

In certain forms, the connectors 8228 may be removable from the oral plenum chamber 8204. In some forms, an insert 7244 may be used to removably connect the connectors 8228 to the oral plenum chamber 8204. The insert 7244 may have a similar profile to the oral plenum chamber 8204 and may include the AAV 3900 disposed between the connectors 8228. The AAV 3900 on the insert 7244 may be inserted into the AAV receiving region (e.g., with a press fit, friction fit, or snap fit) in order to connect the insert 7244 to the oral plenum chamber 8204. This engagement may form a seal to limit airflow from leaving the oral plenum chamber 8204 around the AAV 3900. The oral plenum chamber 8204 may also include a cut out 8248 approximately the same size as the outer perimeter of the insert 7244. When connected to the oral plenum chamber 8204, the insert 7244 may have a further engagement (e.g., with a press fit, friction fit, or snap fit) with the cut out 8248. The cut out 8248 may be recessed a depth that is approximately the same as a thickness of the insert 7244 so that the insert 7244 may be substantially flush with the oral plenum chamber 8204 when connected.

### 5.3.2.3 Connected plenum chambers

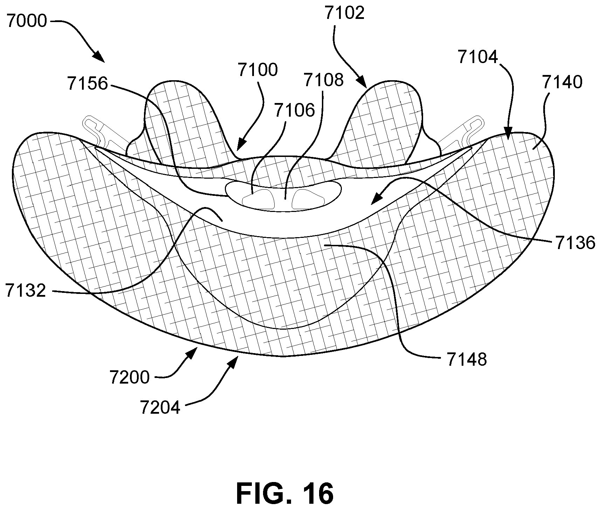

As shown in Fig. 16, the patient interface 7000 may include a passage 7156. In the illustrated example, the passage 7156 may include a round (e.g., elliptical) shape, although any other shape may be used (e.g., circular, rectangular, trapezoidal, etc.).

In some forms, the elliptical passage 7156 may include a length along a major axis of about 5 mm to about 50 mm. In some forms, the elliptical passage 7156 may include a length along a major axis of about 10 mm to about 40 mm. In some forms, the elliptical passage 7156 may include a length along a major axis of about 20 mm to about 30 mm. In some forms, the elliptical passage 7156 may include a length along a major axis of about 22 mm. In use, the major axis may be substantially perpendicular to the patient's sagittal plane.

In some forms, the elliptical passage 7156 may include a length along a minor axis (e.g., substantially perpendicular to the major axis) of about 1 mm to about 50 mm. In some forms, the elliptical passage 7156 may include a length along a minor axis of about 2 mm to about 25 mm. In some forms, the elliptical passage 7156 may include a length along a minor axis of about 5 mm to about 10 mm. In some forms, the elliptical passage 7156 may include a length along a minor axis of about 8 mm. In use, the major axis may be substantially parallel to or co-planar with the patient's sagittal plane.

In some forms, a length of the passage 7156 (e.g., along the major axis) may be larger than the area of both naris openings 7106 and the bridge portion 7108. In other words, a distance between the lateral sides or edges of the naris openings 7106 may be less than the lateral distance across the passage 7156. In some forms, the total area of the passage 7156 may also be larger than the combined area of the naris openings 7106.

As shown in Figs. 17 and 18, the passage 7156 may connect the nasal cavity 7110 and the oral cavity 7136. This may provide a fluid pathway between the nasal cavity 7110 and the oral cavity 7136.

In some forms, the passage 7156 may extend between the opposing connecting regions 7152, 7208. For example, a plane across the opening to the passage 7156 may intersect the connecting regions 7152, 7208 of the seal-forming structure 7100 and the plenum chamber 7200. The width of the passage 7156 in the anterior-posterior direction (e.g., along the minor axis) may be less than the width between the anterior surface of the plenum chamber 7200 and the seal-forming structure 7100.

In certain forms, the patient interface 7000 may be constructed with different sized passages 7156. For example, the patient interface 7000 may include a larger passage 7156 in order to allow more airflow to pass between the nasal cavity 7110 and the oral cavity 7136. Alternatively, the patient interface 7000 may include a smaller passage 7156 in order to increase mechanical decoupling between the nasal seal-forming structure 7102 and the oral seal-forming structure 7104. For example, the first face 7232 and the second face 7236 may be longer as a result of the smaller passage 7156, which may allow for more relative movement about the connecting region 7208 (or the connecting region 7152).

In use (i.e., when the seal-forming structure 7100 is sealed against the patient's face), the flow of pressurized breathable gas may enter the nasal cavity 7110 through the plenum chamber inlet ports 7212 (or the plenum chamber inlet port 8212). The patient may inhale the pressurized air into his airways through either naris opening 7106, or the pressurized air may exit the nasal plenum chamber 7202 through the vent opening 7216 (e.g., through the vent 3400). In addition to either of these options, the passage 7156 allows the flow of pressurized breathable gas to travel to the oral cavity 7136. This may allow the patient to breathe through either his nose or his mouth in order to introduce the pressurized breathable gas to his airways.

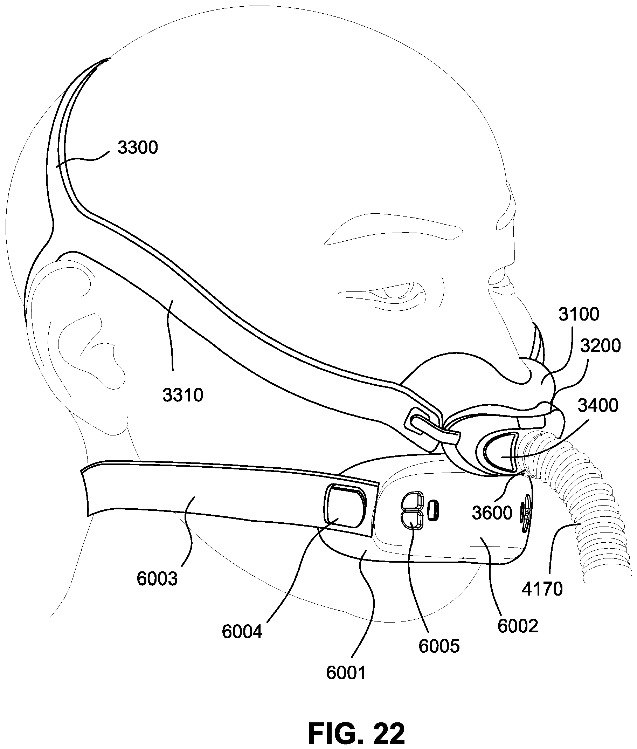

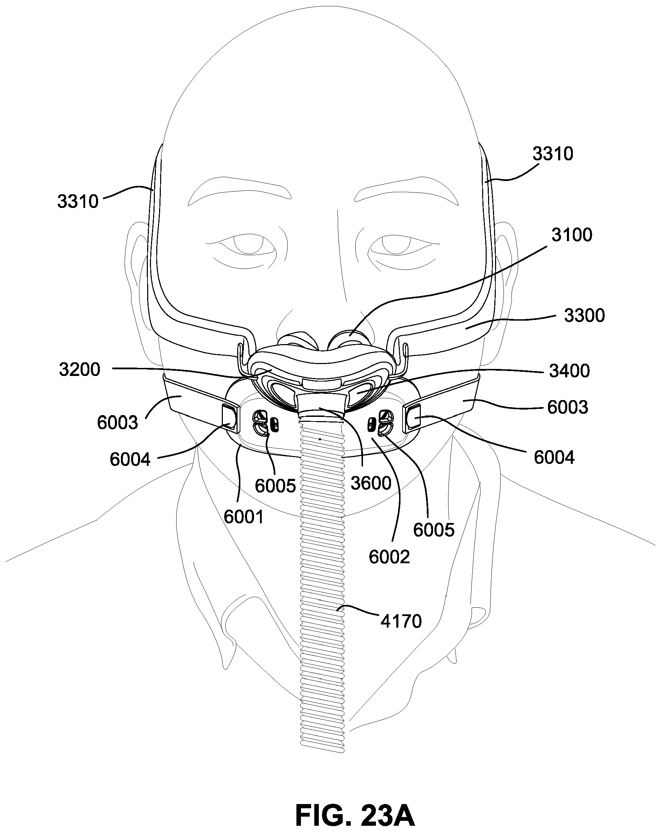

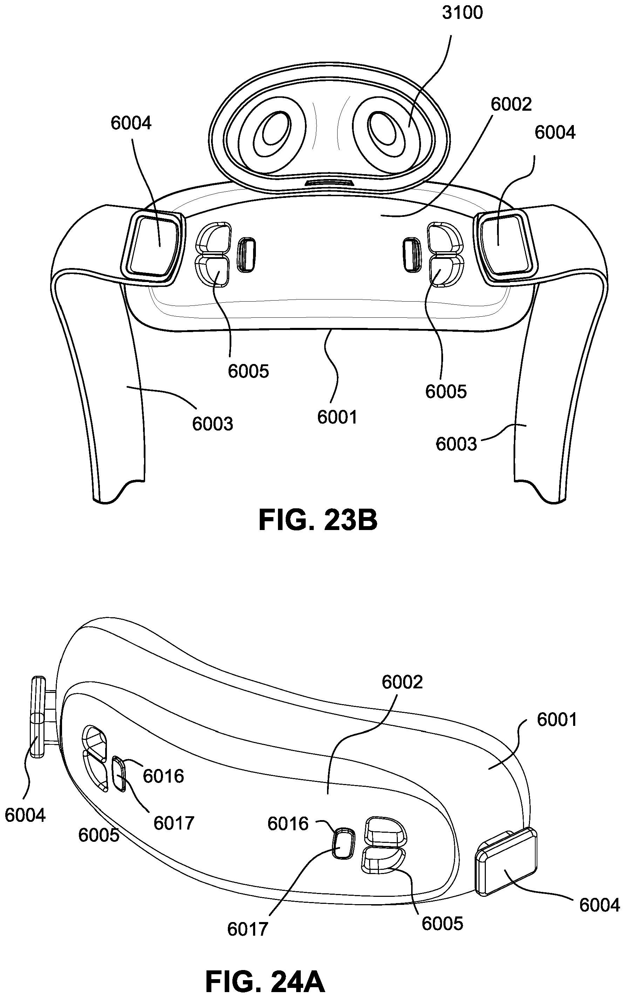

The examples of Figs. 22-23B may also include the features of the connected plenum chambers described above. For example, the nasal and oral portions may be joined with an opening approximately 5mm x 10mm to allow flow therebetween. The Fig. 22 includes a nasal cradle cushion for the nasal portion, whereas the Figs. 23A and 23B show nasal pillows for the nasal portion.

### 5.3.2.4 Separate plenum chambers

As shown in Fig. 19, the nasal cavity of 7110 of the patient interface 7000 may be at least partially isolated from the oral cavity 7136. For example, a membrane 7160 may extend between the plenum chamber 7200 and the seal-forming structure 7100 in order to limit fluid flow between the nasal cavity 7110 and the oral cavity 7136.

In some forms, the membrane 7160 way extend completely between the connecting region 7152 of the seal-forming structure 7100 and the connecting region 7208 of the plenum chamber 7200. The membrane 7160 may completely fill the region between the nasal and oral cavities 7110, 7136 so that airflow entering the nasal cavity 7110 is blocked from entering the oral cavity 7136.

In some forms, the membrane 7160 may be constructed from the same or similar material as the remainder of the patient interface 7000 (e.g., from silicone). In some forms, the membrane 7160 may have a thickness of about 0.1 mm thick to about 1 mm thick. In some forms, the membrane 7160 may have a thickness of about 0.2 mm thick to about 0.5 mm thick. In some forms, the membrane 7160 may have a thickness of about 0.3 mm thick to about 0.4 mm thick. In some forms, the membrane 7160 may have a thickness of about 0.35 mm thick.

In some forms, the membrane 7160 may be substantially thin in order to limit interfering with movement of the connecting regions 7152, 7208. For example, the thickness of the membrane 7160 may allow it to either stretch or bend as a result of movement of either connecting region 7152, 7208. In certain forms, the membrane 7160 may be slack between the connecting regions 7152, 7208 prior to use so that movement of either connecting region 7152, 7208 is not impeded by the membrane 7160.

In some forms, the membrane 7160 may be taut prior to use. The tension in the membrane 7160 may bias the connecting regions 7152, 7208 to a pre-use position (e.g., with the connecting region 7208 at an angle 7240). The tension may assist in returning the membrane 7160 to a pre-use position after the patient removes the patient interface 7000.

In use, the nasal and oral seal-forming structures 7102, 7104 may seal against the patient's face as described above, and airflow may enter the nasal cavity 7110. When the patient's face contacts the nasal seal forming structure 7102, the force may cause the first and second faces 7232, 7236 to move closer together, thereby reducing the angle 7240 of the connecting region 7208. This force also causes the connecting region 7152, 7154 to expand, as described above. In examples where the membrane 7160 is slack prior to use, the force applied by the patient's face in the anterior and/or inferior direction may further compress the membrane 7160. In examples, where the membrane 7160 is taut prior to use, the force applied by the patient's face in the anterior and/or direction may overcome the tension in the membrane 7160 and compress the membrane 7160. In still other examples, the movement of either connecting region 7152, 7154 of the seal-forming structure 7100 and the movement of the connecting region 7208 of the plenum chamber 7200 may be balanced so that the membrane 7160 may have the same level of tension and/or compression as before the patient dons the patient interface.

Because the membrane 7160 blocks the airflow from the nasal cavity 7110 to the oral cavity 7136, the patient interface may encourage the patient to breathe through his nose (i.e., and not his mouth). However, if the patient did open his mouth, mouth leak could be prevented. In other words, if the pressurized air went in the patient's nose and directly out the patient's mouth (i.e., without traveling into the patient's lungs), that pressurized air would not be exhausted to ambient. The oral seal forming structure 7104 and the oral plenum chamber 7204 create a pressurized environment within the oral cavity 7136. If air escapes through the patient's mouth as a result of mouth leak, it will be contained within the pressurized volume of the oral cavity 7136 where it could potentially be re-inhaled since the air never actually reached the patient's lungs.

As shown in Fig. 19-1, other forms of the membrane 7160 may include at least one hole 7162 (e.g., a plurality of holes) which may allow airflow from the nasal cavity 7110 to the oral cavity 7136. In other words, the membrane 7160 may not completely isolate the nasal and oral cavities 7110, 7136 from one another. The holes 7162 in the membrane 7160 may allow a predetermined amount (e.g., as determined by the number and/or size of the holes) of airflow to pass from the nasal cavity 7110 to the oral cavity 7136. This may allow the patient to breathe through his mouth, although breathing through his nose would be easier as more pressurized air would be in the nasal cavity 7110. This could also encourage patients to breathe through their nose without completely preventing them from breathing through their mouth.

In some forms, the holes 7162 may allow pressurized air to enter the oral cavity 7136 in order to help remove humidity. For example, humidity may build up in the oral cavity 7136 as a result of the patient exhaling through his mouth. The pressurized air entering the oral cavity 7136 through the holes 7162 may be less humidified and may circulate the humidified air to limit a build-up of excess humidity.

In some forms, different patient interfaces 7000 may be manufactured with different sized holes 7162. For example, patient interfaces 7000 may be manufactured with small, medium, or large holes 7162. Alternatively or in addition, the different patient interfaces 7000 may be manufactured with different numbers of holes 7162. For example, there could be three different patient interfaces 7000, each with a different number of holes 7162 through the membrane 7160. In either case (or when used in combination), the membrane 7160 in the different patient interfaces 7000 may provide different total areas of openings (e.g., a high, medium, and low) that contribute to different amounts of flow.

In certain forms, a patient may use the different patient interfaces 7000 in order to train to avoid mouth breathing. For example, a patient just beginning therapy could begin with a patient interface 7000 with no membrane 7160 (e.g., Fig. 17), or a membrane 7160 with the largest area of holes 7162. This may allow the patient to acclimate to the therapy, especially if they breathe through their mouth.

The patient may be monitored (e.g., by a sensor in the RPT device 4000 and/or by a clinician) to determine how they are adjusting to the treatment. At a certain point (e.g., when the level of detected mouth breathing drops below a predetermined level), the RPT device 4000 and/or the clinician can recommend that the patient change to a patient interface 7000 with smaller holes 7162 in the membrane 7160. This process may continue until the patient is using the patient interface 7000 that includes a membrane 7160 that blocks fluid communication between the nasal and oral portions 7202, 7204 (see e.g., Fig. 19).

This gradual change between the different sized holes 7162 in the membrane 7160 may train a mouth-breathing patient to be able to use a nasal-only mask without needing a seal around his mouth to limit mouth leak.

In still other forms, the membrane 7160 may completely separate the nasal cavity 7110 from the oral cavity 7136. However, the membrane 7160 may be constructed from a permeable material (e.g., that is configured to allow airflow to pass through). The pressurized airflow may pass through the membrane 7160 from the nasal cavity 7110 to the oral cavity 7136 so that the patient may inhale pressurized air through his mouth.

In certain forms, the membrane 7160 may have a selected or adjusted permeability in order to control the flow of pressurized breathable gas traveling from the nasal cavity 7110 to the oral cavity 7136. For example, the membrane 7160 may include a microscopic and/or molecular porosity that controls the flow rate of pressurized air into the oral cavity 7136.

The membrane 7160 may also be constructed from a different material than the remainder of the plenum chamber 7200. A material for the membrane 7160 may be selected so that airflow is able to pass through, and the material of the plenum chamber 7200 may be selected to block airflow from passing through.

In some forms, the different patient interfaces 7000 may be manufactured with different porosities. For example, patient interfaces 7000 may be manufactured with low, medium, or high porosities. The membrane 7160 in the different patient interfaces 7000 may provide different amounts of flow depending on the porosity.

Like the version above with different sized holes, patient interfaces 7000 having membranes with different porosities can be used to transition a patient from being a mouth breather to a nose breather. As described above, the patient may be monitored and may switch patient interfaces 7000 to progressively lower porosities as they breathe through their mouth less and less. Eventually, the patient may use a patient may use the patient interface 7000 that limits all fluid flow between the nasal and oral portions 7202, 7204, or the patient may use a nasal only mask.

### 5.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another suitable for a small sized head, but not a large sized head.

As shown in Fig. 12, a positioning and stabilizing structure 7300 may be used to support the plenum chamber 7200 and seal-forming structure 7100 against the patient's face. The positioning and stabilizing structure 7300 may apply a tensile force that may oppose the force of gravity. For example, the gravitational force of plenum chamber 7200 and seal-forming structure 7100 may be directed in an inferior direction (e.g., toward the floor) in use. The positioning and stabilizing structure 7300 may apply a tensile force that opposes the gravitational force so that the seal-forming structure 7100 may remain in a desired location on the patient's face.

As shown in Figs. 12 to 12-2, the positioning and stabilizing structure 7300 provides a force F_{PSS} that maintains the plenum chamber 7200 in the sealing position on the patient's face. The positioning and stabilizing force F_{PSS} may be the resultant force from the various force vectors of the different elements of the positioning and stabilizing structure 7300. In the illustrated examples, the positioning and stabilizing force F_{PSS} is represented as an "x" within a circle in order to show the force directed into the page.

In some forms, the positioning and stabilizing structure 7300 may include an upper portion and a lower portion in order to provide forces to the nasal seal forming structure 7102 and the oral seal forming structure 7104 respectively. In the illustrated example, the upper portion may include conduits 7302 that together form conduit headgear. The conduits 7302 may connect to the plenum chamber inlet ports 7212 of the nasal plenum chamber 7202 in order to convey a flow of pressurized breathable gas to the nasal cavity 7110. The conduits 7302 may be removably or permanently connector to the plenum chamber inlet ports 7212 (e.g., via a snap fit connection).

In some forms, the conduits 7302 may contact the patient's cheeks and extend toward a superior portion of the patient's head. The conduits 7302 may also extend at least partially in the posterior direction. When connected to the plenum chamber 7200, the conduits 7302 may be sized to be snug around the patient's head in order to apply a force in the superior and/or posterior direction. This force may oppose the force of gravity and/or maintain the seal-forming structure 7100 in an appropriate sealing position.

In some forms, each conduit 7302 may provide a force F_{conduit} directed in the posterior and respective lateral direction in order to hold the seal-forming structure 7100 against the patient's face (into the upper lip and sealing under the nose) and oppose the effect of the positive pressure in the plenum chamber 7200 to lift off the face (i.e., Fₚₗₑₙᵤₘ). The force F_{conduit} directed may also be directed at least partially in the superior direction in order to overcome the gravitational force F_{g}. The gravitational force F_{g} may be specifically shown for the seal-forming structure 7100 and the plenum chamber 7200, but gravity would act on the entirely of the patient interface 7000 (i.e., in the same direction as the illustrated gravitational force F_{g}).

In some forms, the conduits 7302 may include concertina sections 7304. The concertina sections 7304 may be spaced apart from the plenum chamber 7200, and may contact a superior region of the patient's head in use. The concertina sections 7304 forms stretchable sections that allow the conduits 7302 to increase in length upon the application of tension. This may allow the conduits 7302 to be designed and manufactured at one length and fit a wider range of patients. For example, the length of the conduits 7302 may be manufactured to fit snuggly against a smaller patient's head without substantial extension of the concertina sections 7304. Larger patients may stretch the concertina sections 7304 while wearing the conduits 7302 so that the conduits 7302 provide substantially the same force to all sized patients.

In some forms, the expansion of the concertina sections 7304 may decrease the total force F_{conduit} supplied by the conduits 7302. For example, the expansion of the concertina sections 7304 may produce a force directed along the length of the conduit toward the plenum chamber 7200 (e.g., in a direction opposite to the force F_{conduit}). The force of the concertina sections 7304 may produce a resultant force of F_{conduit} that is less than if the concertina sections 7304 were not present.

In some forms, the lower portion of the positioning and stabilizing structure 7300 may include headgear straps 7308. The straps 7308 may be formed form a textile or other soft and/or flexible material that is comfortable against the patient's skin. The straps 7308 may include hook and loop material and may be connected to a connector 7312. For example, each strap 7308 may be threaded through the respective connector 7312 and doubled back on itself using the hook and loop material in order to secure the straps 7308 to the connectors 7312.

The gravitational force F_{g} may be opposed by a frictional force F_{f}, which may act in a direction directly opposite of the gravitational force F_{g}. As gravity pulls then seal-forming structure 7100 and the plenum chamber 7200 in the inferior direction (as viewed in Fig. 12), the frictional force F_{f} acts in the superior direction. For example, the patient may experience the frictional force F_{f} against his lip superior (and/or other surfaces of the patient's face in contact with the seal-forming structure 7100) in order to oppose the motion in the inferior direction (which may help to stabilizing the cushion in place). Although the frictional force F_{f} is shown specifically opposing the gravitational force F_{g} of the seal-forming structure 7100 and the plenum chamber 7200, components of an overall frictional force (not shown) would also oppose the gravitational force F_{g} associated with the positioning and stabilizing structure 7300 (e.g., the conduits 7302) and any other portions of the patient interface 7000. A force of friction can act along any place where the patient interface 7000 contacts the patient's skin (or hair). The frictional force F_{f} extends in the opposite direction of the gravitational force F_{g} and along the patient's skin (or hair).

There may also be additional components of the frictional force (not shown) that oppose the tensile forces applied by the positioning and stabilizing structure 7300. As tension is applied to each individual element of the positioning and stabilizing structure 7300 (e.g., each conduit 7302, each strap 7308, etc.), friction may oppose the tension in locations where the individual elements. In other words, the patient may experience these tensile forces along his skin (or hair) in a direction directly opposite to the tensile force. Fictional forces may be directed in the anterior and/or inferior directions, or opposite the posterior and/or superior direction of the tensile force of the conduits 7302. Frictional forces may also be directed in the superior and/or anterior directions, or opposite to the inferior and/or posterior direction of straps 7308 on a posterior portion of the patient's head.

In some forms, the conduits 7302 may provide a force F_{TP} directed into the patient's head when the conduits 7302 are formed as air delivery conduits. The force F_{TP} may assist in gripping the patient's head. The force may be caused by the inflation of the conduits during normal use. In some forms, the force F_{TP} may provide a cushioning effect to the patient's head. The conduits 7302 may be designed in order to limit expansion of the conduits in order to prevent over-gripping the patient's head.

The position of the patient's head may also change the force F_{TP}. For example, if the patient is sleeping on his side (as in Fig. 12-2), the weight of the patient's head may compress one conduit, and the other conduit 7302 (e.g., the conduit 7302 not between the patient's head and a sleeping surface, like a pillow) may additionally expand (and cause a greater F_{TP}) in order to keep substantially the same flow rate of pressurized air.

In some forms, an air delivery conduit attached to the decoupling structure 3500 may provide a tube drag force F_{TD} (e.g., not shown in Fig. 12; air circuit 4170 in Fig. 20). Because the decoupling structure 3500 may be pivotable and because the patient's position may change while sleeping (e.g., rolling over), the direction of the tube drag force F_{TD} may change throughout the night, or night to night. In the illustrated examples, the tube drag force F_{TD} is represented as an "x" within a circle in order to show the force directed into the page.

In some forms, the sum of the various forces may equal zero so that the patient interface 7000 is at equilibrium (e.g., not moving along the patient's face while in use). Specifically, the gravitational force F_{g} and the blowout force Fₚₗₑₙᵤₘ tend to move the seal-forming structure 7100 away from the desired sealing position. The positioning and stabilizing force F_{PSS} is applied in order to counteract the gravitational force F_{g} and the blowout force Fₚₗₑₙᵤₘ (as well as any frictional forces F_{f}) and keep the seal-forming structure 7100 properly situated. In the illustrated examples, the blowout force Fₚₗₑₙᵤₘ is represented as a dot within a circle in order to show the force directed out of the page.

Although the positioning and stabilizing force F_{PSS} may exceed the sum of the other forces and still maintain the seal-forming structure 7100 in an appropriate sealing position, patient comfort may be sacrificed. Maximum patient comfort may be achieved when the net force on the patient interface 7000 is zero and the positioning and stabilizing force F_{PSS} is exactly strong enough to achieve this. As described below, various positions of the patient's head while using the patient interface 7000 may determine the positioning and stabilizing force F_{PSS} necessary to achieve equilibrium.

As shown in Fig. 12, the gravitational force F_{g} of the patient interface 7000 may be directed substantially perpendicular to the plenum chamber force Fₚₗₑₙᵤₘ and/or the positioning and stabilizing force F_{PSS}. In this orientation, the patient may be in an upright position (e.g., sitting up in bed). As described above, the positioning and stabilizing force F_{PSS} may have to counteract both the gravitational force F_{g} and the plenum chamber force Fₚₗₑₙᵤₘ.

As shown in Fig. 12-1, the gravitational force F_{g} of the patient interface 3000 may be directed substantially parallel to the plenum chamber force Fₚₗₑₙᵤₘ and/or the positioning and stabilizing force F_{PSS}. In this orientation, the patient may be in a reclined position (e.g., laying on his back). In this case, the gravitational force F_{g} may be directed into the patient's face (e.g. upper lip) and may counteract the plenum chamber force Fₚₗₑₙᵤₘ. In other words, the positioning and stabilizing force F_{PSS} and gravitational force F_{g} may be directed into the patient's face and may counteract the plenum chamber force Fₚₗₑₙᵤₘ. In the illustrated example, the gravitational force F_{g} is represented as an "x" within a circle in order to show the force directed into the page. The frictional force F_{f} may be oriented opposite the gravitational force F_{g} and is represented as a dot within a circle in order to show the force directed out of the page. A lower positioning and stabilizing force F_{PSS} may be needed to hold the seal-forming structure 7100 in the sealing position (and achieve equilibrium) because the gravitational force F_{g} is acting in complement to counteract the plenum chamber force Fₚₗₑₙᵤₘ.

As shown in Fig. 12-2, the gravitational force F_{g} of the patient interface 7000 may be directed substantially perpendicular to the plenum chamber force Fₚₗₑₙᵤₘ and/or the positioning and stabilizing force F_{PSS}. In this orientation, the patient may be laying on their side. As described above, the positioning and stabilizing force F_{PSS} may have to counteract both the gravitational force F_{g} and the plenum chamber force Fₚₗₑₙᵤₘ. Also, the plenum chamber 7200 and/or the positioning and stabilizing structure 7300 may tend to compress on the inferior side, yet be in tension on the superior side.

In some forms, each connector 7312 may include a magnet (not shown) that may be used to connect to the connector 7228 on the oral plenum chamber 7204. The connector 7312 may be removably connected to the connector 7228, and the patient may adjust the length of the straps 7308 (e.g., by adjusting the position that the hook and loop materials connect) so that the straps 7308 are both comfortable and provide a sufficient tensile force to maintain the position of the seal-forming structure 7100. The patient may also be able to remove each connector 7312 for the respective connector 7228 without changing the length of the strap 7308.

In use, the patient may don the positioning and stabilizing structure 7300 so that the conduits 7302 and the straps 7308 contact his head. For example, each conduits 7302 may contact the patient's head between the respective ear and eye (e.g., between the left ear and left eye) and may overlay the patient's temporal bone and/or the sphenoid bone. Each conduit 7302 may also extend toward the superior portion of the patient's head and also overlay the frontal bone and/or the parietal bone. For example, the concertina section 7304 of each conduit 7302 may overlay the frontal bone and/or the parietal bone.

**In** use, each strap 7308 may overlay the patient's masseter muscle and extend toward a posterior portion of the patient's head. The straps 7308 may meet at a posterior portion (not shown) that may contact a posterior region of the patient's head. For example, the straps 7308 may overlay the patient's occipital bone and assist in anchoring the positioning and stabilizing structure 7300 to the patient's head.

**In** certain forms (not shown), portions of the straps 7308 may also extend from the posterior portion of the patient's head in a superior and/or anterior direction, and may connect with the conduits 7302. For example, the conduits 7302 may include loops and the straps may be threaded through the loops to tighten and further secure the positioning and stabilizing structure 7300.

**In** use, the conduits 7302 apply a force to the nasal plenum chamber 7202 and the straps 7308 provide a force to the oral plenum chamber 7204. Each force provides tension to the plenum chamber 7200 and pulls the respective seal forming structure 7102, 7104 into a sealing position against the patient's face.

The connecting regions 7152, 7208 may be used to decouple the forces from the positioning and stabilizing structure 7300 from the nasal and oral plenum chambers 7202, 7204 respectively. **In** other words, the conduits 7302 (e.g., with the help of the concertina sections 7304) may be sized to provide a force to the nasal plenum chamber 7202 in order to provide a comfortable sealing force to the nasal seal forming structure 7102. The force may be specifically designed in order to provide a sufficient but not overly tight seal force against the patient's face. For example, the alar rims may be sensitive regions any may not be able to withstand large amounts of force without becoming uncomfortable. Therefore, the patient may not want the force supplied by the straps 7308 to substantially increase to force that the positioning and stabilizing structure 7300 applies to the nasal seal forming structure 7102 (or vice versa). The connecting regions 7152, 7208 may assist in decoupling the force supplied by the conduits 7302 from the oral plenum chamber 7204 and decoupling the force supplied by the straps 7308 from the nasal plenum chamber 7202. Specifically, the connecting regions 7152, 7208 may be able to pivot or flex as described above. As the force from the straps 7308 (or the conduits 7302) are applied, the connecting regions 7152, 7208 may pivot or flex as a result of absorbing at least some of the force. The movement may cause less force to be transferred from the positioning and stabilizing structure 7300 on one plenum chamber (e.g., the oral plenum chamber 7204) to the other (e.g., the nasal plenum chamber 7202). By decoupling the sealing forces through the connecting regions 7152, 7208, the straps 7308 can be tightened more to provide a good fit around the patient's mouth (e.g., where the nasolabial sulcus may be adapted to comfortably taking more force) without substantially disturbing the comfort of the nasal seal forming structure 7102.

In some forms, the movement of the connecting regions 7152, 7208, and the accompanying absorption of forces, may limit movement of the seal-forming structure 7100 against the patient's face to avoid disturbing the integrity of the seal against the patient's face. As previously described, the connecting regions 7152, 7208 may absorb or shift forces so that the applied forces to do shift the seal-forming structure and create leaks. This may be useful when the patient is asleep and wearing the patient interface 7000. For example, the patient may roll or move while sleeping, which may create different forces (e.g., tube drag) on the patient interface 7000. Movement of the connecting regions 7152, 7208 may assist in limiting the effect of the forces on the seal-forming structure 7100.

In some forms, the movement of the first and second connecting regions 7152, 7208 may change as the patient moves. For example, the positioning and stabilizing force F_{PSS} may be looser when the positioning and stabilizing structure 7300 is adjusted for a patient sleeping on his back (see e.g., Fig. 12-1) because the gravitational force F_{g} is directed at least partially in the same direction. However, if the patient moves while sleeping (e.g., rolls onto his side or stomach), the direction of the positioning and stabilizing force F_{PSS} may no longer be in a similar direction as the gravitational force F_{g} is directed, and instead at least a component of each force may oppose the other. In this situation, the positioning and stabilizing structure 7300 may need a greater positioning and stabilizing force F_{PSS} to hold the seal-forming structure 7100 in the sealing position. The connecting regions 7152, 7208 may move toward the pre-use position (e.g., the angle 7240 increases) in order to release some of the stored force.

In some forms, the positioning and stabilizing force F_{PSS} may be tighter when the positioning and stabilizing structure 7300 is adjusted for a patient sleeping on his side (see e.g., Fig. 12-2) or stomach because the gravitational force F_{g} is directed at least partially in the same direction. If the patient moves while sleeping (e.g., rolls onto his back), the direction of the positioning and stabilizing force F_{PSS} may now be in a similar direction as the gravitational force F_{g} is directed, and at least a component of each force may act in the same direction. In this situation, the positioning and stabilizing structure 7300 may need less positioning and stabilizing force F_{PSS} to hold the seal-forming structure 7100 in the sealing position. The connecting regions 7152, 7208 may further move to decouple the forces (e.g., the angle 7240 decreases) in order to decouple the force.

In some forms, movement of the first and second faces 7232, 7236 to reduce the angle 7240 may not cause substantial discomfort in the patient. Because the first and second faces 7232, 7236 are on an anterior surface of the plenum chamber 7200, the patient's skin may not be pinched as the angle decreases. This allows for freer pivoting without risking disturbing the patient.

Although the description above is specification related to the connecting region 7152 of the seal-forming structure 7100, the description is equally applicable to the connecting region 7154.

As shown in Fig. 20, the positioning and stabilizing structure 8300 may be similar to the positioning and stabilizing structure 7300 of Fig. 12. Only some similarities and differences may be described below.

In some forms, the positioning and stabilizing structure 8300 may include headgear straps. The headgear straps may be constructed from a comfortable and/or flexible material in order to maximize comfort against the patient's face. For example, the headgear straps may be constructed from a textile material.

In some forms, the positioning and stabilizing structure 8300 may include upper straps 8304 and lower straps 8308. The upper straps 8304 may include hook and loop material and may wrap around the connectors 8214 of the nasal plenum chamber 8202. The patient may thread the upper straps 8304 and connect the hook and loop material at a desired location to provide a selected force on the nasal plenum chamber 8202.

In use, the upper straps 8304 may contact the patient's head in a similar location as the conduits 7302. The upper straps 8304 may contact the patient's head between the respective ear and eye and may overlay the temporal bone and/or the sphenoid bone. However, the upper straps 8304 may not extend to the superior portion of the patient's head (e.g., and may not overlay the frontal bone). Instead, the upper straps 8304 may extend in the posterior direction and may overlay the parietal bone and/or the occipital bone. In this way, the upper straps 8304 may provide a tensile force directed in a more posterior direction than the conduits 7302 provide.

In some forms, the upper straps 8304 may be similar to the conduits 7302, and may assist in providing a similar force to counteract the force of gravity.

In some forms, the positioning and stabilizing structure 8300 may also include lower straps 8308 that may be similar to the straps 7308 of the positioning and stabilizing structure 7300. Each lower strap 8308 may also connect to a connector 8312, which may include a magnet for removably connecting to the connectors 8228. The lower straps 8308 may contact the patient's face in substantially the same location as the lower straps 7308, and may be similarly tightened by threading a different amount of material through the connector 8312.

In use, the connecting region 8208 (as well as the unseen connecting region of the seal-forming structure 8100) may decouple the nasal plenum chamber 8202 from the oral plenum chamber 8204 in a similar manner as the connecting regions 7152, 7208 described above. This may allow the forces applied to the oral plenum chamber 8204 to be substantially decoupled form the nasal plenum chamber 8202.

In alternate forms where the patient interface is a full-face mask, a connecting region may be formed in the middle of the plenum chamber (e.g., which may be formed at least partially from a flexible material) so that forces applied near the mouth (e.g., by lower headgear straps) do not substantially affect the seal against the patient's nose (e.g., which may be more sensitive).

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes 3404, for example, about 20 to about 80 holes 3404, or about 40 to about 60 holes 3404, or about 45 to about 55 holes 3404.

The vent 3400 may be located in the plenum chamber 3200. Alternatively, the vent 3400 is located in a decoupling structure, e.g., a swivel.

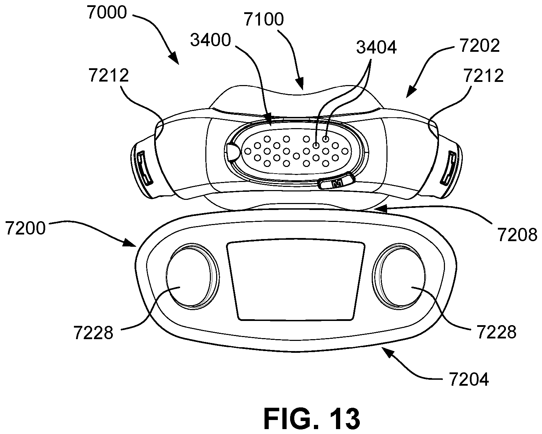

As shown in Fig. 13, the vent 3400 may be coupled to the nasal plenum chamber 7202 between the plenum chamber inlet ports 7212. Holes 3404 may be disposed throughout the vent 3400 and oriented to exhaust air (e.g., exhaled carbon dioxide) in an anterior direction away from the patient's face.

With reference to Fig. 14, the vent 3400 may be positioned in the vent receiving region 7220 and/or the vent opening 7216 when connected to the nasal plenum chamber 7202. In some examples, the vent 3400 may be removably connected to the nasal plenum chamber 7202. Patients may remove the vent 3400 after use in order to clean and/or replace the vent 3400.

In some forms, the vent 3400 may be slightly larger than the vent opening 7216. A flexible material of the nasal plenum chamber 7202 may stretch as the vent 3400 is inserted into the vent opening 7216. Once inserted, the vent 3400 may be retained against the vent receiving region 7220 may a friction fit or press fit with the vent opening 7216.

In some forms, the vent 3400 may be retained against the vent receiving region 7220 using at least one clip 7222 (or similar mechanical fastener). The clip 7222 may be used in addition to or instead of the press fit or friction fit. The clips 7222 may engage an edge of the vent 3400 in order to limit translation of the vent 3400 away from the vent opening 7216 (e.g., in an anterior direction in use). The vent 3400 may engage the clips 7222 with a snap fit in order to retain the vent 3400 in position.

In other examples, the vent 3400 may be permanently connected to the nasal plenum chamber 7202 and may not be removed by the patient. In certain forms, the vent 3400 may include a filter (not shown), which may be removable and/or replaceable, even if the vent 3400 is not.

### 5.3.5 Decoupling structure(s)

In one form the patient interface 3000 includes at least one decoupling structure 3500, for example, a swivel or a ball and socket. The decoupling structure 3500 may also be referred to as an elbow. As shown in Fig. 12, the decoupling structure 3500 is connected between the concertina sections 7304 and is positioned at a superior portion of the patient's head in use.

Fig. 20 shows another form, where the decoupling structure 3500 is connected (e.g., directly connected or using an intermediate connector) to the nasal plenum chamber 8202. The decoupling structure 3500 may extend in the anterior direction from the patient's face, in use.

### 5.3.6 Connection port

Connection port 3600 allows for connection to the air circuit 4170.

As shown in Fig. 12, a connection port 7600 may be connected between the conduits 7302. For example, the connection port 7600 may be formed between the concertina sections 7304. In use, the connection port 7600 may be positioned at the top of the patient's head.

### 5.3.7 Forehead support

In one form, the patient interface 3000 includes a forehead support 3700.

### 5.3.8 Anti-asphyxia valve

In one form, the patient interface 3000 includes an anti-asphyxia valve (AAV) 3900.

As shown in Figs. 14 and 21, the oral plenum chamber 7204, 8204 may include an AAV 3900. The AAV 3900 may be substantially centered on the oral plenum chamber 7204, 8204 and may be disposed in the superior-inferior direction in order to approximately align with the patient's mouth while the patient interface 7000, 8000 is worn.

With continued reference to Figs. 14 and 21, the AAV 3900 may be formed through an anterior wall of the oral plenum chamber 7204, 8204. The AAV 3900 may extend directly into the oral cavity 7136 (or the unillustrated oral cavity in the oral plenum chamber 8204) and may provide fluid communication between the oral cavity 7136 and the ambient environment.

In other forms, the oral plenum chamber 7204 may include an opening that extends directly into the oral cavity 7136 (e.g., the AAV 3900 in Fig. 14 may be replaced with an opening). As shown in Fig. 14-1, an insert 7244 may include the AAV 3900 and may removably connect to the oral plenum chamber 7204. For example, a posterior surface of the insert 7244 may include a projection 7252 that may be inserted into the opening with a press fit. As described above, the insert 7244 may be substantially flush with the remainder of the anterior surface of the oral plenum chamber 7204. Although this is described with respect to the patient interface 7000 in Fig. 14, the same or similar insert 7244 may be used with the patient interface 8000.

In use, the AAV 3900 may include a flap (not shown) that may selectively covers the opening to the AAV 3900. For example, when pressurized air flows into the oral cavity 7136, the pressure from the airflow may close the flap of the AAV 3900 so that the pressurized air is unable to exhaust to ambient. This may be used in the patient interface 7000 of Fig. 17 when the nasal cavity 7110 and the oral cavity 7136 are connected to one another. The flap may be biased toward an open position so that when the flow of pressurized air stops, the AAV 3900 is open and the patient may inhale air from the ambient without removing the patient interface 7000.

In other examples like Fig. 19 where the oral cavity 7136 is separated from the nasal cavity 7110 (e.g., by the membrane 7160), the AAV 3900 may be incorporated into the vent 3400 so that the vent 3400 may be fully open when the flow of pressurized air stops, and the patient may inhale ambient air through his nose. In some forms, the oral plenum chamber 7204 may include a one-way vent and/or a through hole so that the patient may be able to breathe through his mouth in the absence of the flow of pressurized air. With the one-way vent, the AAV 3900 may include a flap that is biased toward a normally closed position to prevent pressurized air from escaping to ambient from the patient's mouth. The flap may be configured to be pulled into the oral cavity 7136 into an open position by negative pressure within the oral plenum chamber 7204 in response to oral inhalation by the patient. The flap may return to the closed position when pressure with the oral plenum chamber 7204 is equal to or greater than ambient air pressure.

In some forms like in Figs. 20 and 21, the patient interface 8000 may alternatively or in addition include an AAV 3900 on the elbow 3500. A flap in this AAV 3900 may be biased to a normally opened position, and may move to a closed position as a result of the flow or pressurized air. The flap may return to the opened position when the flow of pressurized air stops, which may allow the patient to inhale ambient air.

In certain forms, the AAV 3900 in the oral plenum chamber 7204 may allow a certain amount of air to continuously vent to the ambient. For example, in patient interfaces 7000 that allow fluid communication between the nasal and oral cavities 7110, 7136, some venting may occur through the AAV 3900 (or through a surrounding, separate vent) that allows an amount of waste gas (e.g., exhaled carbon dioxide) to escape the oral cavity 7136 and limit build-up.

### 5.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 5.3.10 Mouth Leak Controlling Device

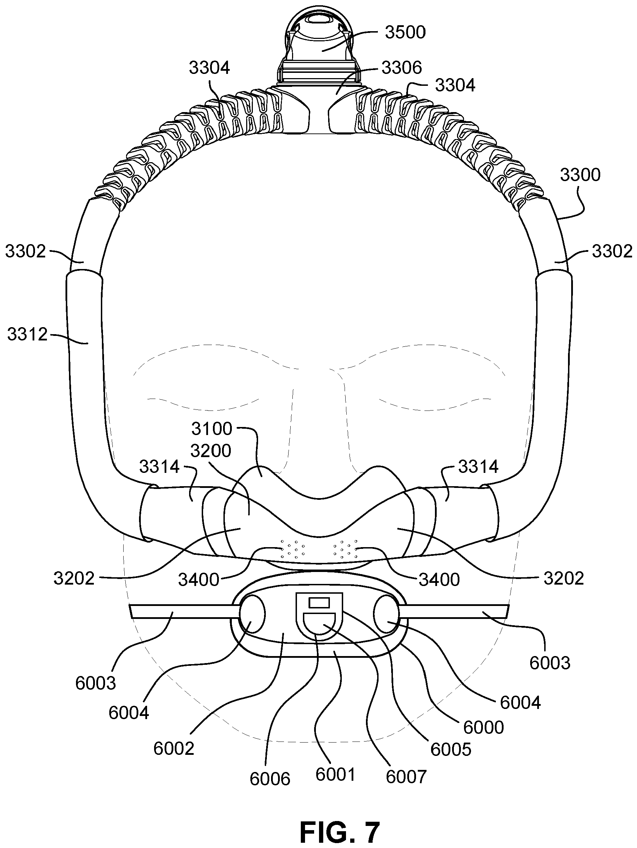

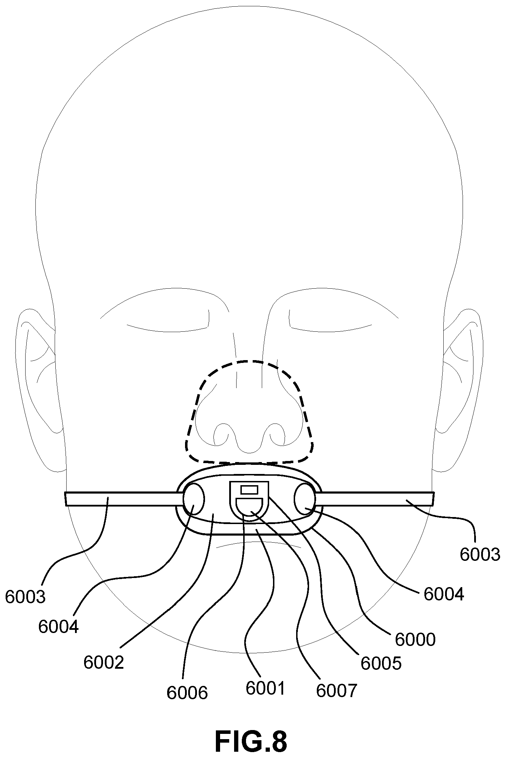

Figs. 7-11C shows examples of a mouth leak controlling device 6000 of the present technology, as will be described below. When a patient receives respiratory pressure therapy through only the nose, which may be more comfortable for some patients and may also be preferable therapeutically, there is a risk that the patient's mouth may open during therapy. If the patient's mouth opens while receiving nasal respiratory pressure therapy, the pressurized flow air may leak out of the mouth, thereby reducing the therapeutic benefit of such therapy. The mouth leak controlling devices 6000 disclosed below cover the patient's mouth during therapy so that if the patient's mouth opens during therapy, the pressurized flow of air is prevented from being discharged to directly to ambient from the patient's mouth. This may allow the patient to continue to receive the therapeutically beneficial pneumatic airway splint described above.

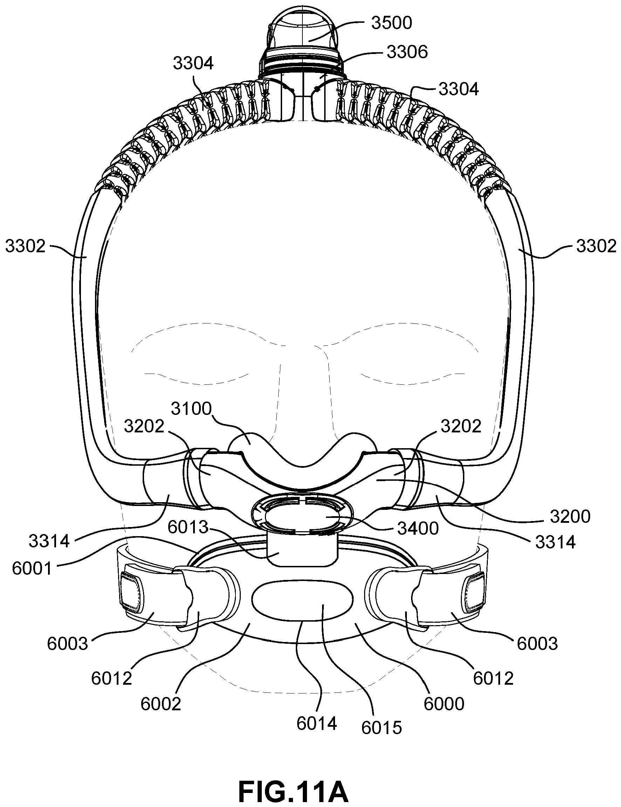

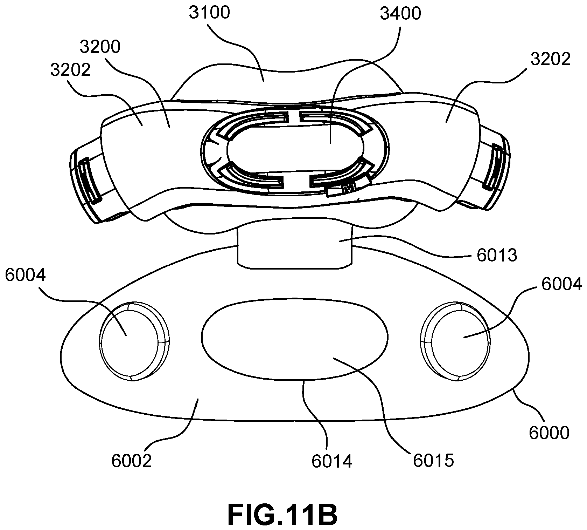

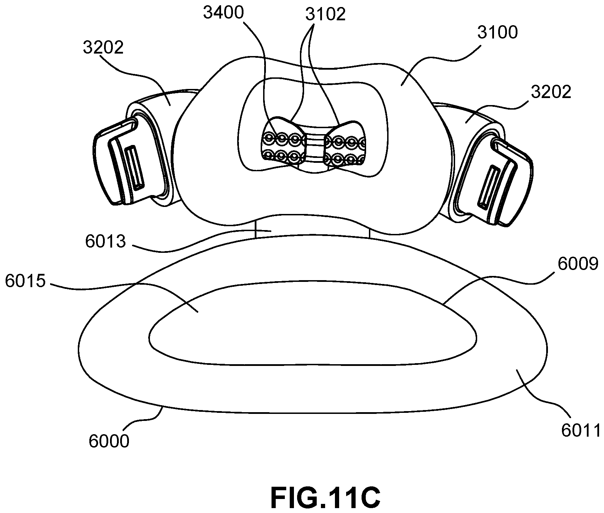

Figs. 7 and 11A each show examples of the mouth leak controlling device 6000 worn, along with the patient interface 3000, by the patient. The patient interface 3000 may include a positioning and stabilising structure 3300. In these examples, the positioning and stabilising structure 3300 includes lateral portions 3302 and superior portions 3304 in the form of conduits or tubes that direct a flow pressurised gas from a hub 3306 to ends 3314. The positioning and stabilising structure 3300 may be arranged such that the hub 3306 and the decoupling structure 3500 are positioned superior to the patient's head in use. The decoupling structure 3500 may be rotatable within the hub 3306 and when the patient is wearing the patient interface 3000, *e.g.,* during therapy, the location of the hub 3306 and the decoupling structure 3500 superior to the patient's head allows the patient to move more freely without becoming entangled with the air circuit 4170.

The patient interface 3000 may also include a seal-forming structure 3100 and a plenum chamber 3200. At each lateral side of the plenum chamber 3200 there may be a plenum chamber lateral end 3202 in the form of a hollow passageway. A plenum chamber connector 3204 may also be provided at each lateral side of the plenum chamber 3200 laterally outward of the plenum chamber lateral end 3202. The plenum chamber connectors 3204 may connect to respective ends 3314 of the positioning and stabilising structure 3300. The connection between the plenum chamber connectors 3204 and respective ends 3314 of the positioning and stabilising structure 3300 may be releasable at both sides. In other examples, one side may have a permanent connection while the other side has a releasable connection. In still further examples, both connections between the plenum chamber connectors 3204 and respective ends 3314 of the positioning and stabilising structure 3300 may be permanent.

The plenum chamber lateral ends 3202 may receive the flow of pressurised gas from the positioning and stabilising structure 3300. The flow of pressurised gas may then pass through the plenum chamber 3200, then through the seal-forming structure 3100, and into the patient's airways for inhalation.

The vent 3400 may be located in the plenum chamber 3200. The plenum chamber vent 3400 may comprise a plurality of holes, as described above. The holes of the plenum chamber vent 3400 may be divided into two groups spaced apart laterally. The axis of the flow path through each of the holes of the plenum chamber vent 3400 may be parallel such that cross-flow is avoided to prevent generation of additional noise. The vent holes may be circular.

Although the depicted examples show the mouth leak controlling device 6000 used in conjunction with the patient interface 3000, it should also be understood that the mouth leak controlling device 6000 could be used on its own without the patient interface 3000 as well.

### 5.3.10.1 Valved Mouth Leak Controlling Device

Figs. 7-10B show examples of the present technology that include a valve 6005 on the mouth leak controlling device 6000 to allow the patient to breathe orally from ambient in the absence of the pressurized flow of air to the patient interface 3000.

The mouth leak controlling device 6000 may seal around the patient's lip inferior and lip superior to prevent leak from the mouth while using nasal patient interface 3000. The mouth leak controlling device 6000 may be constructed without any connector to receive an incoming flow of pressurized air from an RPT device 4000. The mouth leak controlling device 6000 may form a cavity anterior to the patient's mouth to prevent air leaking from the patient's mouth to ambient during nasal CPAP therapy. The mouth leak controlling device 6000 may include a flexible portion 6001 that is configured to contact the patient's lip superior and lip inferior and leave the patient's nose uncovered during use. The flexible portion 6001 may also have an oral hole 6009. The flexible portion 6001 may be connected to a support portion 6002 that supports the flexible portion 6001 against the patient's face during use. The flexible portion 6001 may be constructed from silicone rubber. The support portion 6002 may be constructed from a more rigid material than the flexible portion 6001. The support portion 6002 may be constructed from polycarbonate. In further examples, flexible portion 6001 may be overmolded onto the support portion 6002. The support portion 6002 and the flexible portion 6001 may each be constructed from silicone with the support portion 6002 being constructed from a less flexible silicone. In further examples, the support portion 6002 may be flexible, but less flexible than the flexible portion 6001, to allow the mouth leak controlling device 6000 to flex and seal on a broader range of mouth profiles.

The flexible portion 6001 may have a c-shaped wall profile that may increase in thickness from 0.7-2.0mm at the edge surrounding the oral hole 6009 to where the flexible portion 6001 connects to the support portion 6002. The material of the flexible portion 6001 may be an elastomeric material with a hardness of approximately 30 to approximately 45 Shore A. The flexible portion 6001 may alternatively be constructed of a compressible foam. If silicone is used for the flexible portion 6001 it may be covered by a fabric at face-contacting locations for additional comfort in further examples. In still further examples, the flexible portion 6001 may have a thickness in the range of approximately 0.2 mm to 0.3 mm for increase comfort at sensitive facial regions.

The oral hole 6009 may be shaped and dimensioned such that the patient's upper and lower vermilion and cheilions fit therein. The flexible portion 6001 may also be sufficiently stiff so as to restrict the lips from opening when the patient's upper and lower vermilion and cheilions are within the oral hole 6009. Furthermore, upper and lower edges of the oral hole 6009 may be positioned above and below the upper and lower vermilion, respectively.

Corners of the flexible portion 6001 may also be shaped to be relatively firm so as to maintain the flexible portion 6001 in position on the patient's face during use. The peaked corners may be located just outside or just inside the cheilions.

The mouth leak controlling device 6000 may also include a positioning and stabilizing structure, e.g., in the form of a strap 6003 to retain the flexible portion 6001 against the patient's face during use. The mouth leak controlling device 6000 may also include connectors 6004 to releasably connect the strap 6003 to the mouth leak controlling device 6000. The connectors 6004 may be formed on the support portion 6002. The connectors 6004 and the strap 6003 may be releasably connected via magnetic clips 6012 in an example. Alternatively, a hook and loop connection may be used to releasably connect the connectors 6004 and the strap 6003. The strap 6003 may also be elastic to allow for length adjustment.

**In** a further example, the strap 6003 may be formed from two portions, each permanently connected to the support portion 6002 at a first end and joinable to one another at respective second ends, e.g., via a hook and loop connection. One of the portions may have loop material at its second end and the other may have hook material at its second end. The hook material may be oriented on its respective portion to face away from the patient during use to avoid discomfort that may be caused by contact with the hook material. Also, one or the other of the hook and loop materials may be longer than the other to allow attachment between respective second ends for length adjustment. **In** a further variation of this example, a loop may be permanently connected to one lateral side of the support portion 6002 and the strap 6003 may be connected permanently to the other lateral side of the support portion 6002. The free end of the strap 6003 may be passed through the loop and connected back onto itself, e.g., with a hook and loop connection, to secure the mouth leak controlling device 6000 onto the patient's head and to allow length adjustment. The free end of the strap 6003 may have a portion of hook or loop material and a portion of the other of hook or loop material may be positioned along a portion of the strap 6003 to allow the free end to be attached thereto at various locations as desired by the patient for a secure fit.

In a further example, the strap 6003 may be formed from a single piece of elastic material to allow for length adjustment when passed around the back of the patient's head/neck. The strap 6003 may be permanently connected at each end to the support portion 6002.

The mouth leak controlling device 6000 may also have a valve 6005, e.g., an AAV, to allow air to flow in and out of the mouth leak controlling device 6000 if there is no internal pressure, e.g., in the absence of pressurized air flow from the RPT device 4000 to the patient interface 3000. If the pressure within the mouth leak controlling device 6000 increases during nasal CPAP therapy due to opening of the patient's mouth, the valve 6000 includes a valve flap 6007 that closes off a valve hole 6006 to prevent pressurized air from escaping to ambient from the patient's mouth. The valve hole 6006 may be formed in a valve frame 6008. The valve flap 6007 may be configured to be pulled away from the valve frame 6008 to open the valve hole 6006 by negative pressure within the mouth leak controlling device 6000 in response to oral inhalation by the patient. The valve flap 6007 may also be configured to occlude the valve hole 6006 when pressure with the mouth leak controlling device 6000 is equal to or greater than ambient air pressure.

The mouth leak controlling device 6000 may also include a vent to allow a flow of air to pass to ambient continuously throughout the patient's respiratory cycle and independent of whether the patient's mouth is open. This may allow air warmed and moistened within the mouth leak controlling device 6000 by the patient to escape, otherwise that warm, moist air could accumulate inside of the mouth leak controlling device 6000 and cause discomfort for the patient. The vent may be positioned on the support portion 6002. The vent may be positioned on the flexible portion 6001. The valve 6005 may include the vent. The vent may include one or more holes that are configured to remain uncovered and open throughout the patient's respiratory cycle. The vent may be configured to permit a vent flow to ambient equal to or less than approximately 5L/min at 12 cmH₂O of pressure within the mouth leak controlling device 6000.

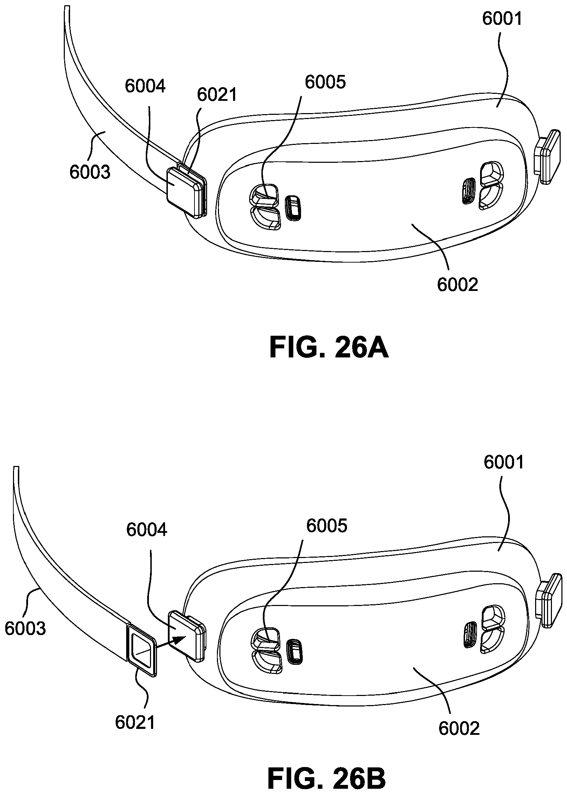

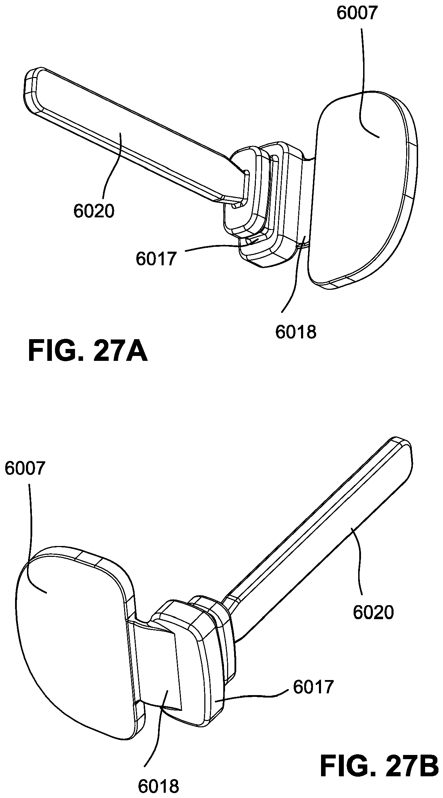

Figs. 22-27A show further examples of a patient interface 3000 with a mouth leak controlling device 6000.

In these examples, the mouth leak controlling device 6000 includes the flexible portion 6001 joined to the support portion 6002. The support portion 6002 may be more rigid than the flexible portion 6001, which contacts the patient's face around the mouth during use, so that the support portion 6002 can resist forces that might otherwise deform the flexible portion 6001 and disrupt the seal with the patient's face around the mouth. The support portion 6002 and the flexible portion 6001 may be constructed from different materials, e.g., polycarbonate and silicone, respectively, or the support portion 6002 and the flexible portion 6001 may be constructed from the same material, e.g., silicone, but the support portion 6002 being thicker and/or having a higher durometer to increase rigidity.

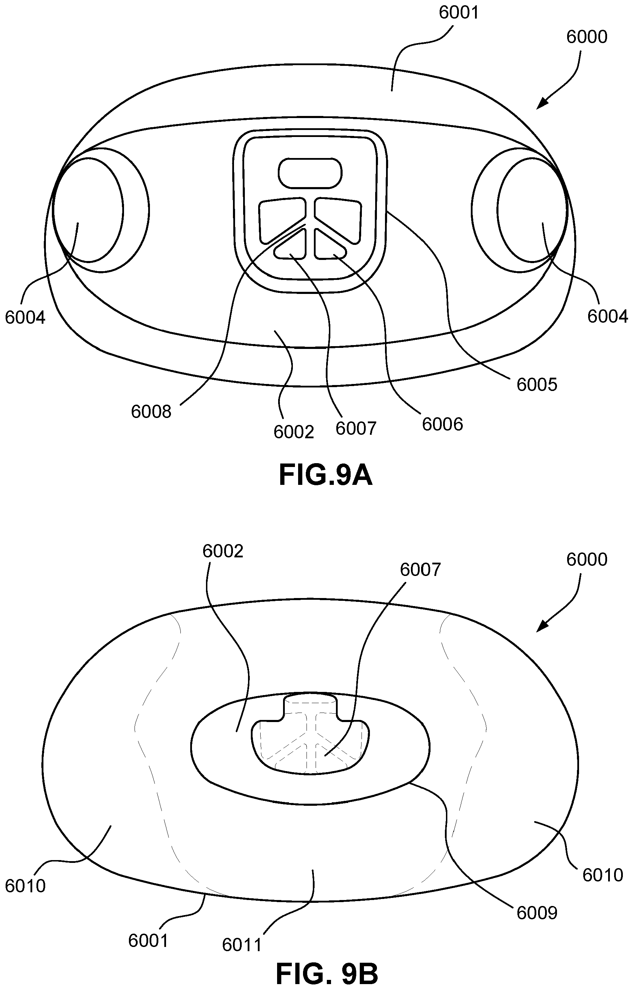

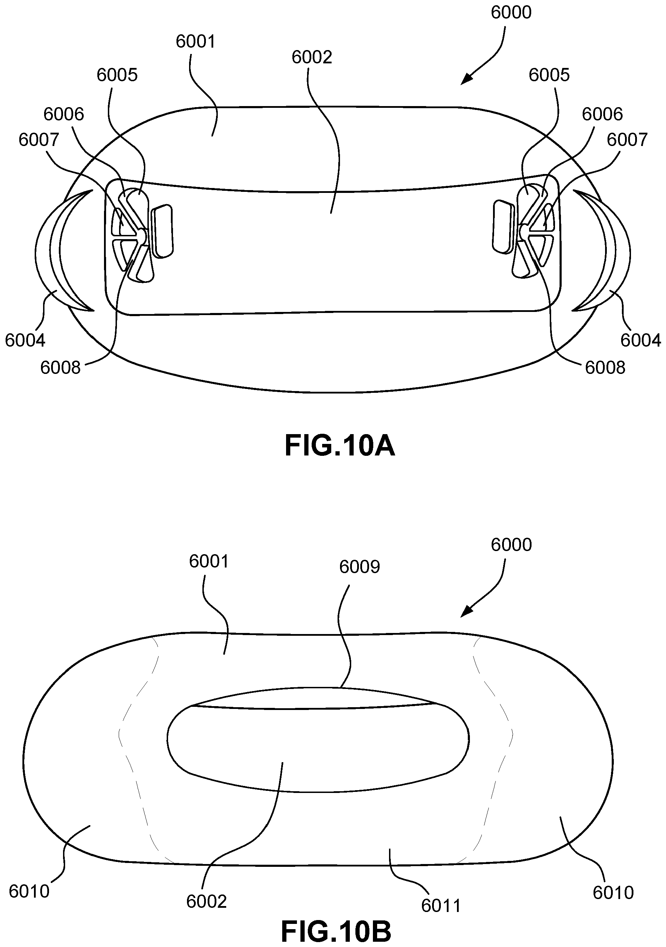

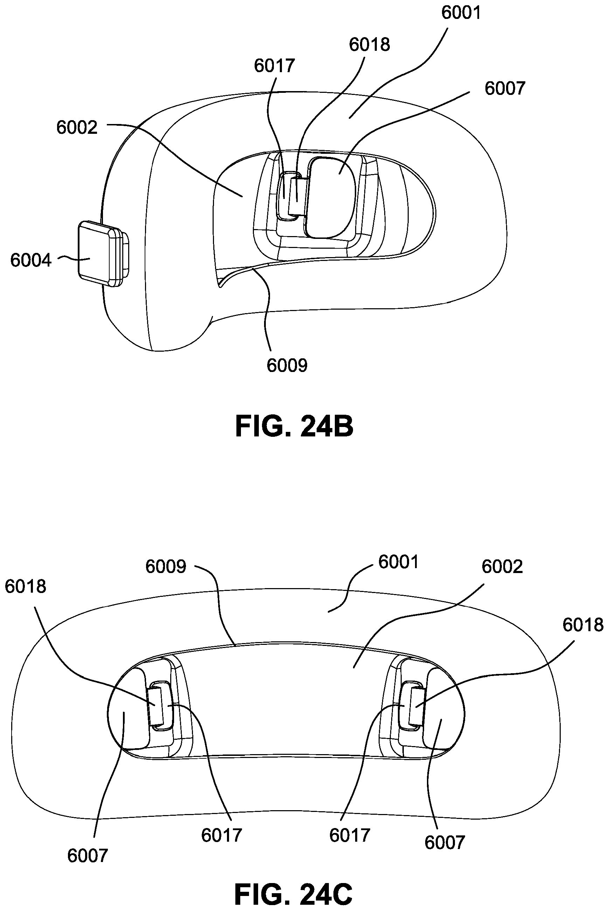

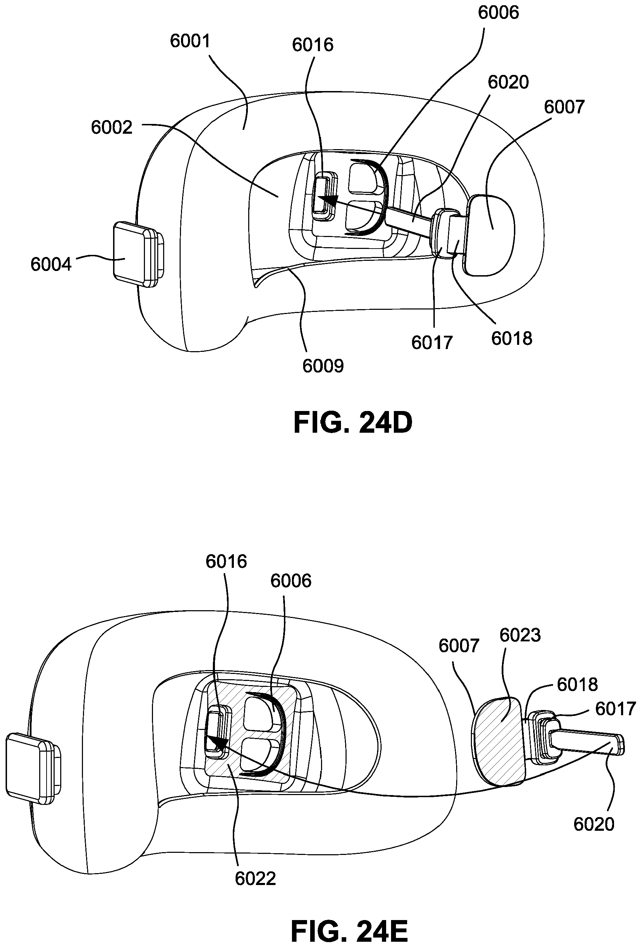

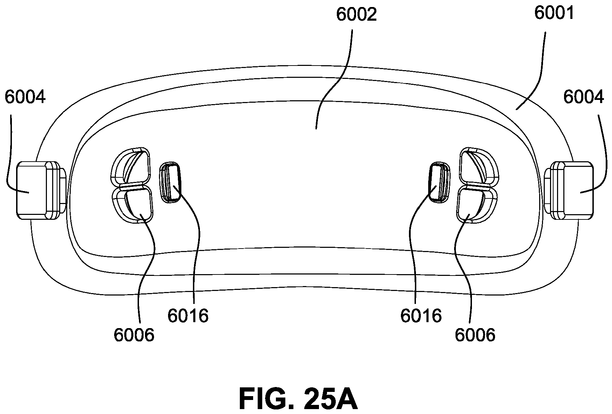

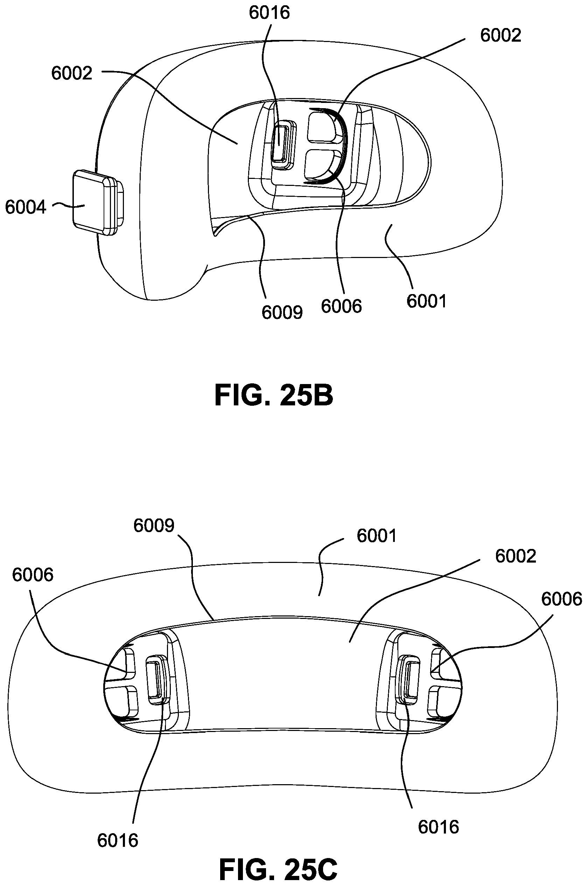

Figs. 24C and 25C also show that the corners of the flexible portion 6001 are squarer in shape as compared to the flexible portion 6001 in Figs. 9B and 10B. This may allow for greater clearance around the patient's mouth to ensure more complete contact and sealing around the patient's mouth. Also, the squared-off corners may be more rigid and resistant to deformation, which may disrupt sealing.

These examples show the mouth leak controlling device 6000 with the oral hole 6009 in the flexible portion 6001. In other examples, the flexible portion 6001 may be solid across where it contacts the patient's mouth and does not have an oral hole 6009. In this example, there may be no valves 6005 because the patient's mouth is covered by the flexible portion 6001 and the mouth leak controlling device 6000 cannot receive flow/pressure from the patient's mouth.

In these examples, the mouth leak controlling device 6000 includes two valves 6005, but one or more than two valves 6005 may be included in variations. Each valve 6005 includes two valve holes 6006, but one or more than two valve holes 6006 may be included in variations. Each valve 6005 also includes one valve flap 6007 that is movable in response to flow/pressure changes within the mouth leak controlling device 6000. The valve flap 6007 of each valve 6005 may be sized and shaped to cover both valve holes 6006 in the closed position. Alternatively, there may be one valve flap 6007 to cover each valve hole 6006, whether there is a single or multiple valve holes 6006 per valve 6005. In a further alternative with multiple valve holes 6006 per valve 6005, each valve flap 6007 may be sized and shaped to cover all of the valve holes 6006 in a given valve 6005.

The valve flap 6007 in these examples is joined to a tab hole 6016 in the support portion 6002 by a flap tab 6017. The valve flap 6007 and the flap tab 6017 are connected by a flexible joint 6018. An assembly tab 6020 may be formed on the flap tab 6017 to facilitate assembly by passing through the tab hole 6016 and then being pulled to draw the flap tab 6017 into the tab hole 6016. The valve flap 6007, flap tab 6017, flexible joint 6018, and assembly tab 6020 may be constructed from a single piece of flexible material, such as silicone. Alternatively, one or more of these components may be constructed from a rigid material, although the flexible joint 6018 would remain sufficiently flexible so as to allow the valve flap 6007 to move in response to expected/predetermined pressure/flow changes. Once the flap tab 6017 is inserted into the tab hole 6016, the assembly tab 6020 may be removed from the flap tab 6017. The flexible joint 6018 may extend from the flap tab 6017 such that the valve flap 6007 is positioned near enough to the valve hole(s) 6006 to close them in response to a predetermined increase flow/pressure within the mouth leak controlling device 6000 but not so close that the valve hole(s) 6006 are closed by the valve flap(s) 6007 in an undeformed state. **In** other words, the flexible joint 6018 may be designed to require a predetermined amount of deformation for the valve flap(s) 6007 to move into the closed position.

A flap surface 6023 of the valve flap 6007 may have a frosted finish and a valve surface 6022 of the support portion 6002 surrounding the valve hole(s) 6006 may also have a frosted finish. The frosted finishes may reduce friction between the valve surface 6022 and the flap surface 6023 when the valve flap 6007 is in the closed position, which allows the valve flap 6007 to easily open in response to changes in flow/pressure.

**In** further examples, the valve flap(s) 6007 may be sized and shaped such that one or more of the valve holes 6006 may remain uncovered when the valve flap(s) 6007 close, or there may be one or more other vent holes in the support portion 6002. The holes that remain open regardless of the position of the valve flap(s) 6007 may allow air from within the mouth leak controlling device 6000 to leak to atmosphere at a relatively low rate such that the position of the valve flap(s) 6007 is not otherwise disrupted by humidity accumulated in the mouth leak controlling device 6000 from the patient's breath can be released to atmosphere. The hole(s) that remain open regardless of the position of the valve flap(s) 6007 may be sized to allow a flow rate of 2l/min at 10cmH2O to 30L/min at 10cm H2O.

The mouth leak controlling device 6000 may also include a connector 6004 positioned on each lateral side thereof. The connectors 6004 are shown on the flexible portion 6001 in these examples, but they may be positioned on the support portion 6002 in other examples. The connectors 6004 may have a mushroom-like shape in which the cross-section is relatively narrow at the base and increases away from the mouth leak controlling device 6000. This may allow a receiver 6021 on each of the straps 6003 to attach to the connector 6004 by the connector 6004 extending the connector 6004 through the receiver 6021. Additionally, this allow the straps 6003 to be removable from the mouth leak controlling device 6000. The straps 6003 may also be elastic or inelastic. The straps 6003 may also be length-adjustable. In alternatives, the connectors 6004 and the receivers 6021 may be releasably connect by magnets, hook and loop connections, or clips.

### 5.3.10.2Porous Mouth Leak Controlling Device

Figs. 11A-11C show examples of the present technology that include a porous structure 6015 in the mouth leak controlling device 6000. This example may include features similar to the example of the mouth leak controlling device 6000 described above, except that instead of the valve 6005 and the vent, it uses an air flow hole 6014 in the support portion 6002 that is covered with the porous structure 6015 to restrict air flow into and out of the mouth leak controlling device 6000.

This example also includes a patient interface connector 6013 that may attach the mouth leak controlling device 6000 to the underside of the patient interface 3000, e.g., the plenum chamber 3200 or the seal-forming structure 3100. The patient interface connector 6013 may be relatively thin (e.g., approximately 0.5mm), and it may be constructed from silicone or another flexible material. The patient interface connector 6013 may be rectangular in shape. The patient interface connector 6013 may be glued at each end to the mouth leak controlling device 6000 and to the patient interface 3000. Alternatively, the patient interface connector 6013 may be moulded in one piece with one or both of the mouth leak controlling device 6000 and the patient interface 3000. In a further alternative, the patient interface connector 6013 may be detachable, for example to allow the mouth leak controlling device 6000 to be used with different patient interfaces 3000. The flexible nature of the patient interface connector 6013 may allow the mouth leak controlling device 6000 fit to many different facial geometries. Attaching the mouth leak controlling device 6000 to the patient interface 3000 may allow for increased stability in use and for easier fitting as the mouth leak controlling device 6000 may be held approximately in position relative to the patient's mouth as the patient interface 3000 is fitted.

In this example, the support portion 6002 is constructed from silicone. The flexible nature of the support portion 6002 allows it to bend to different mouth shapes. Alternatively, the support portion 6002 may be made from a more rigid material to increase stiffness, and the support portion 6002 may be shaped so as to fit particular mouth shapes. The support portion 6002 may also provide a mounting point for the attachment to the patient interface 3000 and to the strap 6003 via connectors 6004.

In this example, the support portion 6002 may also have an air flow hole 6014 that remains open during use. The air flow hole 6014 may be covered by a layer of the porous structure 6015, which may be foam, textile, or a composite of foam and textile. The porous structure 6015 may be exposed to ambient through the air flow hole 6014. The porous structure 6015 may restrict air flow into and out of the device during use, which may reduce the build-up of moisture and heat within the mouth leak controlling device 6000. The impedance to flow created by the porous structure 6015 may be high enough to limit pressurized air flow to ambient when the patient's mouth is open during therapy, but low enough to allow the patient to breathe from ambient in the absence of such flow and to allow warmed, moistened flow to escape to ambient continuously during therapy to prevent discomfort for the patient. In further examples, the porous structure 6015 may be supplemented with the valve 6005 described above to ensure that air can enter from ambient and escape to ambient as desired.

The flexible portion 6001 may also be formed from the same or similar material as the porous structure 6015, e.g., foam, textile, or a composite of foam and textile. The flexible portion 6001 may air-permeable to create a cooling and wicking effect, or the flexible portion 6001 may be air-impermeable to seal against the patient's face. The flexible portion may have a thickness of approximately 5 mm to approximately 9mm, or greater, depending on the amount of compression desired in the flexible portion 6001 when it is held against the patient's face. Alternatively, the flexible portion 6001 may be made entirely of silicone or TPE so that it can be moulded as one piece with the support portion 6002. The flexible portion 6001 may be formed from a flat sheet of material or it may be pre-formed into a desired geometry to accommodate facial contours.

The oral hole 6009 in the flexible portion 6001 may allow for space for the patient's lips. The flexible portion 6001 may also have a thickness such that when the mouth leak controlling device 6000 is worn by the patient, the porous structure 6015 is offset from the contacting surface 6011 in an anterior direction relative to the patient. This may allow a surface of the porous structure 6015 to minimize or avoid contact with the patient's face, particularly the lips, which may be uncomfortable. The flexible portion 6001 may be made by adding a layer of the same or similar material to the porous structure 6015 on top of the porous structure 6015 so that the additional layer of the flexible portion 6001 forms the offset.

In a further example, there may be one or a plurality of air flow holes 6014 formed in the support portion 6002 that allow flow to escape to atmosphere therethrough when pressure within the mouth leak controlling device 6000 exceeds ambient pressure. In that example, the porous structure 6015 may be omitted and the size and quantity of air flow holes 6014 regulate flow out of the mouth leak controlling device 6000.

In a still further example, there may be no air flow hole 6014 such that when the mouth leak controlling device 6000 is worn by the patient over the mouth the mouth leak controlling device 6000 prevents all flow escaping from the patient's mouth (except in some circumstances where there may be leak through the interface between the mouth leak controlling device 6000 and the patient's face) to atmosphere.

### 5.4 RPT DEVICE

An RPT device 4000 in accordance with one aspect of the present technology comprises mechanical, pneumatic, and/or electrical components and is configured to execute one or more algorithms, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 4 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Air filter(s)

An RPT device in accordance with one form of the present technology may include an air filter 4110, or a plurality of air filters 4110.

In one form, an inlet air filter 4112 is located at the beginning of the pneumatic path upstream of a pressure generator 4140.

In one form, an outlet air filter 4114, for example an antibacterial filter, is located between an outlet of the pneumatic block 4020 and a patient interface 3000.

### 5.4.1.2 Muffler(s)

An RPT device in accordance with one form of the present technology may include a muffler 4120, or a plurality of mufflers 4120.

In one form of the present technology, an inlet muffler 4122 is located in the pneumatic path upstream of a pressure generator 4140.

In one form of the present technology, an outlet muffler 4124 is located in the pneumatic path between the pressure generator 4140 and a patient interface 3000.

### 5.4.1.3 Pressure generator

In one form of the present technology, a pressure generator 4140 for producing a flow, or a supply, of air at positive pressure is a controllable blower 4142. For example, the blower 4142 may include a brushless DC motor 4144 with one or more impellers. The impellers may be located in a volute. The blower may be capable of delivering a supply of air, for example at a rate of up to about 120 litres/minute, at a positive pressure in a range from about 4 cmH2O to about 20 cmH2O, or in other forms up to about 30 cmH2O when delivering respiratory pressure therapy. The blower may be as described in any one of the following patents or patent applications:U.S.Patent No. 7,866,944; U.S. Patent No. 8,638,014; U.S. Patent No. 8,636,479; and PCT Patent Application Publication No. WO 2013/020167.

The pressure generator 4140 may be under the control of the therapy device controller 4240.

In other forms, a pressure generator 4140 may be a piston-driven pump, a pressure regulator connected to a high pressure source (e.g. compressed air reservoir), or a bellows.

### 5.4.1.4 Transducer(s)

Transducers may be internal of the RPT device, or external of the RPT device. External transducers may be located for example on or form part of the air circuit, e.g., the patient interface. External transducers may be in the form of noncontact sensors such as a Doppler radar movement sensor that transmit or transfer data to the RPT device.

In one form of the present technology, one or more transducers 4270 are located upstream and/or downstream of the pressure generator 4140. The one or more transducers 4270 may be constructed and arranged to generate signals representing properties of the flow of air such as a flow rate, a pressure or a temperature at that point in the pneumatic path.

In one form of the present technology, one or more transducers 4270 may be located proximate to the patient interface 3000.

In one form, a signal from a transducer 4270 may be filtered, such as by low-pass, high-pass or band-pass filtering.

### 5.4.1.5 Anti-spill back valve

In one form of the present technology, an anti-spill back valve 4160 is located between the humidifier 5000 and the pneumatic block 4020. The anti-spill back valve is constructed and arranged to reduce the risk that water will flow upstream from the humidifier 5000, for example to the motor 4144.

### 5.4.2 RPT device electrical components

### 5.4.2.1 Power supply

A power supply 4210 may be located internal or external of the external housing 4010 of the RPT device 4000.

In one form of the present technology, power supply 4210 provides electrical power to the RPT device 4000 only. In another form of the present technology, power supply 4210 provides electrical power to both RPT device 4000 and humidifier 5000.

### 5.4.2.2 Input devices

In one form of the present technology, an RPT device 4000 includes one or more input devices 4220 in the form of buttons, switches or dials to allow a person to interact with the device. The buttons, switches or dials may be physical devices, or software devices accessible via a touch screen. The buttons, switches or dials may, in one form, be physically connected to the external housing 4010, or may, in another form, be in wireless communication with a receiver that is in electrical connection to the central controller.

In one form, the input device 4220 may be constructed and arranged to allow a person to select a value and/or a menu option.

### 5.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349, which is incorporated herewithin in its entirety by reference.

### 5.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000.

### 5.6 HUMIDIFIER

### 5.6.1 Humidifier overview

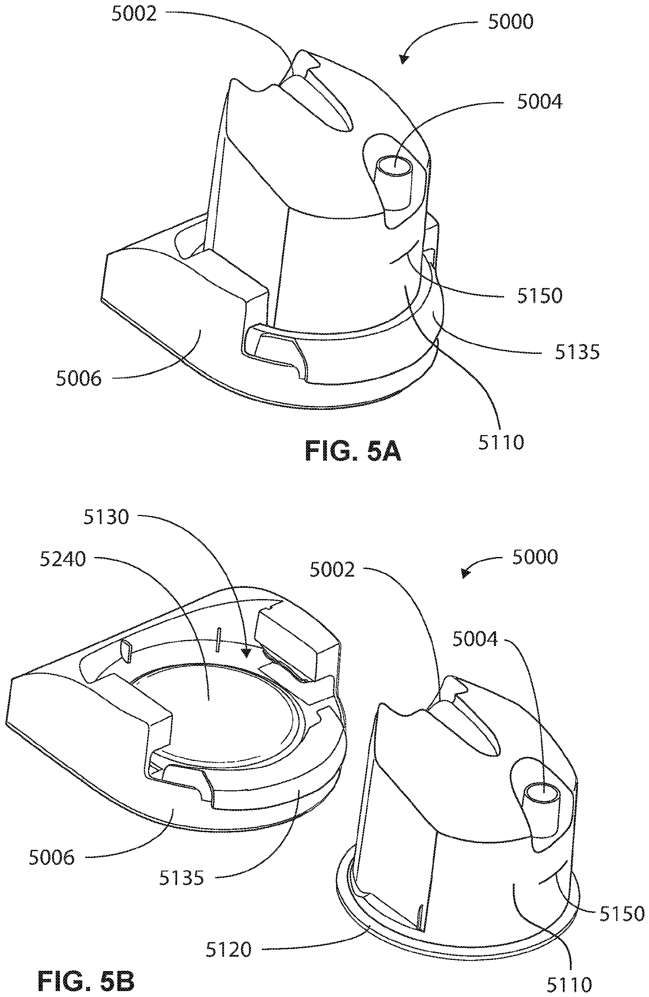

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

### 5.7 BREATHING WAVEFORMS

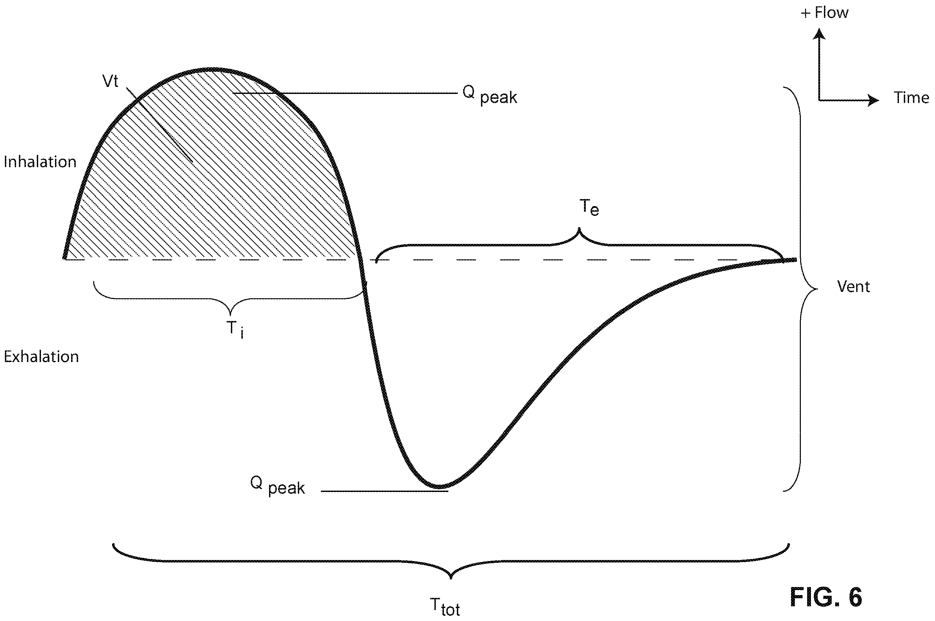

Fig. 6A shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak -0.5* L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 5.8 GLOSSARY

For the purposes of the present technology disclosure, in certain forms of the present technology, one or more of the following definitions may apply. In other forms of the present technology, alternative definitions may apply.

### 5.8.1 General

*Air:* In certain forms of the present technology, air may be taken to mean atmospheric air, and in other forms of the present technology air may be taken to mean some other combination of breathable gases, e.g. oxygen enriched air.

*Ambient:* In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air:* Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or greater.

*Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 5.8.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 5.8.1.2 Mechanical properties

Resilience: Ability of a material to absorb energy when deformed elastically and to release the energy upon unloading.

Resilient: Will release substantially all of the energy when unloaded. Includes e.g. certain silicones, and thermoplastic elastomers.

Hardness: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

Stiffness (or rigidity) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

Floppy structure or component: A structure or component that will change shape, e.g. bend, when caused to support its own weight, within a relatively short period of time such as 1 second.

Rigid structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH₂O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 5.8.2 Respiratory cycle

Apnea: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

Breathing rate: The rate of spontaneous respiration of a patient, usually measured in breaths per minute.

Duty cycle: The ratio of inhalation time, Ti to total breath time, Ttot.

Effort (breathing): The work done by a spontaneously breathing person attempting to breathe.

Expiratory portion of a breathing cycle: The period from the start of expiratory flow to the start of inspiratory flow.

Flow limitation: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

Hypopnea: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

Hyperpnea: An increase in flow to a level higher than normal.

Inspiratory portion of a breathing cycle: The period from the start of inspiratory flow to the start of expiratory flow will be taken to be the inspiratory portion of a breathing cycle.

Patency (airway): The degree of the airway being open, or the extent to which the airway is open. A patent airway is open. Airway patency may be quantified, for example with a value of one (1) being patent, and a value of zero (0), being closed (obstructed).

Positive End-Expiratory Pressure (PEEP): The pressure above atmosphere in the lungs that exists at the end of expiration.

Peak flow rate (Qpeak): The maximum value of flow rate during the inspiratory portion of the respiratory flow waveform.

Respiratory flow rate, patient airflow rate, respiratory airflow rate (Qr): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

Tidal volume (Vt): The volume of air inhaled or exhaled during normal breathing, when extra effort is not applied. In principle the inspiratory volume Vi (the volume of air inhaled) is equal to the expiratory volume Ve (the volume of air exhaled), and therefore a single tidal volume Vt may be defined as equal to either quantity. In practice the tidal volume Vt is estimated as some combination, e.g. the mean, of the inspiratory volume Vi and the expiratory volume Ve.

Inhalation Time (Ti): The duration of the inspiratory portion of the respiratory flow rate waveform.

Exhalation Time (Te): The duration of the expiratory portion of the respiratory flow rate waveform.

Total Time (Ttot): The total duration between the start of one inspiratory portion of a respiratory flow rate waveform and the start of the following inspiratory portion of the respiratory flow rate waveform.

Typical recent ventilation: The value of ventilation around which recent values of ventilation Vent over some predetermined timescale tend to cluster, that is, a measure of the central tendency of the recent values of ventilation.

Upper airway obstruction (UAO): includes both partial and total upper airway obstruction. This may be associated with a state of flow limitation, in which the flow rate increases only slightly or may even decrease as the pressure difference across the upper airway increases (Starling resistor behaviour).

Ventilation (Vent): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 5.8.3 Ventilation

Adaptive Servo-Ventilator (ASV): A servo-ventilator that has a changeable, rather than fixed target ventilation. The changeable target ventilation may be learned from some characteristic of the patient, for example, a respiratory characteristic of the patient.

Backup rate: A parameter of a ventilator that establishes the minimum breathing rate (typically in number of breaths per minute) that the ventilator will deliver to the patient, if not triggered by spontaneous respiratory effort.

Cycled: The termination of a ventilator's inspiratory phase. When a ventilator delivers a breath to a spontaneously breathing patient, at the end of the inspiratory portion of the breathing cycle, the ventilator is said to be cycled to stop delivering the breath.

Expiratory positive airway pressure (EPAP): a base pressure, to which a pressure varying within the breath is added to produce the desired interface pressure which the ventilator will attempt to achieve at a given time.

End expiratory pressure (EEP): Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

Inspiratory positive airway pressure (IPAP): Maximum desired interface pressure which the ventilator will attempt to achieve during the inspiratory portion of the breath.

Pressure support: A number that is indicative of the increase in pressure during ventilator inspiration over that during ventilator expiration, and generally means the difference in pressure between the maximum value during inspiration and the base pressure (e.g., PS = IPAP - EPAP). In some contexts, pressure support means the difference which the ventilator aims to achieve, rather than what it actually achieves.

Servo-ventilator: A ventilator that measures patient ventilation, has a target ventilation, and which adjusts the level of pressure support to bring the patient ventilation towards the target ventilation.

Spontaneous/Timed (S/T): A mode of a ventilator or other device that attempts to detect the initiation of a breath of a spontaneously breathing patient. If however, the device is unable to detect a breath within a predetermined period of time, the device will automatically initiate delivery of the breath.

Swing: Equivalent term to pressure support.

Triggered: When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 5.8.4 Anatomy

### 5.8.4.1 Anatomy of the face

Ala: the external outer wall or "wing" of each nostril (plural: alar)

### Alar angle:

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 5.8.4.2 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 5.8.5 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

### Functional dead space: (description to be inserted here)

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. **In** some forms, a shell may be faceted. **In** some forms a shell may be airtight. **In** some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. **In** one form, the swivel may be constructed to rotate through an angle of at least 360 degrees. **In** another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 5.8.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

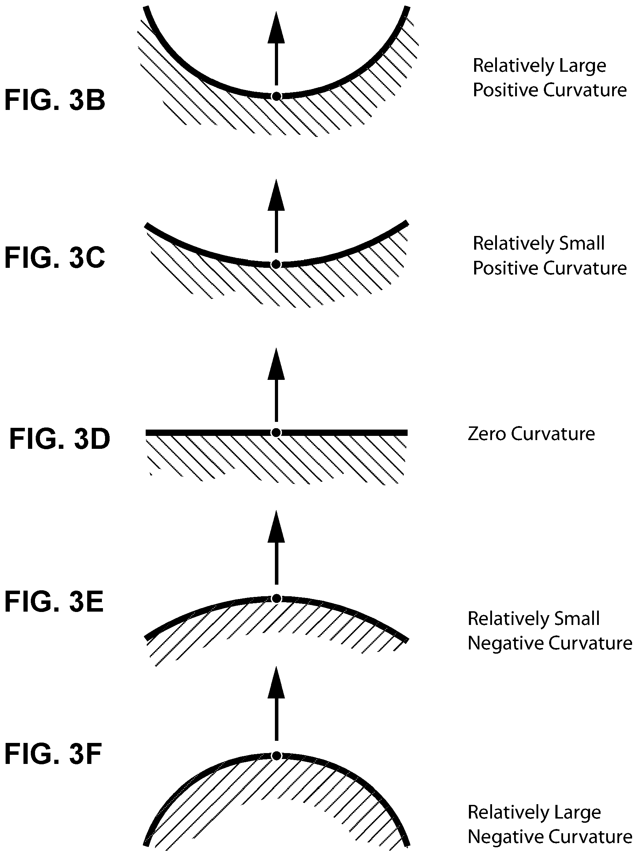

To facilitate describing the shape of the three-dimensional structures and the surfaces, we first consider a cross-section through a surface of the structure at a point, p. See Fig. 3B to Fig. 3F, which illustrate examples of cross-sections at point p on a surface, and the resulting plane curves. Figs. 3B to 3F also illustrate an outward normal vector at p. The outward normal vector at p points away from the surface. In some examples we describe the surface from the point of view of an imaginary small person standing upright on the surface.

### 5.8.6.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 5.8.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 5.8.6.3 Space curves

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 30).

Osculating plane: The plane containing the unit tangent vector and the unit principal normal vector. See Figures 30 and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

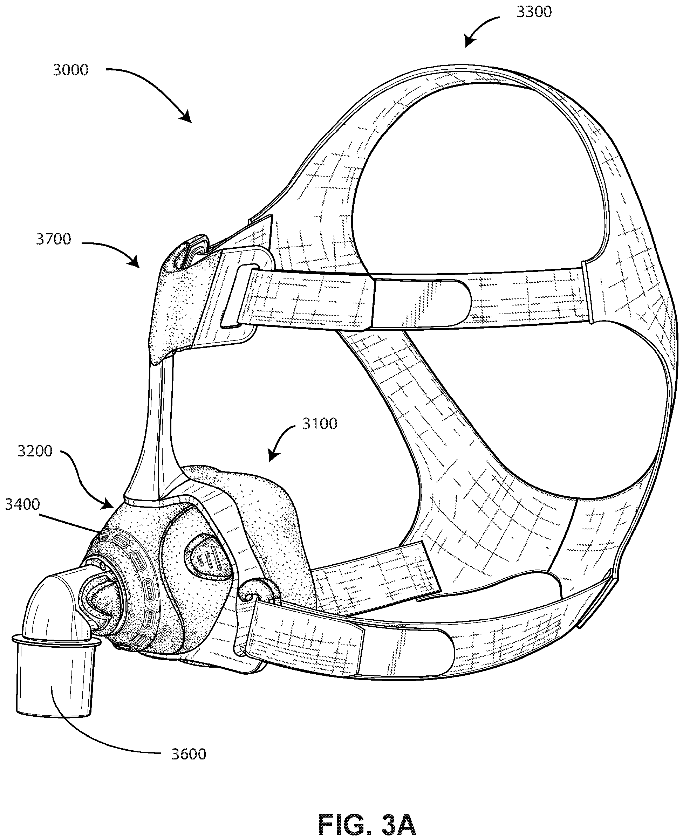

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 30), a space curve turning towards the direction of the left-hand binormal may be considered as having a left-hand positive torsion (e.g. a left-hand helix). Hence left-hand positive is equivalent to right-hand negative. See Fig. 3T.

### 5.8.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 5.9 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology.

### 5.10 REFERENCE SIGNS LIST

| | |
|---|---|
| patient | 1000 |
| bed partner | 1100 |
| patient interface | 3000 |
| seal - forming structure | 3100 |
| plenum chamber | 3200 |
| plenum chamber lateral end | 3202 |
| plenum chamber connector | 3204 |
| chord | 3210 |
| superior point | 3220 |
| inferior point | 3230 |
| positioning and stabilising structure | 3300 |
| lateral portion | 3302 |
| superior portion | 3304 |
| hub | 3306 |
| end | 3314 |
| vent | 3400 |
| hole | 3404 |
| decoupling structure | 3500 |
| connection port | 3600 |
| forehead support | 3700 |
| anti - asphyxia valve | 3900 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| outlet air filter | 4114 |
| muffler | 4120 |
| inlet muffler | 4122 |
| outlet muffler | 4124 |
| pressure generator | 4140 |
| blower | 4142 |
| motor | 4144 |
| anti - spill back valve | 4160 |
| air circuit | 4170 |
| electrical components | 4200 |
| Printed Circuit Board Assembly | 4202 |
| power supply | 4210 |
| input device | 4220 |
| central controller | 4230 |
| therapy device controller | 4240 |
| transducer | 4270 |
| humidifier | 5000 |
| humidifier inlet | 5002 |
| humidifier outlet | 5004 |
| humidifier base | 5006 |
| humidifier reservoir | 5110 |
| humidifier reservoir dock | 5130 |
| heating element | 5240 |
| mouth leak controlling device | 6000 |
| flexible portion | 6001 |
| support portion | 6002 |
| strap | 6003 |
| connector | 6004 |
| valve | 6005 |
| valve hole | 6006 |
| valve flap | 6007 |
| valve frame | 6008 |
| oral hole | 6009 |
| contacting surface | 6011 |
| magnetic clips | 6012 |
| patient interface connector | 6013 |
| air flow hole | 6014 |
| porous structure | 6015 |
| tab hole | 6016 |
| flap tab | 6017 |
| flexible joint | 6018 |
| assembly tab | 6020 |
| receiver | 6021 |
| valve surface | 6022 |
| flap surface | 6023 |
| patient interface | 7000 |
| seal - forming structure | 7100 |
| nasal seal - forming structure | 7102 |
| oral seal - forming structure | 7104 |
| naris opening | 7106 |
| bridge portion | 7108 |
| nasal cavity | 7110 |
| inner sealing portion | 7112 |
| outer sealing portion | 7116 |
| lateral side | 7120 |
| upper nasal sealing region | 7124 |
| nasal sealing region | 7128 |
| oral opening | 7132 |
| oral cavity | 7136 |
| lateral side region | 7140 |
| upper oral sealing region | 7144 |
| oral sealing region | 7148 |
| connecting region | 7152 |
| connecting region | 7154 |
| passage | 7156 |
| membrane | 7160 |
| holes | 7162 |
| plenum chamber | 7200 |
| nasal plenum chamber | 7202 |
| oral plenum chamber | 7204 |
| connecting region | 7208 |
| plenum chamber inlet port | 7212 |
| vent opening | 7216 |
| vent receiving region | 7220 |
| clip | 7222 |
| AAV receiving region | 7224 |
| connector | 7228 |
| first face | 7232 |
| second face | 7236 |
| angle | 7240 |
| insert | 7244 |
| projection | 7252 |
| positioning and stabilizing structure | 7300 |
| conduit | 7302 |
| concertina section | 7304 |
| strap | 7308 |
| connector | 7312 |
| connection port | 7600 |
| patient interface | 8000 |
| seal - forming structure | 8100 |
| plenum chamber | 8200 |
| nasal plenum chamber | 8202 |
| oral plenum chamber | 8204 |
| connecting region | 8208 |
| plenum chamber inlet port | 8212 |
| connector | 8214 |
| AAV receiving region | 8224 |
| connectors | 8228 |
| positioning and stabilizing structure | 8300 |
| upper strap | 8304 |
| lower strap | 8308 |
| connector | 8312 |

## Claims

1. A patient interface (7000) for sealed delivery of a flow of air at a continuously positive pressure with respect to ambient air pressure to an entrance to a patient's airways including at least an entrance of a patient's nares, wherein the patient interface (7000) is configured to maintain a therapy pressure in a range of about 4 cmH₂O to about 30 cmH₂O above ambient air pressure in use, throughout a patient's respiratory cycle, while the patient is sleeping, to ameliorate sleep disordered breathing; said patient interface (7000) comprising:
a cushion comprising:
a nasal portion comprising:
a nasal plenum chamber (7202) at least partially forming a nasal cavity (7110) pressurisable to a therapeutic pressure of at least 4 cmH₂O above ambient air pressure, the nasal plenum chamber (7202) including at least one plenum chamber inlet port (7212) configured to convey the flow of air into the nasal plenum chamber (7202); and
a nasal seal-forming structure (7102) constructed and arranged to form a seal with a region of the patient's face surrounding an entrance to the patient's nares, said nasal seal-forming structure (7102) having a hole (7106) therein such that the flow of air at said therapeutic pressure is delivered to at least an entrance to the patient's nares, the nasal seal-forming structure (7102) constructed and arranged to maintain said therapeutic pressure in the nasal plenum chamber (7202) throughout the patient's respiratory cycle in use; and
an oral portion comprising:
an oral plenum chamber (7204) at least partially forming an oral cavity (7136) pressurisable above ambient air pressure; and
an oral seal-forming structure (7104) constructed and arranged to form a seal with a region of the patient's face surrounding an entrance to the patient's mouth, said oral seal-forming structure (7104) having a hole (7132) therein such that air in the oral cavity (7136) is delivered to at least an entrance to the patient's mouth, the oral seal-forming structure (7104) constructed and arranged to maintain said therapeutic pressure in the oral plenum chamber (7204) throughout the patient's respiratory cycle in use;
a positioning and stabilising structure (7300) to provide a force to hold the nasal seal-forming structure (7102) and the oral seal-forming structure (7104) in a therapeutically effective position on the patient's head; and
a vent structure (3400) connected to the nasal plenum chamber (7202) and configured to allow a continuous flow of gases exhaled by the patient from an interior of the nasal plenum chamber (7202) to ambient, said vent structure (3400) being sized and shaped to maintain the therapeutic pressure in the nasal plenum chamber (7202) in use;
wherein the patient interface (7000) is configured to allow the patient to breath from ambient through their mouth in the absence of a flow of pressurised air through the at least one plenum chamber inlet port (7212);
wherein the nasal portion is permanently connected to the oral portion with a decoupling portion formed between the nasal plenum chamber (7202) and the oral plenum chamber (7204), the decoupling portion including an angled portion extending toward the nasal seal-forming structure (7102) and the oral seal-forming structure (7104), the decoupling portion configured to allow the nasal portion to pivot relative to the oral portion;
wherein a curved surface opposite to the decoupling portion connects the nasal seal-forming structure (7102) and the oral seal-forming structure (7104), wherein the curved surface forms a continuous surface between the nasal seal-forming structure (7102) and the oral seal-forming structure (7104), and wherein the curved surface is configured stretch toward a planar orientation while worn by the patient;
wherein the at least one plenum chamber inlet port (7212) includes a pair of plenum chamber inlet ports, each positioned at a lateral side of the nasal plenum chamber (7202); and
wherein conduits (7302) connect to the pair of plenum chamber inlet ports, the conduits (7302) configured to convey the flow of air through each plenum chamber inlet port (7212) of the pair of plenum chamber inlet ports.

2. The patient interface (7000) of claim 1, wherein the nasal seal-forming structure (7102) is configured to contact the patient's columella and is not configured to contact the patient's nasal ridge superior to the patient's pronasale.

3. The patient interface (7000) of claim 1, wherein the conduits (7302) form a portion of the positioning and stabilizing structure (7300) and are configured to provide a tensile force to the nasal portion in use.

4. The patient interface (7000) of any one of claims 1 to 3, wherein the conduits (7302) include concertina sections configured to expand upon application of a tensile force.

5. The patient interface (7000) of any one of claims 1 to 4, wherein the oral portion includes a pair of connectors (7228) spaced apart from one another, and wherein the pair of connectors (7228) are magnets.

6. The patient interface (7000) of claim 5, wherein the oral portion is molded around the pair of connectors (7228).

7. The patient interface (7000) of claim 5, further comprising an insert (7244) including the pair of connectors (7228), the insert (7244) being selectively coupled to the oral plenum chamber (7204) with a press fit, friction fit, and/or snap fit,
wherein the pair of connectors are selectively removable from the oral portion, and
wherein the insert (7244) includes an anti-asphyxia valve (3900).

8. The patient interface (7000) of any one of claims 5 to 7, wherein the positioning and stabilizing structure (7300) includes lower straps selectively connected to the oral portion via the connectors.

9. The patient interface (7000) of any one of claims 1 to 8, wherein the decoupling portion is configured to at least partially decouple a force applied by the positioning and stabilizing structure (7300) to the oral portion from the nasal portion.

10. The patient interface (7000) of any one of claims 1 to 9, wherein an angle (7240) of the decoupling portion between the oral portion and the nasal portion in a relaxed position is about 1° to about 90°, preferably about 10° to about 50°.

11. The patient interface (7000) of any one of claims 1 to 10, further comprising a passage (7156) formed between the nasal cavity (7110) and the oral cavity (7136), the passage (7156) configured to provide fluid communication between the nasal cavity (7110) and the oral cavity (7136),
wherein the passage (7156) is a substantially elliptical shape, and
wherein the passage (7156) is substantially aligned with the decoupling portion and the curved surface.

12. The patient interface (7000) of any one of claims 1 to 10, further comprising a membrane (7160) formed between the nasal cavity (7110) and the oral cavity (7136),
wherein the membrane (7160) extends between the decoupling portion and the curved surface,
wherein the membrane (7160) is slack before the patient interface (7000) contacts the patient, and
wherein the membrane (7160) is substantially solid and is configured to prevent airflow from traveling within the cushion between the nasal cavity (7110) and the oral cavity (7136).

13. The patient interface (7000) of any one of claims 1 to 10, further comprising a membrane (7160) formed between the nasal cavity (7110) and the oral cavity (7136),
wherein the membrane (7160) extends between the decoupling portion and the curved surface,
wherein the membrane (7160) is slack before the patient interface (7000) contacts the patient,
wherein the membrane (7160) is configured to allow airflow from traveling within the cushion between the nasal cavity (7110) and the oral cavity (7136) through at least one hole (7162),
wherein the at least one hole (7162) is a plurality of spaced apart holes, and wherein the plurality of spaced apart holes are sized to allow a predetermined flow of air between the nasal cavity (7110) and the oral cavity (7136), and/or
where the membrane (7160) includes a porosity on a microscopic and/or molecular level that is configured to allow a predetermined flow of air traveling within the cushion to flow between the nasal cavity (7110) and the oral cavity (7136).

14. The patient interface (7000) of any one of claims 1 to 12, wherein the curved surface is configured to lie flat against a patient's face in use to form a single seal along the patient's lip superior.

## Patentansprüche

1. Patientenschnittstelle (7000) zur abgedichteten Zufuhr eines Luftstroms mit einem kontinuierlich positiven Druck in Bezug auf den Umgebungsluftdruck an einen Eingang der Atemwege eines Patienten, der zumindest einen Eingang der Nasenlöcher eines Patienten umfasst, wobei die Patientenschnittstelle (7000) derart konfiguriert ist, dass sie einen therapeutischen Druck in einem Bereich von etwa 4 cmH₂O bis etwa 30 cmH₂O über dem Umgebungsluftdruck bei der Verwendung während des gesamten Atemzyklus eines Patienten aufrechterhält, während der Patient schläft, um eine schlafgestörte Atmung zu verbessern; wobei die Patientenschnittstelle (7000) aufweist:
ein Polster, welches aufweist:
einen nasalen Abschnitt, welcher aufweist:
eine nasale Plenumkammer (7202), die zumindest teilweise einen nasalen Hohlraum (7110) bildet, der auf einen therapeutischen Druck von zumindest 4 cmH₂O über dem Umgebungsluftdruck beaufschlagbar ist, wobei die nasale Plenumkammer (7202) zumindest eine Plenumkammer-Einlassöffnung (7212) aufweist, die derart konfiguriert ist, dass sie den Luftstrom in die nasale Plenumkammer (7202) leitet; und
eine nasale dichtungsbildende Struktur (7102), die derart konstruiert und angeordnet ist, dass sie eine Abdichtung mit einem Bereich des Gesichts des Patienten bildet, der einen Eingang der Nasenlöcher des Patienten umgibt, wobei die nasale dichtungsbildende Struktur (7102) ein Loch (7106) darin aufweist, so dass der Luftstrom mit dem therapeutischen Druck zumindest an einen Eingang der Nasenlöcher des Patienten zugeführt wird, wobei die nasale dichtungsbildende Struktur (7102) derart konstruiert und angeordnet ist, dass sie den therapeutischen Druck in der nasalen Plenumkammer (7202) während des gesamten Atemzyklus des Patienten bei der Verwendung aufrechterhält; und
einen oralen Abschnitt, welcher aufweist:
eine orale Plenumkammer (7204), die zumindest teilweise einen oralen Hohlraum (7136) bildet, der über den Umgebungsluftdruck beaufschlagbar ist; und
eine orale dichtungsbildende Struktur (7104), die derart konstruiert und angeordnet ist, dass sie eine Dichtung mit einem Bereich des Gesichts des Patienten bildet, der einen Eingang des Munds des Patienten umgibt, wobei die orale dichtungsbildende Struktur (7104) ein Loch (7132) darin aufweist, so dass Luft in dem oralen Hohlraum (7136) an zumindest einen Eingang des Munds des Patienten zugeführt wird, wobei die orale dichtungsbildende Struktur (7104) derart konstruiert und angeordnet ist, dass sie den therapeutischen Druck in der oralen Plenumkammer (7204) während des gesamten Atemzyklus des Patienten bei der Verwendung aufrechterhält;
eine Positionierungs- und Stabilisierungsstruktur (7300), um eine Kraft bereitzustellen, um die nasale dichtungsbildende Struktur (7102) und die orale dichtungsbildende Struktur (7104) in einer therapeutisch wirksamen Position am Kopf des Patienten zu halten; und
eine Entlüftungsstruktur (3400), die mit der nasalen Plenumkammer (7202) verbunden und derart konfiguriert ist, dass sie einen kontinuierlichen Strom von durch den Patienten ausgeatmeten Gasen aus dem Inneren der nasalen Plenumkammer (7202) in die Umgebung ermöglicht, wobei die Entlüftungsstruktur (3400) derart bemessen und geformt ist, dass sie den therapeutischen Druck in der nasalen Plenumkammer (7202) bei der Verwendung aufrechterhält;
wobei die Patientenschnittstelle (7000) derart konfiguriert ist, dass sie es dem Patienten ermöglicht, durch den Mund aus der Umgebung zu atmen, wenn kein Strom von Druckluft durch die zumindest eine Plenumkammer-Einlassöffnung (7212) fließt;
wobei der nasale Abschnitt mittels eines Entkopplungsabschnitts permanent mit dem oralen Abschnitt verbunden ist, der zwischen der nasalen Plenumkammer (7202) und der oralen Plenumkammer (7204) ausgebildet ist, wobei der Entkopplungsabschnitt einen abgewinkelten Abschnitt aufweist, der sich in Richtung der nasalen dichtungsbildenden Struktur (7102) und der oralen dichtungsbildenden Struktur (7104) erstreckt, wobei der Entkopplungsabschnitt derart konfiguriert ist, dass er es dem nasalen Abschnitt ermöglicht, relativ zu dem oralen Abschnitt zu schwenken;
wobei eine gekrümmte Oberfläche, die dem Entkopplungsabschnitt gegenüberliegt, die nasale dichtungsbildende Struktur (7102) und die orale dichtungsbildende Struktur (7104) verbindet, wobei die gekrümmte Oberfläche eine kontinuierliche Oberfläche zwischen der nasalen dichtungsbildenden Struktur (7102) und der oralen dichtungsbildenden Struktur (7104) bildet, und wobei die gekrümmte Oberfläche derart konfiguriert ist, dass sie sich beim Tragen durch den Patienten in Richtung einer planaren Ausrichtung dehnt;
wobei die zumindest eine Plenumkammer-Einlassöffnung (7212) ein Paar Plenumkammer-Einlassöffnungen aufweist, die jeweils an einer lateralen Seite der nasalen Plenumkammer (7202) angeordnet sind; und
wobei Leitungen (7302) mit dem Paar Plenumkammer-Einlassöffnungen verbunden sind, wobei die Leitungen (7302) derart konfiguriert sind, dass sie den Luftstrom durch jede Plenumkammer-Einlassöffnung (7212) des Paars Plenumkammer-Einlassöffnungen befördern.

2. Patientenschnittstelle (7000) nach Anspruch 1, wobei die nasale dichtungsbildende Struktur (7102) derart konfiguriert ist, dass sie die Columella des Patienten berührt und nicht derart konfiguriert ist, dass sie den Nasenkamm des Patienten oberhalb der Pronasale des Patienten berührt.

3. Patientenschnittstelle (7000) nach Anspruch 1, wobei die Leitungen (7302) einen Teil der Positionierungs- und Stabilisierungsstruktur (7300) bilden und derart konfiguriert sind, dass sie bei der Verwendung auf den nasalen Abschnitt eine Zugkraft ausüben.

4. Patientenschnittstelle (7000) nach einem der Ansprüche 1 bis 3, wobei die Leitungen (7302) Faltenbalgabschnitte aufweisen, die derart konfiguriert sind, dass sie sich bei Aufbringung einer Zugkraft ausdehnen.

5. Patientenschnittstelle (7000) nach einem der Ansprüche 1 bis 4, wobei der orale Abschnitt ein Paar Verbinder (7228) aufweist, die voneinander beabstandet sind, und
wobei das Paar Verbinder (7228) Magnete sind.

6. Patientenschnittstelle (7000) nach Anspruch 5, wobei der orale Abschnitt um das Paar Verbinder (7228) herum geformt ist.

7. Patientenschnittstelle (7000) nach Anspruch 5, ferner aufweisend einen Einsatz (7244), der das Paar Verbinder (7228) aufweist, wobei der Einsatz (7244) selektiv mit oralen Plenumkammer (7204) mit einer Presspassung, Reibungspassung und/oder Schnapppassung gekoppelt ist,
wobei das Paar Verbinder selektiv von dem oralen Abschnitt abnehmbar ist, und
wobei der Einsatz (7244) ein Anti-Asphyxie-Ventil (3900) aufweist.

8. Patientenschnittstelle (7000) nach einem der Ansprüche 5 bis 7, wobei die Positionierungs- und Stabilisierungsstruktur (7300) untere Riemen aufweist, die selektiv mit dem oralen Abschnitt über die Verbinder verbunden sind.

9. Patientenschnittstelle (7000) nach einem der Ansprüche 1 bis 8, wobei der Entkopplungsabschnitt derart konfiguriert ist, dass er eine von der Positionierungs- und Stabilisierungsstruktur (7300) auf den oralen Abschnitt ausgeübte Kraft zumindest teilweise von dem nasalen Abschnitt entkoppelt.

10. Patientenschnittstelle (7000) nach einem der Ansprüche 1 bis 9, wobei ein Winkel (7240) des Entkopplungsabschnitts zwischen dem oralen Abschnitt und dem nasalen Abschnitt in einer entspannten Position etwa 1° bis etwa 90°, bevorzugt etwa 10° bis etwa 50° beträgt.

11. Patientenschnittstelle (7000) nach einem der Ansprüche 1 bis 10, ferner aufweisend einen Durchgang (7156), der zwischen dem nasalen Hohlraum (7110) und dem oralen Hohlraum (7136) ausgebildet ist, wobei der Durchgang (7156) derart konfiguriert ist, dass er eine Fluidverbindung zwischen dem nasalen Hohlraum (7110) und dem oralen Hohlraum (7136) herstellt,
wobei der Durchgang (7156) eine im Wesentlichen elliptische Form hat, und
wobei der Durchgang (7156) im Wesentlichen mit dem Entkopplungsabschnitt und der gekrümmten Oberfläche ausgerichtet ist.

12. Patientenschnittstelle (7000) nach einem der Ansprüche 1 bis 10, ferner aufweisend eine Membran (7160), die zwischen dem nasalen Hohlraum (7110) und dem oralen Hohlraum (7136) ausgebildet ist,
wobei sich die Membran (7160) zwischen dem Entkopplungsabschnitt und der gekrümmten Oberfläche erstreckt,
wobei die Membran (7160) schlaff ist, bevor die Patientenschnittstelle (7000) den Patienten berührt, und
wobei die Membran (7160) im Wesentlichen fest ist und derart konfiguriert ist, dass sie verhindert, dass sich der Luftstrom innerhalb des Polsters zwischen dem nasalen Hohlraum (7110) und dem oralen Hohlraum (7136) bewegt.

13. Patientenschnittstelle (7000) nach einem der Ansprüche 1 bis 10, ferner aufweisend eine Membran (7160), die zwischen dem nasalen Hohlraum (7110) und dem oralen Hohlraum (7136) ausgebildet ist,
wobei sich die Membran (7160) zwischen dem Entkopplungsabschnitt und der gekrümmten Oberfläche erstreckt,
wobei die Membran (7160) schlaff ist, bevor die Patientenschnittstelle (7000) den Patienten berührt,
wobei die Membran (7160) derart konfiguriert ist, dass sie einen Luftstrom aus innerhalb des Polsters zwischen dem nasalen Hohlraum (7110) und dem oralen Hohlraum (7136) durch zumindest ein Loch (7162) zulässt,
wobei das zumindest eine Loch (7162) eine Vielzahl von beabstandeten Löchern ist, und wobei die Vielzahl von beabstandeten Löchern derart bemessen ist, dass sie einen vorbestimmten Luftstrom zwischen dem nasalen Hohlraum (7110) und dem oralen Hohlraum (7136) ermöglicht, und/oder
wobei die Membran (7160) eine Porosität auf mikroskopischer und/oder molekularer Ebene aufweist, die derart konfiguriert ist, dass ein vorbestimmter Luftstrom innerhalb des Polsters zwischen dem nasalen Hohlraum (7110) und dem oralen Hohlraum (7136) fließen kann.

14. Patientenschnittstelle (7000) nach einem der Ansprüche 1 bis 12, wobei die gekrümmte Oberfläche derart konfiguriert ist, dass sie bei der Verwendung flach am Gesicht des Patienten anliegt, um eine einzige Dichtung entlang der oberen Lippe des Patienten zu bilden.

## Revendications

1. Interface patient (7000) destinée à une administration étanche d'un flux d'air à une pression positive continue par rapport à une pression d'air ambiant à une entrée des voies aériennes d'un patient comprenant au moins une entrée des narines du patient, dans laquelle l'interface patient (7000) est configurée pour maintenir une pression thérapeutique comprise dans une plage allant d'environ 4 cmH₂O à environ 30 cmH₂O au-dessus de la pression d'air ambiant en utilisation, tout au long du cycle respiratoire du patient, tandis que le patient dort, de façon à améliorer une respiration de trouble du sommeil ; ladite interface patient (7000) comprenant :
un coussin comprenant :
une partie nasale comprenant :
une chambre de distribution nasale (7202) formant au moins partiellement une cavité nasale (7110) pouvant être mise sous pression à une pression thérapeutique **d'au** moins 4 cmH₂O au-dessus de la pression **d'air** ambiant, la chambre de distribution nasale (7202) comprenant au moins un orifice d'entrée de chambre de distribution (7212) configuré pour transporter le flux d'air dans la chambre de distribution nasale (7202) ; et
une structure de formation **d'étanchéité** nasale (7102) structurée et conçue pour former une étanchéité avec une région du visage du patient entourant une entrée des narines du patient, ladite structure de formation d'étanchéité nasale (7102) comportant un trou (7106) en son sein de sorte que le flux **d'air** à ladite pression thérapeutique soit administré à au moins une entrée des narines du patient, la structure de formation d'étanchéité nasale (7102) étant structurée et conçue pour maintenir ladite pression thérapeutique dans la chambre de distribution nasale (7202) tout au long du cycle respiratoire du patient en utilisation ; et
une partie orale comprenant :
une chambre de distribution orale (7204) formant au moins partiellement une cavité orale (7136) pouvant être mise sous pression à une pression supérieure à la pression d'air ambiant ; et
une structure de formation **d'étanchéité** orale (7104) structurée et conçue pour former une étanchéité avec une région du visage du patient entourant une entrée de la bouche du patient, ladite structure de formation d'étanchéité orale (7104) comportant un trou (7132) en son sein de sorte que de l'air dans la cavité orale (7136) soit administré à au moins une entrée de la bouche du patient, la structure de formation **d'étanchéité** orale (7104) étant structurée et conçue pour maintenir ladite pression thérapeutique dans la chambre de distribution orale (7204) tout au long du cycle respiratoire du patient en utilisation ;
une structure de positionnement et de stabilisation (7300) servant à établir une force pour maintenir la structure de formation **d'étanchéité** nasale (7102) et la structure de formation **d'étanchéité** orale (7104) dans une position thérapeutiquement efficace sur la tête du patient ; et
une structure d'évent (3400) raccordée à la chambre de distribution nasale (7202) et configurée pour permettre un flux continu de gaz exhalés par le patient de l'intérieur de la chambre de distribution nasale (7202) vers l'environnement ambiant, ladite structure d'évent (3400) étant dimensionnée et formée pour maintenir la pression thérapeutique dans la chambre de distribution nasale (7202) en utilisation ;
dans laquelle l'interface patient (7000) est configurée pour permettre au patient de respirer à partir de l'environnement ambiant par la bouche en l'absence d'un flux d'air sous pression à travers l'au moins un orifice d'entrée de chambre de distribution (7212) ;
dans laquelle la partie nasale est reliée en permanence à la partie orale par une partie de désaccouplement formée entre la chambre de distribution nasale (7202) et la chambre de distribution orale (7204), la partie de désaccouplement comprenant une partie inclinée s'étendant vers la structure de formation d'étanchéité nasale (7102) et vers la structure de formation d'étanchéité orale (7104), la partie de désaccouplement étant configurée pour permettre à la partie nasale de pivoter par rapport à la partie orale ;
dans laquelle une surface incurvée opposée à la partie de désaccouplement relie la structure de formation d'étanchéité nasale (7102) et la structure de formation d'étanchéité orale (7104), dans laquelle la surface incurvée forme une surface continue entre la structure de formation d'étanchéité nasale (7102) et la structure de formation d'étanchéité orale (7104), et dans laquelle la surface incurvée est configurée pour s'étendre vers une orientation plane tandis qu'elle est portée par le patient ;
dans laquelle l'au moins un orifice d'entrée de chambre de distribution (7212) comprend une paire d'orifices d'entrée de chambre de distribution, chacun étant positionné au niveau d'un côté latéral de la chambre de distribution nasale (7202) ; et
dans laquelle des conduits (7302) raccordent la paire d'orifices d'entrée de chambre de distribution, les conduits (7302) étant configurés pour transporter le flux d'air à travers chaque orifice d'entrée de chambre de distribution (7212) de la paire d'orifices d'entrée de chambre de distribution.

2. Interface patient (7000) selon la revendication 1, dans laquelle la structure de formation d'étanchéité nasale (7102) est configurée pour contacter la columelle du patient et n'est pas configurée pour contacter le dos du nez du patient supérieur au point pronasal du patient.

3. Interface patient (7000) selon la revendication 1, dans laquelle les conduits (7302) forment une partie de la structure de positionnement et de stabilisation (7300) et sont configurés pour établir une force de tension sur la partie nasale en utilisation.

4. Interface patient (7000) selon l'une quelconque des revendications 1 à 3, dans laquelle les conduits (7302) comprennent des sections de concertina configurées pour se déployer lors d'une application d'une force de tension.

5. Interface patient (7000) selon l'une quelconque des revendications 1 à 4, dans laquelle la partie orale comprend une paire de connecteurs (7228) espacés l'un de l'autre, et dans laquelle la paire de connecteurs (7228) sont des aimants.

6. Interface patient (7000) selon la revendication 5, dans laquelle la partie orale est moulée autour de la paire de connecteurs (7228).

7. Interface patient (7000) selon la revendication 5, comprenant en outre un insert (7244) comprenant la paire de connecteurs (7228), l'insert (7244) étant accouplé sélectivement à la chambre de distribution orale (7204) par un emmanchement à force, un emmanchement à friction et/ou par un encliquetage,
dans laquelle la paire de connecteurs peuvent être sélectivement retirés de la partie orale, et
dans laquelle l'insert (7244) comprend une valve anti-asphyxie (3900).

8. Interface patient (7000) selon l'une quelconque des revendications 5 à 7, dans laquelle la structure de positionnement et de stabilisation (7300) comprend des sangles inférieures reliées sélectivement à la partie orale par le biais des connecteurs.

9. Interface patient (7000) selon l'une quelconque des revendications 1 à 8, dans laquelle la partie de désaccouplement est configurée pour désaccoupler au moins partiellement, de la partie nasale, une force appliquée par la structure de positionnement et de stabilisation (7300) à la partie orale.

10. Interface patient (7000) selon l'une quelconque des revendications 1 à 9, dans laquelle un angle (7240) de la partie de désaccouplement entre la partie orale et la partie nasale dans une position détendue est d'environ 1° à environ 90°, de préférence d'environ 10° à environ 50°.

11. Interface patient (7000) selon l'une quelconque des revendications 1 à 10, comprenant en outre un passage (7156) formé entre la cavité nasale (7110) et la cavité orale (7136), le passage (7156) étant configuré pour établir une communication fluidique entre la cavité nasale (7110) et la cavité orale (7136),
dans laquelle le passage (7156) a une forme sensiblement elliptique, et
dans laquelle le passage (7156) est sensiblement aligné avec la partie de désaccouplement et avec la surface incurvée.

12. Interface patient (7000) selon l'une quelconque des revendications 1 à 10, comprenant en outre une membrane (7160) formée entre la cavité nasale (7110) et la cavité orale (7136),
dans laquelle la membrane (7160) s'étend entre la partie de désaccouplement et la surface incurvée,
dans laquelle la membrane (7160) est détendue avant que l'interface patient (7000) ne contacte le patient, et
dans laquelle la membrane (7160) est sensiblement solide et est configurée pour empêcher qu'un flux d'air ne passe à l'intérieur du coussin entre la cavité nasale (7110) et la cavité orale (7136).

13. Interface patient (7000) selon l'une quelconque des revendications 1 à 10, comprenant en outre une membrane (7160) formée entre la cavité nasale (7110) et la cavité orale (7136),
dans laquelle la membrane (7160) s'étend entre la partie de désaccouplement et la surface incurvée,
dans laquelle la membrane (7160) est détendue avant que l'interface patient (7000) ne contacte le patient,
dans laquelle la membrane (7160) est configurée pour permettre à un flux d'air de passer à l'intérieur du coussin entre la cavité nasale (7110) et la cavité orale (7136) à travers au moins un trou (7162),
dans laquelle l'au moins un trou (7162) est constitué d'une pluralité de trous espacés, et dans laquelle les trous de la pluralité de trous espacés sont dimensionnés pour permettre un écoulement d'air prédéterminé entre la cavité nasale (7110) et la cavité orale (7136), et/ou
la membrane (7160) ayant une porosité à un niveau microscopique et/ou moléculaire qui est configurée pour permettre un flux d'air prédéterminé passant à l'intérieur du coussin de s'écouler entre la cavité nasale (7110) et la cavité orale (7136).

14. Interface patient (7000) selon l'une quelconque des revendications 1 à 12, dans laquelle la surface incurvée est configurée pour s'étendre à plat contre le visage d'un patient en utilisation de façon à former une étanchéité unique le long de la lèvre supérieure du patient.
